# EUROPÄISCHE PATENTSCHRIFT

(11) **EP 4 097 087 B1**
(45) Veröffentlichungstag und Bekanntmachung des Hinweises auf die Patenterteilung: **31.07.2024**
(21) Anmeldenummer: 21702910.7
(22) Anmeldetag: 28.01.2021
(51) Int. Cl.: C07D 231/18, C07D 401/04, C07D 403/04, C07D 409/04, C07D 417/04, A01N 43/78, A01N 43/60, A01N 43/56, A01N 43/80

(54) **[(1,4,5-TRISUBSTITUIERTE-1H-PYRAZOL-3-YL)SULFANYL]ESSIGSÄURE DERIVATE SOWIE DEREN SALZE UND IHRE VERWENDUNG ALS HERBIZIDE WIRKSTOFFE**
[(1,4,5-TRISUBSTITUTED-1H-PYRAZOL-3-YL)SULFANYL]ACETIC ACID DERIVATIVES, THEIR SALTS AND USE OF SAID COMPOUNDS AS HERBICIDAL AGENTS
DÉRIVÉS D'ACIDE [(1,4,5-TRISUBSTITUÉ-1H-PYRAZOL-3-YL)SULFANYL]ACÉTIQUE ET LEURS SELS ET LEUR UTILISATION EN TANT QUE PRINCIPES ACTIFS HERBICIDES

(30) Priorität: 31.01.2020 EP 20154965
(43) Veröffentlichungstag der Anmeldung: 07.12.2022
(73) Patentinhaber: Bayer Aktiengesellschaft, 51373 Leverkusen (DE)
(72) Erfinder: MUELLER, Thomas, 60323 Frankfurt (DE); HOFFMANN, Michael, Gerhard, 78467 Konstanz (DE); BUSCATO ARSEQUELL, Estella, 08174 SANT CUGAT DEL VALLES (ES); JAKOBI, Harald, 51373 Leverkusen (DE); SCHMUTZLER, Dirk, 65795 Hattersheim (DE); ROSINGER, Christopher, Hugh, 65719 Hofheim am Taunus (DE); MACHETTIRA, Anu, Bheemaiah, 60326 Frankfurt (DE); ASMUS, Elisabeth, 63768 Hösbach (DE)
(74) Vertreter: BIP Patents
(86) Internationale Anmeldenummer: PCT/EP2021/051920
(87) Internationale Veröffentlichungsnummer: WO 2021/151976

(56) Entgegenhaltungen:
- WO-A1-2018/080859
- WO-A2-2009/156090
- JP-A- 2007 284 387

## Beschreibung

Die vorliegende Erfindung betrifft das technische Gebiet der Pflanzenschutzmittel, insbesondere das der Herbizide zur Bekämpfung von Unkräutern und Ungräsern in Nutzpflanzenkulturen.

Speziell betrifft diese Erfindung neue, substituierte [(1,4,5-Trisubstituierte-1H-pyrazol-3-yl)sulfanyl]essigsäure Derivate der Formel (I) oder ein agrochemisch akzeptables Salz davon, Verfahren zu ihrer Herstellung und ihre Verwendung als Herbizide.

Bisher bekannte Pflanzenschutzmittel zur Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es, dass sie (a) keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen und/oder (d) ein toxikologisch ungünstiges Profil besitzen. Weiterhin führen manche Wirkstoffe, die als Pflanzenwachstumsregulatoren bei einigen Nutzpflanzen eingesetzt werden können, bei anderen Nutzpflanzen zu unerwünscht verminderten Ernteerträgen oder sind mit der Kulturpflanze nicht oder nur in einem engen Aufwandmengenbereich verträglich. Einige der bekannten Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab.

Die herbizide Wirkung dieser bekannten Verbindungen, insbesondere bei niedrigen Aufwandmengen, bzw. deren Verträglichkeit gegenüber Kulturpflanzen bleiben verbesserungswürdig.

In EP1122244 werden 2-({4-[5-(4-tert-butyl-3-methyl-2,6-dioxo-3,6-dihydropyrimidin-1(2H)-yl)-2-chlor-4-fluorphenoxy]-1,5-dimethyl-1H-pyrazol-3-yl}sulfanyl)propionsäure-Derivate als Herbizide beschrieben. In WO2018/080859 werden [(1,5-Disubstituierte-1H-pyrazol-3-yl)sulfanyl]essigsäure Derivate beschrieben, die fungizide Eigenschaften besitzen. Aus Russian Chemical Bulletin 2010, 59(9), 1786-1790 und Yakugaku Zasshi 1971, 91(3), 311-323 ist die Synthese von [(1,5-Dimethyl-4-nitro-1H-pyrazol-3-yl)sulfanyl]essigsäure Derivaten und 1,2,3,4-tetrasubstituierte-3-pyrazolin-5-thion Derivaten bekannt. Im SciFinder^{®} sind Ethyl-{[4-nitro-1-phenyl-5-(1H-tetrazol-5-yl)-1H-pyrazol-3-yl]sulfanyl}acetat CAS [1360702-86-8 ], Ethyl-{[5-(dichlormethyl)-4-nitro-1-phenyl-1H-pyrazol-3-yl]sulfanyl}acetat CAS [1360691-01-5], Methyl-{[5-(dichlormethyl)-4-nitro-1-phenyl-1H-pyrazol-3-yl]sulfanyl} acetat CAS [1360690-82-9] und Methyl-{[5-(5-methyl-1,3,4-oxadiazol-2-yl)-4-nitro-1-phenyl-1H-pyrazol-3-yl]sulfanyl}acetat CAS [1360690-60-3] ohne Angabe einer Literaturstelle erwähnt. Andere Pyrazolyl-sulfanyl-Derivate sind auch aus WO2009/156090 und JP2007/284387 als Insektizide, Herbizide oder Wachstumsregulatoren bekannt.

Die Verwendung von [(1,4,5-Trisubstituierte-1H-pyrazol-3-yl)sulfanyl]essigsäure Derivaten oder deren Salze als herbizide Wirkstoffe, die am Pyrazol einen der definierten substituierten Phenyl- oder Hetaryl-Reste tragen ist dagegen noch nicht beschrieben. Überraschenderweise wurde nun gefunden, dass substituierte [(1,4,5-Trisubstituierte-1H-pyrazol-3-yl)sulfanyl]essigsäure Derivate oder deren Salze als herbizide Wirkstoffe, die am Pyrazol einen der definierten substituierten Phenyl- oder Hetaryl-Reste tragen, als Herbizide besonders gut geeignet sind.

Gegenstand der vorliegenden Erfindung sind damit substituierte [(1,4,5-Trisubstituierte-1H-pyrazol-3-yl)sulfanyl]essigsäure Derivate der Formel **(I)** oder ein agrochemisch akzeptables Salz davon, 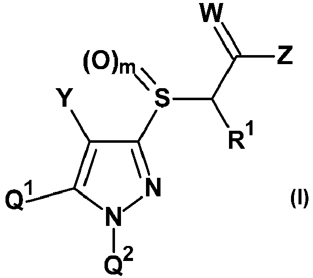 worin
Q¹ für Phenyl und Hetaryl steht,
wobei das Phenyl und das Hetaryl unsubstituiert oder jeweils unabhängig voneinander substituiert sind durch m Reste ausgewählt aus der Gruppe bestehend aus Wasserstoff, Halogen, Cyano, Isocyano, Nitro, Hydroxy, (C₁-C₆)-Alkyl, (C₁-C₆)-Haloalkyl, (C₃-C₆)-Cycloalkyl, (C₃-C₆)-Halocycloalkyl, (C₁-C₆)-Halogenalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, (C₁-C₄)-Halolkyl- S(O)ₙ, CHO, (C₁-C₄)-Alkyloxycarbonyl und NH₂ (Amino),
Q² für Phenyl steht,
welches unsubstituiert oder jeweils unabhängig voneinander substituiert ist durch m Reste ausgewählt aus der Gruppe bestehend aus Wasserstoff, Halogen, Cyano, Isocyano, NO₂, (C₁-C₆)-Alkyl, (C₁-C₆)-Haloalkyl, (C₁-C₆)-Haloalkoxy, (C₃-C₆)-Cycloalkyl, (C₃-C₆)-Halocycloalkyl, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, (C₁-C₄)-Haloalkyl- S(O)ₙ, CHO, (C₁-C₄)-Alkyloxycarbonyl und NH₂ (Amino),
Z für die Gruppen 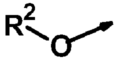 und 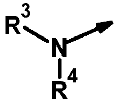 steht,
Y für - Halogen, Cyano, Isocyano, NO₂, NH₂ (Amino), (C₁-C₆)-Alkyl, (C₁-C₆)-Haloalkyl, (C₁-C₆)-Cyanoalkyl, (C₁-C₆)-Hydroxyalkyl, (C₁-C₆)-Alkoxy-(C₁-C₆)-alkyl, (C₃-C₆)-Cycloalkyl, (C₁-C₄)-Alkyloxycarbonyl , (C₁-C₆)-Alkylcarbonyl, CHO, (C₃-C₆)-Halocycloalkyl, (C₁-C₆)-Alkoxy, (C₁-C₆)-Haloalkoxy, (C₁-C₄)-Alkyl-S(O)ₙ, (C₁-C₄)-Haloalkyl- S(O)ₙ, (C₂-C₆)-Alkinyl, (C₂-C₆)-Haloalkinyl, (C₂-C₆)-Alkenyl und (C₂-C₆)-Haloalkenyl steht,
W für Sauerstoff oder Schwefel steht,
R¹ für Wasserstoff, Cyano, (C₁-C₆)-Alkyl, (C₁-C₆)-Haloalkyl, (C₁-C₆)-Alkoxy, (C₁-C₆)-Alkoxy-(C₁-C₆)-alkyl, (C₃-C₆)-Cycloalkyl, (C₃-C₆)-Halocycloalkyl, (C₂-C₆)-Alkinyl, (C₂-C₆)-Haloalkinyl, (C₂-C₆)-Alkenyl und (C₂-C₆)-Haloalkenyl steht,
R² für Wasserstoff, (C₁-C₁₀)-Alkyl und (C₃-C₁₀)-Cycloalkyl steht,
wobei das Alkyl und Cycloalkyl unsubstituiert oder jeweils unabhängig voneinander substituiert sind durch m Reste ausgewählt aus der Gruppe bestehend aus Halogen, Cyano, Isocyano, Nitro, NH₂ (Amino), (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl, Heterocyclyl-(C₁-C₄)-alkyl Heteroaryl-(C₁-C₄)-alkyl und Aryl-(C₁-C₄)-alkyl, wobei das Aryl, Heterocyclyl und Heteroaryl unsubstituiert oder jeweils unabhängig voneinander substituiert ist durch m Reste ausgewählt aus der Gruppe bestehend aus Halogen, (C₁-C₆)-Alkyl, (C₁-C₆)-Haloalkyl,
R³ für Wasserstoff und (C₁-C₁₂)-Alkyl steht,
R⁴ für Wasserstoff, Cyano, Nitro, (C₁-C₁₂)-Alkyl, (C₁-C₁₀)-Haloalkyl, (C₂-C₁₀)-Alkenyl, (C₃-C₁₀)-Alkinyl, (C₁-C₁₀)-Alkoxy-(C₁-C₁₀)-alkyl, (C₁-C₁₀)-Haloalkoxy-(C₁-C₁₀)-alkyl, (C₁-C₄)-Alkyl-S(O)ₙ-(C₁-C₄)-alkyl, (C₁-C₄)-Haloalkyl-S(O)ₙ-(C₁-C₄)-Alkyl, (C₃-C₁₀)-Cycloalkyl, (C₃-C₁₀)-Halocycloalkyl, Hydroxy-(C₁-C₁₀)-alkylcarbonyl, Amino-(C₁-C₁₀)-alkyl, (C₁-C₁₀)-Alkoxycarbonyl-(C₁-C₁₀)-alkyl, (C₁-C₁₀)-Cyanoalkyl, S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸, Aryl, Heteroaryl und Heterocyclyl steht,
welche unsubstituiert oder jeweils unabhängig voneinander substituiert sind durch m Reste ausgewählt aus der Gruppe bestehend aus Wasserstoff, Halogen, Cyano, Nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
   oder
R³ und R⁴ bilden mit dem N-Atom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring,
R⁵ für Wasserstoff, (C₁-C₈)-Alkyl, (C₃-C₆)-Cycloalkyl, (C₁-C₈)-Haloalkyl und Aryl steht,
R⁶ für Wasserstoff und R⁵ steht,
R⁷ für Wasserstoff, (C₁-C₆)-Alkyl, (C₃-C₆)-Cycloalkyl, (C₃-C₄)-Alkenyl, (C₃-C₄)-Alkinyl und (C₁-C₁₀)-alkylcarbonyl-(C₁-C₆)-alkyl steht,
R⁸ für Wasserstoff, (C₁-C₆)-Alkyl, (C₃-C₆)-Cycloalkyl, (C₃-C₄)-Alkenyl, und (C₃-C₄)-Alkinyl, steht,
m für 0, 1 oder 2 steht,
   und
n für 0, 1 oder 2 steht,
wobei die Verbindungen Ethyl-{[4-nitro-1-phenyl-5-(1H-tetrazol-5-yl)-1H-pyrazol-3 -yl] sulfanyl} acetat CAS [1360702-86-8 ] und Methyl-{[5-(5-methyl-1,3,4-oxadiazol-2-yl)-4-nitro-1-phenyl-1H-pyrazol-3-yl]sulfanyl} acetat CAS [1360690-60-3] ausgenommen sind.

Die Verbindungen der allgemeinen Formel **(I)** können durch Anlagerung einer geeigneten anorganischen oder organischen Säure, wie beispielsweise Mineralsäuren, wie beispielsweise HCl, HBr, H₃SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel *p*-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion. Geeignete Substituenten, die in deprotonierter Form, wie z.B. Sulfonsäuren, bestimmte Sulfonsäureamide oder Carbonsäuren, vorliegen, können innere Salze mit ihrerseits protonierbaren Gruppen, wie Aminogruppen bilden. Salzbildung kann auch durch Einwirkung einer Base auf Verbindungen der allgemeinen Formel **(I)** erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der azide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel [NR^{a}R^{b}R^{c}R^{d}]⁺, worin R^{a} bis R^{d} jeweils unabhängig voneinander einen organischen Rest, insbesondere Alkyl, Aryl, Arylalkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die erfindungsgemäßen substituierten [(1,4,5-Trisubstituierte-1H-pyrazol-3-yl)sulfanyl]essigsäure Derivate der allgemeinen Formel **(I)** können in Abhängigkeit von äußeren Bedingungen, wie pH-Wert, Lösungsmittel und Temperatur in verschiedenen tautomeren Strukturen vorliegen, die alle von der allgemeinen Formel **(I)** umfasst sein sollen.

Im Folgenden werden die erfindungsgemäß verwendeten Verbindungen der Formel **(I)** und ihre Salze "Verbindungen der allgemeinen Formel **(I)**" bezeichnet.

Bevorzugter Erfindungsgegenstand sind Verbindungen der allgemeinen Formel **(I),** worin
Q¹ für die Gruppen Q¹-1.1 bis Q¹-6.5

| | | | | |
|---|---|---|---|---|
| 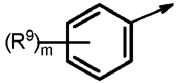 | 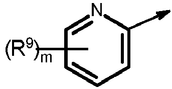 | 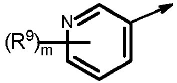 | 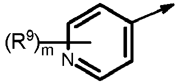 | 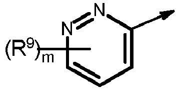 |
| Q¹-1.1 | Q¹-2.1 | Q¹-2.2 | Q¹-2.3 | Q¹-3.1 |
| 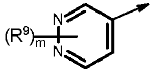 | 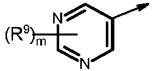 | 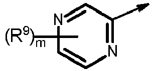 | 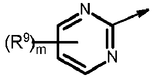 | 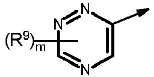 |
| Q¹-3.2 | Q¹-3.3 | Q¹-3.4 | Q¹-3.5 | Q¹-4.1 |
| 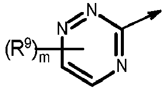 | 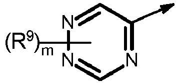 | 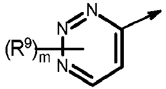 | 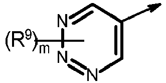 | 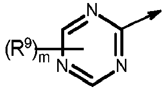 |
| Q¹-4.2 | Q¹-4.3 | Q¹-4.4 | Q¹-4.5 | Q¹-4.6 |
| 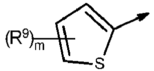 | 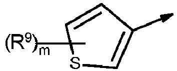 | 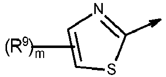 | 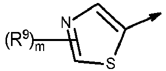 | 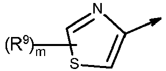 |
| Q¹-5.1 | Q¹-5.2 | Q¹-5.3 | Q¹-5.4 | Q¹-5.5 |
| 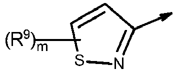 | 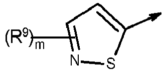 | 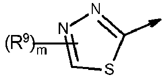 | 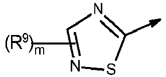 | 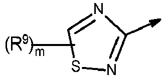 |
| Q¹-5.6 | Q¹-5.7 | Q¹-6.1 | Q¹-6.2 | Q¹-6.3 |
| 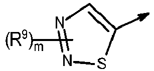 | 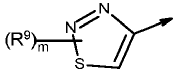 | | | |
| Q¹-6.4 | Q¹-6.5 | | | |

steht,
Q² für Phenyl steht,
welches unsubstituiert oder jeweils unabhängig voneinander substituiert ist durch m Reste ausgewählt aus der Gruppe bestehend aus Wasserstoff, Halogen, Cyano, Isocyano, NO₂, (C₁-C₆)-Alkyl, (C₁-C₆)-Haloalkyl, (C₁-C₆)-Haloalkoxy, (C₃-C₆)-Cycloalkyl, (C₃-C₆)-Halocycloalkyl, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, (C₁-C₄)-Haloalkyl- S(O)ₙ, CHO, (C₁-C₄)-Alkyloxycarbonyl und NH₂ (Amino),
Z für die Gruppen 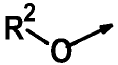 und 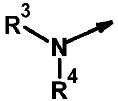 steht,
Y für - Halogen, Cyano, Isocyano, NO₂, NH₂ (Amino), (C₁-C₆)-Alkyl, (C₁-C₆)-Haloalkyl, (C₁-C₆)-Cyanoalkyl, (C₁-C₆)-Hydroxyalkyl, (C₁-C₆)-Alkoxy-(C₁-C₆)-alkyl, (C₃-C₆)-Cycloalkyl, (C₁-C₄)-Alkyloxycarbonyl , (C₁-C₆)-Alkylcarbonyl, CHO, (C₃-C₆)-Halocycloalkyl, (C₁-C₆)-Alkoxy, (C₁-C₆)-Haloalkoxy, (C₁-C₄)-Alkyl- S(O)ₙ, (C₁-C₄)-Haloalkyl- S(O)ₙ, (C₂-C₆)-Alkinyl, (C₂-C₆)-Haloalkinyl, (C₂-C₆)-Alkenyl und (C₂-C₆)-Haloalkenyl steht,
W für Sauerstoff oder Schwefel steht,
R¹ für Wasserstoff, Cyano, (C₁-C₆)-Alkyl, (C₁-C₆)-Haloalkyl, (C₁-C₆)-Alkoxy, (C₁-C₆)-Alkoxy-(C₁-C₆)-alkyl, (C₃-C₆)-Cycloalkyl, (C₃-C₆)-Halocycloalkyl, (C₂-C₆)-Alkinyl, (C₂-C₆)-Haloalkinyl, (C₂-C₆)-Alkenyl und (C₂-C₆)-Haloalkenyl steht,
R² für Wasserstoff ,(C₁-C₁₀)-Alkyl und (C₃-C₁₀)-Cycloalkyl steht,
wobei das Alkyl und Cycloalkyl unsubstituiert oder jeweils unabhängig voneinander substituiert ist durch m Reste ausgewählt aus der Gruppe bestehend aus Halogen, Cyano, Isocyano, Nitro, NH₂ (Amino), (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl, Heterocyclyl-(C₁-C₆)-alkyl Heteroaryl-(C₁-C₄)-alkyl und Aryl-(C₁-C₄)-alkyl, wobei das Aryl, Heterocyclyl und Heteroaryl unsubstituiert oder jeweils unabhängig voneinander substituiert ist durch m Reste ausgewählt aus der Gruppe bestehend aus Halogen, (C₁-C₆)-Alkyl und (C₁-C₆)-Haloalkyl,
R³ für Wasserstoff und (C₁-C₁₀)-Alkyl, steht,
R⁴ für Wasserstoff, Cyano, Nitro, (C₁-C₁₀)-Alkyl, (C₁-C₁₀)-Haloalkyl, (C₂-C₁₀)-Alkenyl, (C₃-C₁₀)-Alkinyl, (C₁-C₁₀)-Alkoxy-(C₁-C₁₀)-alkyl, (C₁-C₁₀)-Haloalkoxy-(C₁-C₁₀)-alkyl, (C₁-C₄)-Alkyl-S(O)ₙ-(C₁-C₄)-alkyl, (C₁-C₄)-Haloalkyl-S(O)ₙ-(C₁-C₄)-Alkyl, (C₃-C₁₀)-Cycloalkyl, (C₃-C₁₀)-Halocycloalkyl, Hydroxy-(C₁-C₁₀)-alkylcarbonyl, Amino-(C₁-C₁₀)-alkyl, (C₁-C₁₀)-Alkoxycarbonyl-(C₁-C₁₀)-alkyl, (C₁-C₁₀)-Cyanoalkyl, S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸; Aryl, Heteroaryl und Heterocyclyl, steht,
welche unsubstituiert sind oder jeweils unabhängig voneinander substituiert sind durch m Reste ausgewählt aus der Gruppe bestehend aus Wasserstoff, Halogen, Cyano, Nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
oder R³ und R⁴ bilden mit dem N-Atom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring,
R⁵ für Wasserstoff, (C₁-C₈)-Alkyl, (C₃-C₆)-Cycloalkyl, (C₁-C₈)-Haloalkyl und Aryl steht,
R⁶ für Wasserstoff und R⁵ steht,
R⁷ für Wasserstoff, (C₁-C₆)-Alkyl, (C₃-C₆)-Cycloalkyl, (C₃-C₄)-Alkenyl, (C₃-C₄)-Alkinyl, und (C₁-C₆)-alkylcarbonyl-(C₁-C₄)-alkyl steht,
R⁸ für Wasserstoff, (C₁-C₆)-Alkyl, (C₃-C₆)-Cycloalkyl, (C₃-C₄)-Alkenyl und (C₃-C₄)-Alkinyl steht,
R⁹ für Wasserstoff, Halogen, Cyano, Isocyano, Nitro, Hydroxy, (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl-S(O)ₙ, CHO, (C₁-C₄)-Alkyloxycarbonyl und NH₂ (Amino) steht,
m für 0, 1 und 2, steht,
n für 0, 1 und 2, steht.

Besonders bevorzugter Erfindungsgegenstand sind Verbindungen der allgemeinen Formel **(I),** worin
Q¹ für die Gruppen Q¹-1.1 bis Q¹-6.3 steht,

| | | | | |
|---|---|---|---|---|
| 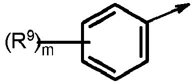 | 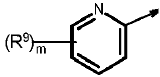 | 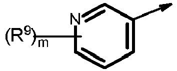 | 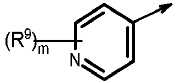 | 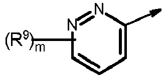 |
| Q¹-1.1 | Q¹-2.1 | Q¹-2.2 | Q¹-2.3 | Q¹-3.1 |
| 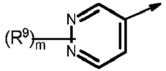 | 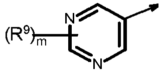 | 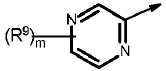 | 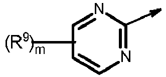 | 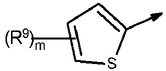 |
| Q¹-3.2 | Q¹-3.3 | Q¹-3.4 | Q¹-3.5 | Q¹-5.1 |
| 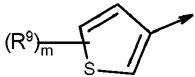 | 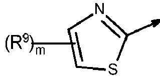 | 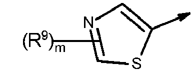 | 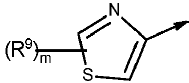 | 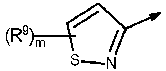 |
| Q¹-5.2 | Q¹-5.3 | Q¹-5.4 | Q¹-5.5 | Q¹-5.6 |
| 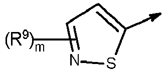 | 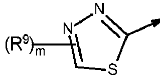 | 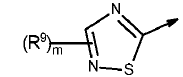 | 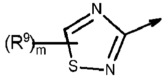 | |
| Q¹-5.7 | Q¹-6.1 | Q¹-6.2 | Q¹-6.3 | |

Q² für Phenyl steht, welches unsubstituiert oder jeweils unabhängig voneinander substituiert ist durch m Reste ausgewählt aus der Gruppe bestehend aus Wasserstoff, Halogen, Cyano, (C₁-C₆)-Alkyl, (C₁-C₆)-Haloalkyl, (C₁-C₆)-Haloalkoxy, (C₃-C₆)-Cycloalkyl, (C₁-C₆)-Alkoxy, (C₁-C₄)-Alkyl- S(O)ₙ und (C₁-C₄)-Haloalkyl- S(O)ₙ,
Z für die Gruppen 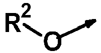 und 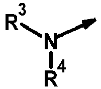 steht,
Y für Halogen, Cyano, Isocyano, NO₂, (C₁-C₆)-Alkyl, (C₁-C₆)-Haloalkyl, (C₁-C₆)-Alkoxy-(C₁-C₆)-alkyl, (C₁-C₄)-Alkyloxycarbonyl , (C₁-C₆)-Alkylcarbonyl, (C₁-C₆)-Alkoxy, (C₁-C₆)-Haloalkoxy, (C₁-C₄)-Alkyl- S(O)ₙ, (C₁-C₄)-Haloalkyl- S(O)ₙ, (C₂-C₆)-Alkinyl, (C₂-C₆)-Haloalkinyl, (C₂-C₆)-Alkenyl und (C₂-C₆)-Haloalkenyl steht,
W für Sauerstoff steht,
R¹ für Wasserstoff, Cyano, (C₁-C₆)-Alkyl, (C₁-C₆)-Haloalkyl, (C₁-C₆)-Alkoxy, (C₁-C₄)-Alkoxy-(C₁-C₄)-alkyl, (C₃-C₆)-Cycloalkyl, (C₂-C₆)-Alkinyl, (C₂-C₆)-Haloalkinyl, (C₂-C₆)-Alkenyl und (C₂-C₆)-Haloalkenyl steht,
R² für Wasserstoff, (C₁-C₆)-Alkyl und (C₃-C₆)-Cycloalkyl steht,
wobei das Alkyl und Cycloalkyl unsubstituiert oder jeweils unabhängig voneinander substituiert ist durch m Reste ausgewählt aus der Gruppe bestehend aus Halogen, Cyano, Nitro, NH₂ (Amino), (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl, Heterocyclyl-(C₁-C₄)-alkyl Heteroaryl-(C₁-C₄)-alkyl und Aryl-(C₁-C₄)-alkyl, wobei das Aryl, Heterocyclyl und Heteroaryl unsubstituiert oder jeweils unabhängig voneinander substituiert ist durch m Reste ausgewählt aus der Gruppe bestehend aus Halogen, (C₁-C₆)-Alkyl, (C₁-C₆)-Haloalkyl,
R³ für Wasserstoff, und (C₁-C₆)-Alkyl, steht,
R⁴ für Wasserstoff, Cyano, Nitro, (C₁-C₆)-Alkyl, (C₁-C₆)-Haloalkyl, (C₂-C₆)-Alkenyl, (C₃-C₆)-Alkinyl, (C₁-C₆)-Alkoxy-(C₁-C₆)-alkyl, (C₁-C₆)-Haloalkoxy-(C₁-C₆)-alkyl, (C₁-C₄)-Alkyl-S(O)ₙ-(C₁-C₄)-alkyl und (C₁-C₄)-Haloalkyl-S(O)ₙ-(C₁-C₄)-Alkyl, (C₃-C₆)-Cycloalkyl, (C₃-C₆)-Halocycloalkyl, Hydroxy-(C₁-C₆)-alkylcarbonyl, Amino-(C₁-C₆)-alkyl, (C₁-C₆)-Alkoxycarbonyl-(C₁-C₆)-alkyl, S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸; Aryl, Heteroaryl und Heterocyclyl steht,
welche unsubstituiert sind oder jeweils unabhängig voneinander substituiert sind durch m Reste ausgewählt aus der Gruppe bestehend aus Wasserstoff, Halogen, Cyano, Nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
oder R³ und R⁴ bilden mit dem N-Atom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring,
R⁵ für Wasserstoff, (C₁-C₈)-Alkyl, (C₃-C₆)-Cycloalkyl, (C₁-C₈)-Haloalkyl und Aryl steht,
R⁶ für Wasserstoff und R⁵ steht,
R⁷ für Wasserstoff, (C₁-C₆)-Alkyl, (C₃-C₆)-Cycloalkyl, (C₃-C₄)-Alkenyl, und (C₃-C₄)-Alkinyl, steht,
R⁸ für Wasserstoff, (C₁-C₆)-Alkyl, (C₃-C₆)-Cycloalkyl, (C₃-C₄)-Alkenyl, (C₃-C₄)-Alkinyl, und (C₁-C₁₀)-alkylcarbonyl-(C₁-C₆)-alkyl, steht,
R⁹ für Wasserstoff, Halogen, Cyano, Isocyano, Nitro, Hydroxy, (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl und NH₂ (Amino) steht,
m für 0, 1 und 2 steht,
n für 0, 1 und 2 steht.

Ganz besonders bevorzugter Erfindungsgegenstand sind Verbindungen der allgemeinen Formel (I), worin
Q¹ für die Gruppen Q¹-1.1 bis Q¹-6.3 steht,

| | | | | |
|---|---|---|---|---|
| 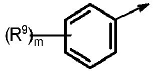 | 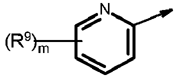 | 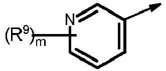 | 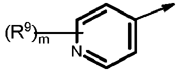 | 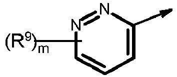 |
| Q¹-1.1 | Q¹-2.1 | Q¹-2.2 | Q¹-2.3 | Q¹-3.1 |
| 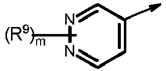 | 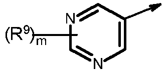 | 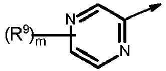 | 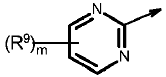 | 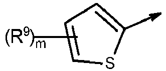 |
| Q¹-3.2 | Q¹-3.3 | Q¹-3.4 | Q¹-3.5 | Q¹-5.1 |
| 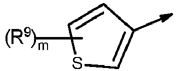 | 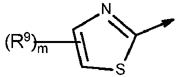 | 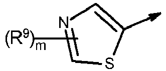 | 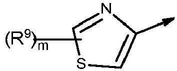 | 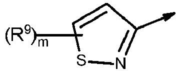 |
| Q¹-5.2 | Q¹-5.3 | Q¹-5.4 | Q¹-5.5 | Q¹-5.6 |
| 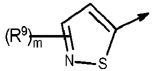 | 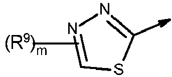 | 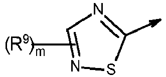 | 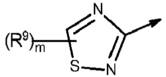 | |
| Q¹-5.7 | Q¹-6.1 | Q¹-6.2 | Q¹-6.3 | |

Q² für Phenyl steht,
welcher unsubstituiert oder jeweils unabhängig voneinander substituiert ist durch m Reste ausgewählt aus der Gruppe bestehend aus Wasserstoff, Fluor, Chlor und Brom,
Z für die Gruppen 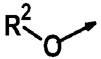 und 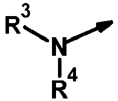 steht,
Y für Fluor, Chlor, Brom, Cyano, NO₂, (C₁-C₂)-Alkyl, (C₁-C₂)-Haloalkyl, (C₁-C₂)-Alkylcarbonyl, (C₁-C₂)-Alkoxy, (C₁-C₂)-Haloalkoxy, (C₁-C₂)-Alkyl- S(O)ₙ, und (C₁-C₂)-Haloalkyl- S(O)ₙ, steht,
W für Sauerstoff steht,
R¹ für Wasserstoff, (C₁-C₄)-Alkyl, (C₁-C₄)-Haloalkyl, und (C₁-C₆)-Alkoxy, steht,
R² für Wasserstoff, (C₁-C₆)-Alkyl und (C₃-C₆)-Cycloalkyl steht,
wobei das Alkyl und Cycloalkyl unsubstituiert oder jeweils unabhängig voneinander substituiert ist durch m Reste ausgewählt aus der Gruppe bestehend aus Halogen, Cyano, Nitro, NH₂ (Amino), (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl, Heterocyclyl-(C₁-C₄)-alkyl Heteroaryl-(C₁-C₄)-alkyl und Aryl-(C₁-C₄)-alkyl, wobei das Aryl, Heterocyclyl und Heteroaryl unsubstituiert oder jeweils unabhängig voneinander substituiert ist durch m Reste ausgewählt aus der Gruppe bestehend aus Halogen, (C₁-C₆)-Alkyl, (C₁-C₆)-Haloalkyl,
R³ für Wasserstoff, und (C₁-C₆)-Alkyl steht,
R⁴ für Wasserstoff, Cyano, Nitro, (C₁-C₆)-Alkyl, (C₁-C₆)-Haloalkyl, (C₂-C₆)-Alkenyl, (C₃-C₆)-Alkinyl, (C₁-C₆)-Alkoxy-(C₁-C₆)-alkyl, (C₁-C₆)-Haloalkoxy-(C₁-C₆)-alkyl, (C₁-C₄)-Alkyl-S(O)ₙ-(C₁-C₄)-alkyl und (C₁-C₄)-Haloalkyl-S(O)ₙ₋(C₁-C₄)-Alkyl, (C₃-C₆)-Cycloalkyl, (C₃-C₆)-Halocycloalkyl, Hydroxy-(C₁-C₆)-alkylcarbonyl, Amino-(C₁-C₆)-alkyl, (C₁-C₆)-Alkoxycarbonyl-(C₁-C₆)-alkyl, S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸; Aryl, Heteroaryl und Heterocyclyl steht,
welche unsubstituiert oder jeweils unabhängig voneinander substituiert sind durch m Reste ausgewählt aus der Gruppe bestehend aus Wasserstoff, Halogen, Cyano, Nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
oder R³ und R⁴ bilden mit dem N-Atom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring,
R⁵ für Wasserstoff, (C₁-C₈)-Alkyl, (C₃-C₆)-Cycloalkyl, (C₁-C₈)-Haloalkyl und Aryl steht,
R⁶ für Wasserstoff und R⁵ steht,
R⁷ für Wasserstoff, (C₁-C₆)-Alkyl, (C₃-C₆)-Cycloalkyl, (C₃-C₄)-Alkenyl, (C₃-C₄)-Alkinyl und (C₁-C₁₀)-alkylcarbonyl-(C₁-C₆)-alkyl steht,
R⁸ für Wasserstoff, (C₁-C₆)-Alkyl, (C₃-C₆)-Cycloalkyl, (C₃-C₄)-Alkenyl und (C₃-C₄)-Alkinyl steht,
R⁹ für Wasserstoff, Halogen, Cyano, Isocyano, Nitro, Hydroxy, (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl und NH₂ (Amino) steht,
m für 0, 1 und 2 steht,
n für 0, 1 und 2 steht.

Im Speziellen bevorzugter Erfindungsgegenstand sind Verbindungen der allgemeinen Formel (I), worin
Q¹ für die Gruppen Q¹-1.1 bis Q¹-5.7

| | | | | |
|---|---|---|---|---|
| 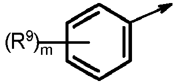 | 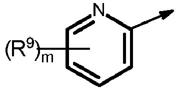 | 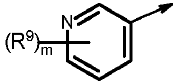 | 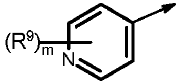 | 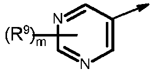 |
| Q¹-1.1 | Q¹-2.1 | Q¹-2.2 | Q¹-2.3 | Q¹-3.3 |
| 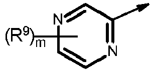 | 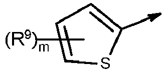 | 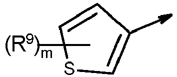 | 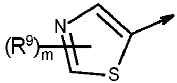 | 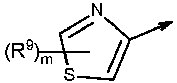 |
| Q¹-3.4 | Q¹-5.1 | Q¹-5.2 | Q¹-5.4 | Q¹-5.5 |
| 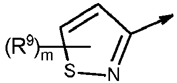 | 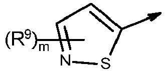 | | | |
| Q¹-5.6 | Q¹-5.7 | | | |

steht,
Q² für Phenyl steht,
welches unsubstituiert oder jeweils unabhängig voneinander substituiert ist durch m Reste ausgewählt aus der Gruppe bestehend aus Wasserstoff, Fluor, Chlor und Brom,
Z für die Gruppen 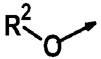 und 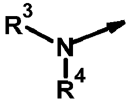 steht,
- Y: für Fluor, Chlor, Brom, Cyano, NO₂, Methyl, CF₃ und OCF₃ steht,
- W: für Sauerstoff steht,
- R¹: für Wasserstoff, Methyl und Ethyl steht,
- R²: für Wasserstoff, Methyl, Ethyl, Allyl, Propargyl und PhCH₂ steht,
- R³: für Wasserstoff steht,
- R⁴: für (C₁-C₆)-Alkyl, S(O)ₙR⁵, OR⁵, SO₂NR⁶R⁷, CO₂R⁸, COR⁸ steht,

welche unsubstituiert sind oder jeweils unabhängig voneinander substituiert sind durch m Reste ausgewählt aus der Gruppe bestehend aus Wasserstoff, OR⁵, S(O)ₙR⁵, SO₂NR⁶R⁷, CO₂R⁸, COR⁶, NR⁶CO₂R⁸,
- R⁵: für Methyl, Ethyl, CF₃, CH₂CF₃ steht,
- R⁶: für Wasserstoff, und R⁵ steht,
- R⁷: für Wasserstoff, Methyl, Ethyl, und Ethyl-2-ethanoyl steht,
- R⁸: für Methyl, und Ethyl steht,
- R⁹: für Wasserstoff, Fluor, Chlor, Brom, Cyano, Hydroxy, Methyl, Ethyl, OCH₃, CF₃, und OCF₃ steht,
- m: für 0, 1 und 2 steht,
- n: für 0, 1 und 2 steht.

Die oben aufgeführten allgemeinen oder in Vorzugsbereichen aufgeführten Restedefinitionen gelten sowohl für die Endprodukte der allgemeinen Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Vor allem aus den Gründen der höheren herbiziden Wirkung, besseren Selektivität und/oder besseren Herstellbarkeit sind erfindungsgemäße Verbindungen der genannten allgemeinen Formel (1) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:
Sofern nicht anders definiert, gilt generell für die Bezeichnung von chemischen Gruppen, dass die Anbindung an das Gerüst bzw. den Rest des Moleküls über das zuletzt genannte Strukturelement der betreffenden chemischen Gruppe erfolgt, d.h. beispielsweise im Falle von (C₂-C₈)-Alkenyloxy über das Sauerstoffatom, und im Falle von Heterocyclyl-(C₁-C₈)-alkyl oder R¹²O(O)C-(C₁-C₈)-Alkyl jeweils über das C-Atom der Alkylgruppe.

Erfindungsgemäß steht "Alkylsulfonyl" - in Alleinstellung oder als Bestandteil einer chemischen Gruppe - für geradkettiges oder verzweigtes Alkylsulfonyl, vorzugsweise mit 1 bis 8, oder mit 1 bis 6 Kohlenstoffatomen, z.B. (aber nicht beschränkt auf) (C₁-C₆)-Alkylsulfonyl wie Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl.

Erfindungsgemäß steht "Heteroarylsulfonyl" für gegebenenfalls substituiertes Pyridylsulfonyl, Pyrimidinylsulfonyl, Pyrazinylsulfonyl oder gegebenenfalls substituiertes polycyclisches Heteroarylsulfonyl, hier insbesondere gegebenenfalls substituiertes Chinolinylsulfonyl, beispielsweise substituiert durch Fluor, Chlor, Brom, Iod, Cyano, Nitro, Alkyl-, Haloalkyl-, Haloalkoxy-, Amino-, Alkylamino-, Alkylcarbonylamino-, Dialkylamino- oder Alkoxygruppen.

Erfindungsgemäß steht "Alkylthio" - in Alleinstellung oder als Bestandteil einer chemischen Gruppe - für geradkettiges oder verzweigtes S-Alkyl, vorzugsweise mit 1 bis 8, oder mit 1 bis 6 Kohlenstoffatomen, wie (C₁-C₁₀)-, (C₁-C₆)- oder (C₁-C₄)-Alkylthio, z.B. (aber nicht beschränkt auf) (C₁-C₆)-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 1,1-Dimethylpropylthio, 1,2-Dimethylpropylthio, 2,2-Dimethylpropylthio, 1-Ethylpropylthio, Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio.

"Cycloalkylthio" bedeutet bedeutet erfindungsgemäßt ein über ein Schwefelatom gebundenen Cycloalkylrest.

"Alkylsulfinyl (Alkyl-S(=O)-)", soweit nicht an anderer Stelle anders definiert steht erfindungsgemäß für Alkylreste, die über -S(=O)- an das Gerüst gebunden sind, wie (C₁-C₁₀)-, (C₁-C₆)- oder (C₁-C₄)-Alkylsulfinyl, z. B. (aber nicht beschränkt auf) (C₁-C₆)-Alkylsulfinyl wie Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 1,1-Dimethylpropylsulfinyl, 1,2-Dimethylpropylsulfinyl, 2,2-Dimethylpropylsulfinyl, 1-Ethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl.

"Alkoxy" bedeutet ein über ein Sauerstoffatom gebundenen Alkylrest, z. B. (aber nicht beschränkt auf) (C₁-C₆)-Alkoxy wie Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Alkenyloxy bedeutet ein über ein Sauerstoffatom gebundenen Alkenylrest, Alkinyloxy bedeutet ein über ein Sauerstoffatom gebundenen Alkinylrest wie (C₂-C₁₀)-, (C₂-C₆)- oder (C₂-C₄)-Alkenoxy bzw. (C₃-C₁₀)-, (C₃-C₆)- oder (C₃-C₄)-Alkinoxy.

"Cycloalkyloxy" bedeutet ein über ein Sauerstoffatom gebundenen Cycloalkylrest.

"Alkylcarbonyl" (Alkyl-C(=O)-), soweit nicht an anderer Stelle anders definiert, steht erfindungsgemäß für Alkylreste, die über -C(=O)- an das Gerüst gebunden sind, wie (C₁-C₁₀)-, (C₁-C₆)- oder (C₁-C₄)-Alkylcarbonyl. Die Anzahl der C-Atome bezieht sich dabei auf den Alkylrest in der Alkylcarbonylgruppe.

"Alkoxycarbonyl (Alkyl-O-C(=O)-)", soweit nicht an anderer Stelle anders definiert: Alkylreste, die über -O-C(=O)- an das Gerüst gebunden sind, wie (C₁-C₁₀)-, (C₁-C₆)- oder (C₁-C₄)-Alkoxycarbonyl. Die Anzahl der C-Atome bezieht sich dabei auf den Alkylrest in der Alkoxycarbonylgruppe. Analog stehen "Alkenyloxycarbonyl" und "Alkinyloxycarbonyl", soweit nicht an anderer Stelle anders definiert, erfindungsgemäß für Alkenyl- bzw. Alkinylreste, die über -O-C(=O)- an das Gerüst gebunden sind, wie (C₂-C₁₀)-, (C₂-C₆)- oder (C₂-C₄)-Alkenyloxycarbonyl bzw. (C₃-C₁₀)-, (C₃-C₆)- oder (C₃-C₄)-Alkinyloxycarbonyl. Die Anzahl der C-Atome bezieht sich dabei auf den Alkenyl- bzw. Alkinylrest in der Alken- bzw. Alkinyloxycarbonylgruppe.

Der Begriff "Alkylcarbonyloxy" (Alkyl-C(=O)-O-) steht erfindungsgemäß, soweit nicht an anderer Stelle anders definiert, für Alkylreste, die über eine Carbonyloxygruppe (-C(=O)-O-) mit dem Sauerstoff an das Gerüst gebunden sind, wie (C₁-C₁₀)-, (C₁-C₆)- oder (C₁-C₄)-Alkylcarbonyloxy. Die Anzahl der C-Atome bezieht sich dabei auf den Alkylrest in der Alkylcarbonyloxygruppe.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst. Bevorzugte Aryl-Substituenten sind hier zum Beispiel Wasserstoff, Halogen, Alkyl, Cycloalkyl, Cycloalkylalkyl, Cycloalkenyl, Halocycloalkyl, Alkenyl, Alkinyl, Aryl, Arylalkyl, Heteroaryl, Heteroarylalkyl, Heterocyclyl, Heterocyclylalkyl, Alkoxyalkyl, Alkylthio, Haloalkylthio, Haloalkyl, Alkoxy, Haloalkoxy, Cycloalkoxy, Cycloalkylalkoxy, Aryloxy, Heteroraryloxy, Alkoxyalkoxy, Alkinylalkoxy, Alkenyloxy, Bis-alkylaminoalkoxy, Tris-[alkyl]silyl, Bis-[alkyl]arylsilyl, Bis-[alkyl]alkylsilyl, Tris-[alkyl]silylalkinyl, Alkylalkinyl, Cycloalkylalkinyl, Haloalkylalkinyl, Heterocyclyl-N-alkoxy, Nitro, Cyano, Amino, Alkylamino, Bis-alkylamino, Alkylcarbonylamino, Cycloalkylcarbonylamino, Arylcarbonylamino, Alkoxycarbonylamino, Alkoxycarbonylalkylamino, Arylalkoxycarbonylalkylamino, Hydroxycarbonyl, Alkoxycarbonyl, Aminocarbonyl, Alkylaminocarbonyl, Cycloalkylaminocarbonyl, Bis-Alkylaminocarbonyl, Heteroarylalkoxy, Arylalkoxy

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P) der gesättigt, ungesättigt, teilgesättigt oder heteroaromatisch ist und dabei unsubstituiert oder substituiert sein kann, wobei die Bindungsstelle an einem Ringatom lokalisiert ist. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfasst, wie beispielsweise 8-Aza-bicyclo[3.2.1]octanyl, 8-Aza-bicyclo[2.2.2]octanyl oder 1-Aza-bicyclo[2.2.1]heptyl. Im Falle von gegebenenfalls substituiertem Heterocyclyl werden auch spirocyclische Systeme umfasst, wie beispielsweise 1-Oxa-5-aza-spiro[2.3]hexyl. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen, wie beispielsweise mit einem Heteroatom aus der Gruppe N, O und S 1- oder 2- oder 3-Pyrrolidinyl, 3,4-Dihydro-2H-pyrrol-2- oder 3-yl, 2,3-Dihydro-1H-pyrrol-1- oder 2- oder 3- oder 4- oder 5-yl; 2,5-Dihydro-1H-pyrrol-1- oder 2- oder 3-yl, 1- oder 2- oder 3- oder 4-Piperidinyl; 2,3,4,5-Tetrahydropyridin-2- oder 3- oder 4- oder 5-yl oder 6-yl; 1,2,3,6-Tetrahydropyridin-1- oder 2- oder 3- oder 4- oder 5- oder 6-yl; 1,2,3,4-Tetrahydropyridin-1- oder 2- oder 3- oder 4- oder 5- oder 6-yl; 1,4-Dihydropyridin-1- oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2- oder 3- oder 4-Azepanyl; 2,3,4,5-Tetrahydro-1H-azepin-1- oder 2- oder 3- oder 4- oder 5- oder 6- oder 7-yl; 2,3,4,7-Tetrahydro-1H-azepin-1- oder 2- oder 3- oder 4- oder 5- oder 6- oder 7-yl; 2,3,6,7-Tetrahydro-1H-azepin-1- oder 2- oder 3- oder 4-yl; 3,4,5,6-Tetrahydro-2H-azepin-2- oder 3- oder 4- oder 5- oder 6- oder 7-yl; 4,5-Dihydro-1H-azepin-1- oder 2- oder 3- oder 4-yl; 2,5-Dihydro-1H-azepin-1- oder -2- oder 3- oder 4- oder 5- oder 6- oder 7-yl; 2,7-Dihydro-1H-azepin-1- oder -2- oder 3- oder 4-yl; 2,3-Dihydro-1H-azepin-1- oder -2- oder 3- oder 4- oder 5- oder 6- oder 7-yl; 3,4-Dihydro-2H-azepin-2- oder 3- oder 4- oder 5- oder 6- oder 7-yl; 3,6-Dihydro-2H-azepin-2- oder 3- oder 4- oder 5- oder 6- oder 7-yl; 5,6-Dihydro-2H-azepin-2- oder 3- oder 4- oder 5- oder 6- oder 7-yl; 4,5-Dihydro-3H-azepin-2- oder 3- oder 4- oder 5- oder 6- oder 7-yl; 1H-Azepin-1- oder -2- oder 3- oder 4- oder 5- oder 6- oder 7-yl; 2H-Azepin-2- oder 3- oder 4- oder 5- oder 6- oder 7-yl; 3H-Azepin-2- oder 3- oder 4- oder 5- oder 6- oder 7-yl; 4H-Azepin-2- oder 3- oder 4- oder 5- oder 6- oder 7-yl, 2- oder 3-Oxolanyl (= 2- oder 3-Tetrahydrofuranyl); 2,3-Dihydrofuran-2- oder 3- oder 4- oder 5-yl; 2,5-Dihydrofuran-2- oder 3-yl, 2- oder 3- oder 4-Oxanyl (= 2- oder 3- oder 4-Tetrahydropyranyl); 3,4-Dihydro-2H-pyran-2- oder 3- oder 4- oder 5- oder 6-yl; 3,6-Dihydro-2H-pyran-2- oder 3-oder 4- oder 5- oder 6-yl; 2H-Pyran-2- oder 3- oder 4- oder 5- oder 6-yl; 4H-Pyran-2- oder 3- oder 4-yl, 2- oder 3- oder 4-Oxepanyl; 2,3,4,5-Tetrahydrooxepin-2- oder 3- oder 4- oder 5- oder 6- oder 7-yl; 2,3,4,7-Tetrahydrooxepin-2- oder 3- oder 4- oder 5- oder 6- oder 7-yl; 2,3,6,7-Tetrahydrooxepin-2- oder 3- oder 4-yl; 2,3-Dihydrooxepin-2- oder 3- oder 4- oder 5- oder 6- oder 7-yl; 4,5-Dihydrooxepin-2- oder 3- oder 4-yl; 2,5-Dihydrooxepin-2- oder 3- oder 4- oder 5- oder 6- oder 7-yl; Oxepin-2- oder 3- oder 4- oder 5- oder 6- oder 7-yl; 2- oder 3-Tetrahydrothiophenyl; 2,3-Dihydrothiophen-2- oder 3- oder 4- oder 5-yl; 2,5-Dihydrothiophen-2- oder 3-yl; Tetrahydro-2H-thiopyran-2- oder 3- oder 4-yl; 3,4-Dihydro-2H-thiopyran-2- oder 3- oder 4- oder 5- oder 6-yl; 3,6-Dihydro-2H-thiopyran-2- oder 3- oder 4- oder 5- oder 6-yl; 2H-Thiopyran-2- oder 3- oder 4- oder 5- oder 6-yl; 4H-Thiopyran-2- oder 3- oder 4-yl. Bevorzugte 3-Ring und 4-Ring-Heterocyclen sind beispielsweise 1- oder 2-Aziridinyl, Oxiranyl, Thiiranyl, 1- oder 2- oder 3-Azetidinyl, 2- oder 3-Oxetanyl, 2- oder 3-Thietanyl, 1,3-Dioxetan-2-yl. Weitere Beispiele für "Heterocyclyl" sind ein partiell oder vollständig hydrierter heterocyclischer Rest mit zwei Heteroatomen aus der Gruppe N, O und S, wie beispielsweise 1- oder 2- oder 3- oder 4-Pyrazolidinyl; 4,5-Dihydro-3H-pyrazol- 3- oder 4- oder 5-yl; 4,5-Dihydro-1H-pyrazol-1- oder 3- oder 4- oder 5-yl; 2,3-Dihydro-1H-pyrazol-1- oder 2- oder 3- oder 4- oder 5-yl; 1- oder 2- oder 3- oder 4- Imidazolidinyl; 2,3-Dihydro-1H-imidazol-1- oder 2- oder 3- oder 4-yl; 2,5-Dihydro-1H-imidazol-1- oder 2- oder 4- oder 5-yl; 4,5-Dihydro-1H-imidazol-1- oder 2- oder 4- oder 5-yl; Hexahydropyridazin-1- oder 2- oder 3- oder 4-yl; 1,2,3,4-Tetrahydropyridazin-1- oder 2- oder 3- oder 4- oder 5- oder 6-yl; 1,2,3,6-Tetrahydropyridazin-1- oder 2- oder 3- oder 4- oder 5- oder 6-yl; 1,4,5,6-Tetrahydropyridazin-1- oder 3- oder 4- oder 5- oder 6-yl; 3,4,5,6-Tetrahydropyridazin-3- oder 4- oder 5-yl; 4,5-Dihydropyridazin-3- oder 4-yl; 3,4-Dihydropyridazin-3- oder 4- oder 5- oder 6-yl; 3,6-Dihydropyridazin-3- oder 4-yl; 1,6-Dihydropyriazin-1- oder 3- oder 4- oder 5- oder 6-yl; Hexahydropyrimidin-1- oder 2- oder 3- oder 4-yl; 1,4,5,6-Tetrahydropyrimidin-1- oder 2- oder 4- oder 5- oder 6-yl; 1,2,5,6-Tetrahydropyrimidin-1- oder 2- oder 4- oder 5- oder 6-yl; 1,2,3,4-Tetrahydropyrimidin-1- oder 2- oder 3- oder 4- oder 5- oder 6-yl; 1,6-Dihydropyrimidin-1- oder 2- oder 4- oder 5- oder 6-yl; 1,2-Dihydropyrimidin-1- oder 2- oder 4- oder 5- oder 6-yl; 2,5-Dihydropyrimidin-2- oder 4- oder 5-yl; 4,5-Dihydropyrimidin- 4- oder 5- oder 6-yl; 1,4-Dihydropyrimidin-1- oder 2- oder 4- oder 5- oder 6-yl; 1- oder 2- oder 3-Piperazinyl; 1,2,3,6-Tetrahydropyrazin-1- oder 2- oder 3- oder 5- oder 6-yl; 1,2,3,4-Tetrahydropyrazin-1- oder 2- oder 3- oder 4- oder 5- oder 6-yl; 1,2-Dihydropyrazin-1- oder 2- oder 3- oder 5- oder 6-yl; 1,4-Dihydropyrazin-1- oder 2- oder 3-yl; 2,3-Dihydropyrazin-2- oder 3- oder 5- oder 6-yl; 2,5-Dihydropyrazin-2- oder 3-yl; 1,3-Dioxolan-2- oder 4- oder 5-yl; 1,3-Dioxol-2- oder 4-yl; 1,3-Dioxan-2- oder 4- oder 5-yl; 4H-1,3-Dioxin-2- oder 4- oder 5- oder 6-yl; 1,4-Dioxan-2- oder 3- oder 5- oder 6-yl; 2,3-Dihydro-1,4-dioxin-2- oder 3- oder 5- oder 6-yl; 1,4-Dioxin-2- oder 3-yl; 1,2-Dithiolan-3- oder 4-yl; 3H-1,2-Dithiol-3- oder 4- oder 5-yl; 1,3-Dithiolan-2- oder 4-yl; 1,3-Dithiol-2- oder 4-yl; 1,2-Dithian-3- oder 4-yl; 3,4-Dihydro-1,2-dithiin-3- oder 4- oder 5- oder 6-yl; 3,6-Dihydro-1,2-dithiin-3- oder 4-yl; 1,2-Dithiin-3- oder 4-yl; 1,3-Dithian-2- oder 4- oder 5-yl; 4H-1,3-Dithiin-2- oder 4- oder 5- oder 6-yl; Isoxazolidin-2- oder 3- oder 4- oder 5-yl; 2,3-Dihydroisoxazol-2- oder 3- oder 4- oder 5-yl; 2,5-Dihydroisoxazol-2- oder 3- oder 4- oder 5-yl; 4,5-Dihydroisoxazol-3- oder 4- oder 5-yl; 1,3-Oxazolidin-2- oder 3- oder 4- oder 5-yl; 2,3-Dihydro-1,3-oxazol-2- oder 3- oder 4- oder 5-yl; 2,5-Dihydro-1,3-oxazol-2- oder 4- oder 5-yl; 4,5-Dihydro-1,3-oxazol-2- oder 4- oder 5-yl; 1,2-Oxazinan-2- oder 3- oder 4- oder 5- oder 6-yl; 3,4-Dihydro-2H-1,2-oxazin-2- oder 3- oder 4- oder 5- oder 6-yl; 3,6-Dihydro-2H-1,2-oxazin-2- oder 3- oder 4- oder 5- oder 6-yl; 5,6-Dihydro-2H-1,2-oxazin-2- oder 3- oder 4- oder 5- oder 6-yl; 5,6-Dihydro-4H-1,2-oxazin-3- oder 4- oder 5- oder 6-yl; 2H-1,2-Oxazin-2- oder 3- oder 4- oder 5- oder 6-yl; 6H-1,2-Oxazin-3- oder 4- oder 5- oder 6-yl; 4H-1,2-Oxazin-3- oder 4- oder 5- oder 6-yl; 1,3-Oxazinan-2- oder 3- oder 4- oder 5- oder 6-yl; 3,4-Dihydro-2H-1,3-oxazin-2- oder 3- oder 4- oder 5- oder 6-yl; 3,6-Dihydro-2H-1,3-oxazin-2- oder 3- oder 4- oder 5- oder 6-yl; 5,6-Dihydro-2H-1,3-oxazin-2- oder 4- oder 5- oder 6-yl; 5,6-Dihydro-4H-1,3-oxazin-2- oder 4- oder 5- oder 6-yl; 2H-1,3-Oxazin-2- oder 4- oder 5- oder 6-yl; 6H-1,3-Oxazin-2- oder 4- oder 5- oder 6-yl; 4H-1,3-Oxazin-2- oder 4- oder 5- oder 6-yl; Morpholin-2- oder 3- oder 4-yl; 3,4-Dihydro-2H-1,4-oxazin-2- oder 3- oder 4- oder 5- oder 6-yl; 3,6-Dihydro-2H-1,4-oxazin-2- oder 3- oder 5- oder 6-yl; 2H-1,4-oxazin-2- oder 3- oder 5- oder 6-yl; 4H-1,4-oxazin-2- oder 3-yl; 1,2-Oxazepan-2- oder 3- oder 4- oder 5- oder 6- oder 7-yl; 2,3,4,5-Tetrahydro-1,2-oxazepin-2- oder 3- oder 4- oder 5- oder 6- oder 7-yl; 2,3,4,7-Tetrahydro-1,2-oxazepin-2- oder 3- oder 4- oder 5- oder 6- oder 7-yl; 2,3,6,7-Tetrahydro-1,2-oxazepin-2- oder 3- oder 4- oder 5- oder 6- oder 7-yl; 2,5,6,7-Tetrahydro-1,2-oxazepin-2- oder 3- oder 4- oder 5- oder 6- oder 7-yl; 4,5,6,7-Tetrahydro-1,2-oxazepin-3- oder 4- oder 5- oder 6- oder 7-yl; 2,3-Dihydro-1,2-oxazepin-2- oder 3- oder 4- oder 5- oder 6- oder 7-yl; 2,5-Dihydro-1,2-oxazepin-2- oder 3- oder 4- oder 5- oder 6- oder 7-yl; 2,7-Dihydro-1,2-oxazepin-2- oder 3- oder 4- oder 5- oder 6- oder 7-yl; 4,5-Dihydro-1,2-oxazepin-3- oder 4- oder 5- oder 6- oder 7-yl; 4,7-Dihydro-1,2-oxazepin-3- oder 4- oder 5- oder 6- oder 7-yl; 6,7-Dihydro-1,2-oxazepin-3- oder 4- oder 5- oder 6- oder 7-yl; 1,2-Oxazepin-3- oder 4- oder 5- oder 6- oder 7-yl; 1,3-Oxazepan-2- oder 3- oder 4- oder 5- oder 6- oder 7-yl; 2,3,4,5-Tetrahydro-1,3-oxazepin-2- oder 3- oder 4- oder 5- oder 6- oder 7-yl; 2,3,4,7-Tetrahydro-1,3-oxazepin-2- oder 3- oder 4- oder 5- oder 6- oder 7-yl; 2,3,6,7-Tetrahydro-1,3-oxazepin-2- oder 3- oder 4- oder 5- oder 6- oder 7-yl; 2,5,6,7-Tetrahydro-1,3-oxazepin-2- oder 4- oder 5- oder 6- oder 7-yl; 4,5,6,7-Tetrahydro-1,3-oxazepin-2- oder 4- oder 5- oder 6- oder 7-yl; 2,3-Dihydro-1,3-oxazepin-2- oder 3- oder 4- oder 5- oder 6- oder 7-yl; 2,5-Dihydro-1,3-oxazepin-2- oder 4- oder 5- oder 6- oder 7-yl; 2,7-Dihydro-1,3-oxazepin-2- oder 4- oder 5- oder 6- oder 7-yl; 4,5-Dihydro-1,3-oxazepin-2- oder 4- oder 5- oder 6- oder 7-yl; 4,7-Dihydro-1,3-oxazepin-2- oder 4- oder 5- oder 6- oder 7-yl; 6,7-Dihydro-1,3-oxazepin-2- oder 4- oder 5- oder 6- oder 7-yl; 1,3-Oxazepin-2- oder 4- oder 5- oder 6- oder 7-yl; 1,4-Oxazepan-2- oder 3- oder 5- oder 6- oder 7-yl; 2,3,4,5-Tetrahydro-1,4-oxazepin-2- oder 3- oder 4- oder 5- oder 6- oder 7-yl; 2,3,4,7-Tetrahydro-1,4-oxazepin-2- oder 3- oder 4- oder 5- oder 6- oder 7-yl; 2,3,6,7-Tetrahydro-1,4-oxazepin-2- oder 3- oder 5- oder 6- oder 7-yl; 2,5,6,7-Tetrahydro-1,4-oxazepin-2- oder 3- oder 5- oder 6- oder 7-yl; 4,5,6,7-Tetrahydro-1,4-oxazepin-2- oder 3- oder 4- oder 5- oder 6- oder 7-yl; 2,3-Dihydro-1,4-oxazepin-2- oder 3- oder 5- oder 6- oder 7-yl; 2,5-Dihydro-1,4-oxazepin-2- oder 3- oder 5- oder 6- oder 7-yl; 2,7-Dihydro-1,4-oxazepin-2- oder 3- oder 5- oder 6- oder 7-yl; 4,5-Dihydro-1,4-oxazepin-2- oder 3- oder 4- oder 5- oder 6- oder 7-yl; 4,7-Dihydro-1,4-oxazepin-2- oder 3- oder 4- oder 5- oder 6- oder 7-yl; 6,7-Dihydro-1,4-oxazepin-2- oder 3- oder 5- oder 6- oder 7-yl; 1,4-Oxazepin-2- oder 3- oder 5- oder 6- oder 7-yl; Isothiazolidin-2- oder 3- oder 4- oder 5-yl; 2,3-Dihydroisothiazol-2- oder 3- oder 4- oder 5-yl; 2,5-Dihydroisothiazol-2- oder 3- oder 4- oder 5-yl; 4,5-Dihydroisothiazol-3- oder 4- oder 5-yl; 1,3-Thiazolidin-2- oder 3- oder 4- oder 5-yl; 2,3-Dihydro-1,3-thiazol-2- oder 3- oder 4- oder 5-yl; 2,5-Dihydro-1,3-thiazol-2- oder 4- oder 5-yl; 4,5-Dihydro-1,3-thiazol-2- oder 4- oder 5-yl; 1,3-Thiazinan-2- oder 3- oder 4- oder 5- oder 6-yl; 3,4-Dihydro-2H-1,3-thiazin-2- oder 3- oder 4- oder 5- oder 6-yl; 3,6-Dihydro-2H-1,3-thiazin-2- oder 3- oder 4- oder 5- oder 6-yl; 5,6-Dihydro-2H-1,3-thiazin-2- oder 4- oder 5- oder 6-yl; 5,6-Dihydro-4H-1,3-thiazin-2- oder 4- oder 5- oder 6-yl; 2H-1,3-Thiazin-2- oder 4- oder 5- oder 6-yl; 6H-1,3-Thiazin-2- oder 4- oder 5- oder 6-yl; 4H-1,3-Thiazin-2- oder 4- oder 5- oder 6-yl. Weitere Beispiele für "Heterocyclyl" sind ein partiell oder vollständig hydrierter heterocyclischer Rest mit 3 Heteroatomen aus der Gruppe N, O und S, wie beispielsweise 1,4,2-Dioxazolidin-2- oder 3- oder 5-yl; 1,4,2-Dioxazol-3- oder 5-yl; 1,4,2-Dioxazinan-2- oder -3- oder 5- oder 6-yl; 5,6-Dihydro-1,4,2-dioxazin-3- oder 5- oder 6-yl; 1,4,2-Dioxazin-3- oder 5- oder 6-yl; 1,4,2-Dioxazepan-2- oder 3- oder 5- oder 6- oder 7-yl; 6,7-Dihydro-5H-1,4,2-Dioxazepin-3- oder 5- oder 6- oder 7-yl; 2,3-Dihydro-7H-1,4,2-Dioxazepin-2- oder 3- oder 5- oder 6- oder 7-yl; 2,3-Dihydro-5H-1,4,2-Dioxazepin-2- oder 3- oder 5- oder 6- oder 7-yl; 5H-1,4,2-Dioxazepin-3- oder 5- oder 6- oder 7-yl; 7H-1,4,2-Dioxazepin-3- oder 5- oder 6- oder 7-yl. Strukturbeispiele für gegebenenfalls weiter substituierte Heterocyclen sind auch im Folgenden aufgeführt:

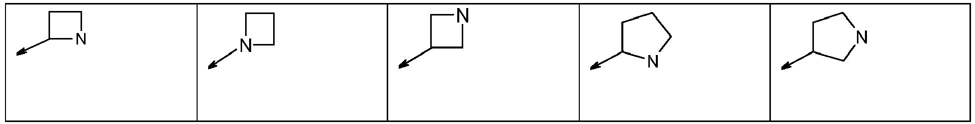

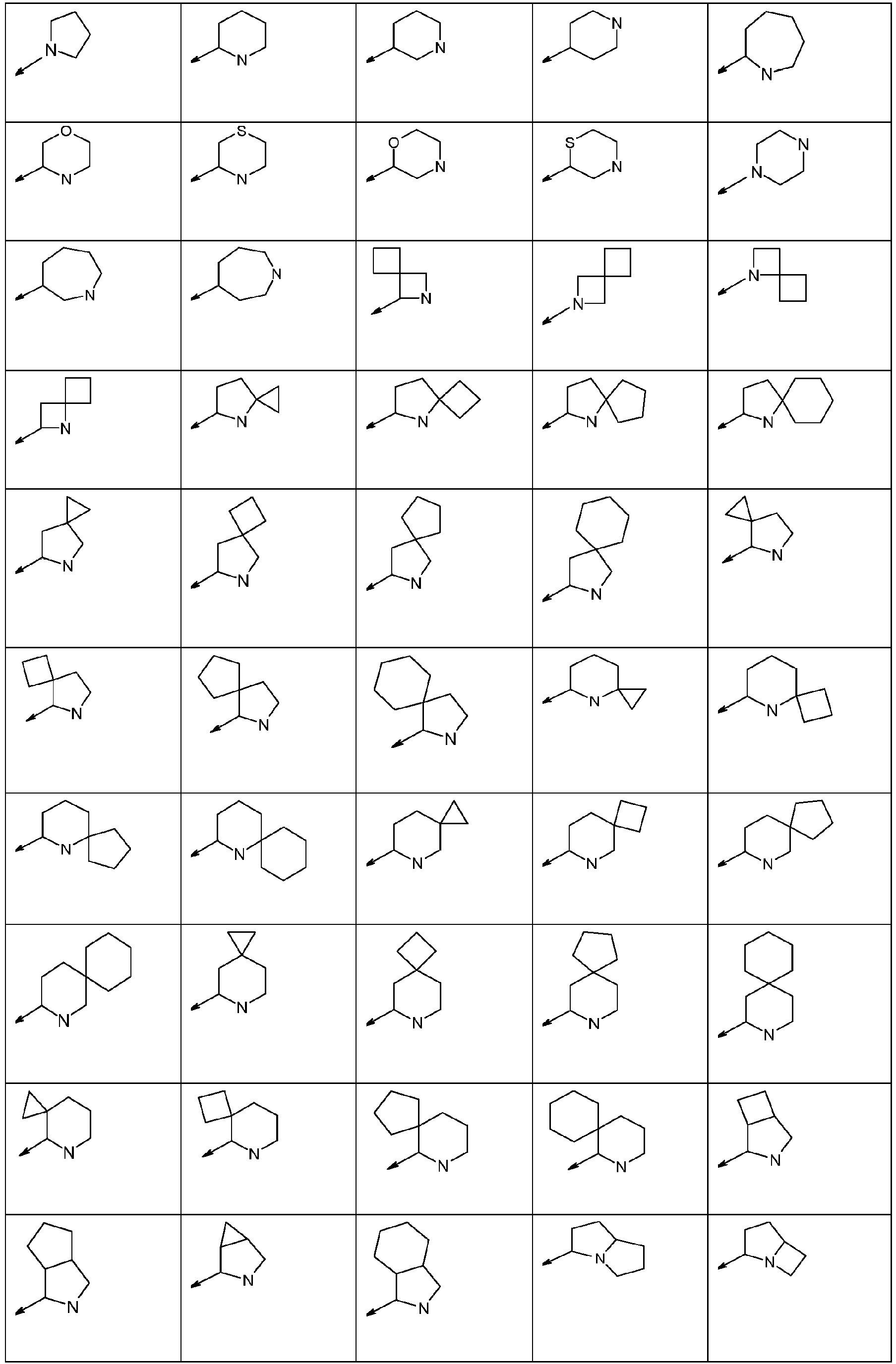

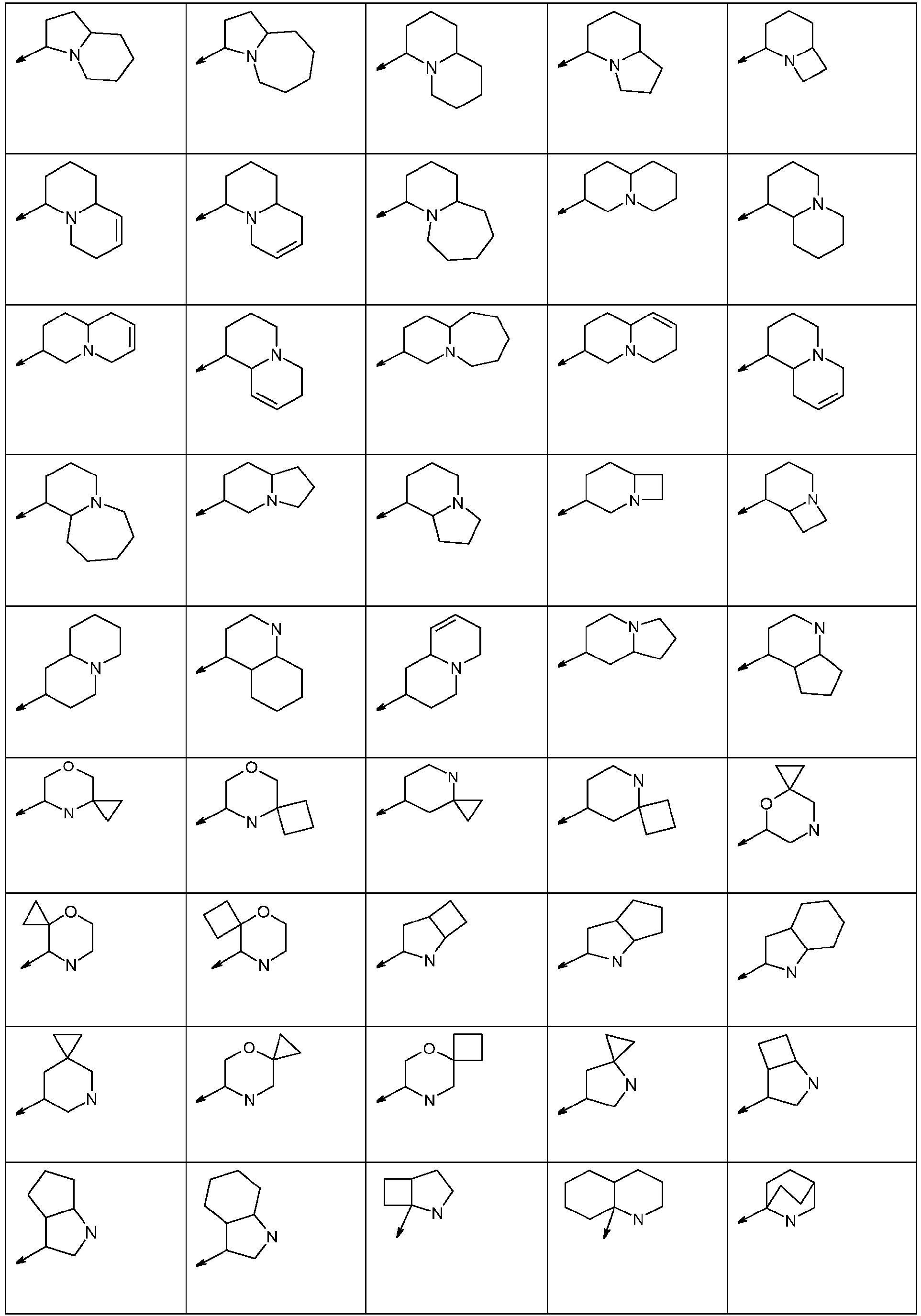

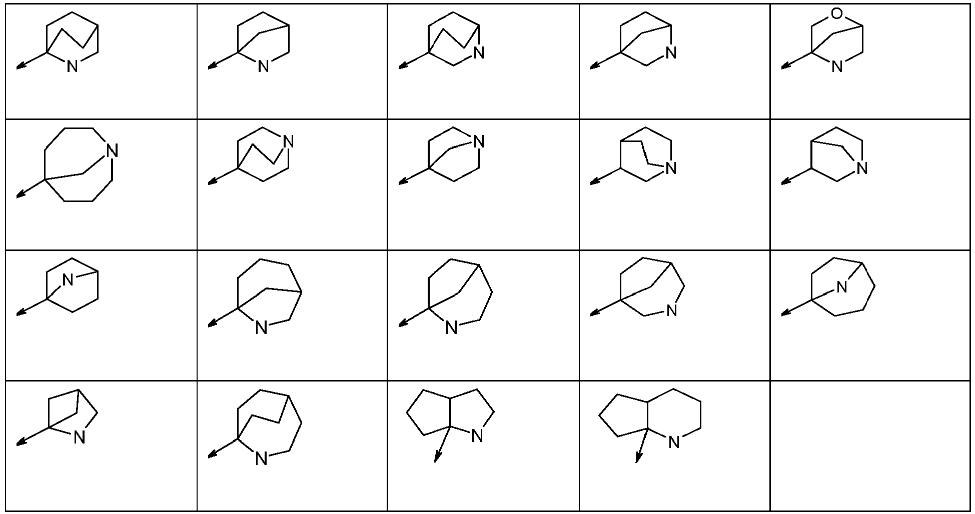

Die oben aufgeführten Heterocyclen sind bevorzugt beispielsweise durch Wasserstoff, Halogen, Alkyl, Haloalkyl, Hydroxy, Alkoxy, Cycloalkoxy, Aryloxy, Alkoxyalkyl, Alkoxyalkoxy, Cycloalkyl, Halocycloalkyl, Aryl, Arylalkyl, Heteroaryl, Heterocyclyl, Alkenyl, Alkylcarbonyl, Cycloalkylcarbonyl, Arylcarbonyl, Heteroarylcarbonyl, Alkoxycarbonyl, Hydroxycarbonyl, Cycloalkoxycarbonyl, Cycloalkylalkoxycarbonyl, Alkoxycarbonylalkyl, Arylalkoxycarbonyl, Arylalkoxycarbonylalkyl, Alkinyl, Alkinylalkyl, Alkylalkinyl, Tris-alkylsilylalkinyl, Nitro, Amino, Cyano, Haloalkoxy, Haloalkylthio, Alkylthio, Hydrothio, Hydroxyalkyl, Oxo, Heteroarylalkoxy, Arylalkoxy, Heterocyclylalkoxy, Heterocyclylalkylthio, Heterocyclyloxy, Heterocyclylthio, Heteroaryloxy, Bis-alkylamino, Alkylamino, Cycloalkylamino, Hydroxycarbonylalkylamino, Alkoxycarbonylalkylamino, Arylalkoxycarbonylalkylamino, Alkoxycarbonylalkyl(alkyl)amino, Aminocarbonyl, Alkylaminocarbonyl, Bis-alkylaminocarbonyl, Cycloalkylaminocarbonyl, Hydroxycarbonylalkylaminocarbonyl, Alkoxycarbonylalkylaminocarbonyl, Arylalkoxycarbonylalkylaminocarbonyl substituiert.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Handelt es sich es sich um einen teilweise oder vollständig gesättigten Stickstoff-Heterocyclus, so kann dieser sowohl über Kohlenstoff als auch über den Stickstoff mit dem Rest des Moleküls verknüpft sein.

Als Substituenten für einen substituierten heterocyclischen Rest kommen die weiter unten genannten Substituenten in Frage, zusätzlich auch Oxo und Thioxo. Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen N(O) , S(O) (auch kurz SO) und S(O)₂ (auch kurz SO₂) im heterocyclischen Ring. Im Fall von -N(O)- und -S(O)-Gruppen sind jeweils beide Enantiomere umfasst.

Erfindungsgemäß steht der Ausdruck "Heteroaryl" für heteroaromatische Verbindungen, d. h. vollständig ungesättigte aromatische heterocyclische Verbindungen, vorzugsweise für 5- bis 7-gliedrige Ringe mit 1 bis 4, vorzugsweise 1 oder 2 gleichen oder verschiedenen Heteroatomen, vorzugsweise O, S oder N. Erfindungsgemäße Heteroaryle sind beispielsweise 1H-Pyrrol-1-yl; 1H-Pyrrol-2-yl; 1H-Pyrrol-3-yl; Furan-2-yl; Furan-3-yl; Thien-2-yl; Thien-3-yl, 1H-Imidazol-1-yl; 1H-Imidazol-2-yl; 1H-Imidazol-4-yl; 1H-Imidazol-5-yl; 1H-Pyrazol-1-yl; 1H-Pyrazol-3-yl; 1H-Pyrazol-4-yl; 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-3-yl, 4H-1,2,4-Triazol-4-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, Azepinyl, Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, Oxepinyl, Thiepinyl, 1,2,4-Triazolonyl und 1,2,4-Diazepinyl, 2H-1,2,3,4-Tetrazol-5-yl, 1H-1,2,3,4-Tetrazol-5-yl, 1,2,3,4-Oxatriazol-5-yl, 1,2,3,4-Thiatriazol-5-yl, 1,2,3,5-Oxatriazol-4-yl, 1,2,3,5-Thiatriazol-4-yl. Die erfindungsgemäßen Heteroarylgruppen können ferner mit einem oder mehreren, gleichen oder verschiedenen Resten substituiert sein. Sind zwei benachbarte Kohlenstoffatome Bestandteil eines weiteren aromatischen Rings, so handelt es sich um annellierte heteroaromatische Systeme, wie benzokondensierte oder mehrfach annellierte Heteroaromaten. Bevorzugt sind beispielsweise Chinoline (z. B. Chinolin-2-yl, Chinolin-3-yl, Chinolin-4-yl, Chinolin-5-yl, Chinolin-6-yl, Chinolin-7-yl, Chinolin-8-yl); Isochinoline (z. B. Isochinolin-1-yl, Isochinolin-3-yl, Isochinolin-4-yl, Isochinolin-5-yl, Isochinolin-6-yl, Isochinolin-7-yl, Isochinolin-8-yl); Chinoxalin; Chinazolin; Cinnolin; 1,5-Naphthyridin; 1,6-Naphthyridin; 1,7-Naphthyridin; 1,8-Naphthyridin; 2,6-Naphthyridin; 2,7-Naphthyridin; Phthalazin; Pyridopyrazine; Pyridopyrimidine; Pyridopyridazine; Pteridine; Pyrimidopyrimidine. Beispiele für Heteroaryl sind auch 5- oder 6-gliedrige benzokondensierte Ringe aus der Gruppe 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 2H-Indazol-3-yl, 2H-Indazol-4-yl, 2H-Indazol-5-yl, 2H-Indazol-6-yl, 2H-Indazol-7-yl, 2H-Isoindol-2-yl, 2H-Isoindol-1-yl, 2H-Isoindol-3-yl, 2H-Isoindol-4-yl, 2H-Isoindol-5-yl, 2H-Isoindol-6-yl; 2H-Isoindol-7-yl, 1H-Benzimidazol-1-yl, 1H-Benzimidazol-2-yl, 1H-Benzimidazol-4-yl, 1H-Benzimidazol-5-yl, 1H-Benzimidazol-6-yl, 1H-Benzimidazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, 1,3-Benzthiazol-2-yl, 1,3-Benzthiazol-4-yl, 1,3-Benzthiazol-5-yl, 1,3-Benzthiazol-6-yl, 1,3-Benzthiazol-7-yl, 1,2-Benzisoxazol-3-yl, 1,2-Benzisoxazol-4-yl, 1,2-Benzisoxazol-5-yl, 1,2-Benzisoxazol-6-yl, 1,2-Benzisoxazol-7-yl, 1,2-Benzisothiazol-3-yl, 1,2-Benzisothiazol-4-yl, 1,2-Benzisothiazol-5-yl, 1,2-Benzisothiazol-6-yl, 1,2-Benzisothiazol-7-yl.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder Iodatom.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist und im letzteren Falle als "substituiertes Alkyl" bezeichnet wird. Bevorzugte Substituenten sind Halogenatome, Alkoxy-, Haloalkoxy-, Cyano-, Alkylthio, Haloalkylthio-, Amino- oder Nitrogruppen, besonders bevorzugt sind Methoxy, Methyl, Fluoralkyl, Cyano, Nitro, Fluor, Chlor, Brom oder Iod. Die Vorsilbe "Bis" schließt auch die Kombination unterschiedlicher Alkylreste ein, z. B. Methyl(Ethyl) oder Ethyl(Methyl).

"Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie z. B. CH₂CH₂Cl, CH₂CH₂Br, CHClCH₃, CH₂Cl, CH₂F; Perhaloalkyl wie z. B. CCl₃, CClF₂, CFCl₂,CF₂CClF₂, CF₂CClFCF₃; Polyhaloalkyl wie z. B. CH₂CHFCl, CF₂CClFH, CF₂CBrFH, CH₂CF₃; Der Begriff Perhaloalkyl umfasst dabei auch den Begriff Perfluoralkyl.

"Teilfluoriertes Alkyl" bedeutet einen geradkettigen oder verzweigten, gesättigten Kohlenwasserstoff, der einfach oder mehrfach durch Fluor substituiert ist, wobei sich die entsprechenden Fluoratome als Substituenten an einem oder mehreren verschiedenen Kohlenstoffatomen der geradkettigen oder verzweigten Kohlenwasserstoffkette befinden können, wie z. B. CHFCH₃, CH₂CH₂F, CH₂CH₂CF₃, CHF₂, CH₂F, CHFCF₂CF₃

"Teilfluoriertes Haloalkyl" bedeutet einen geradkettigen oder verzweigten, gesättigten Kohlenwasserstoff, der durch verschiedenene Halogenatomen mit mindestens einem Fluoratom substituiert ist, wobei alle anderen gegebenenfalls vorhandenen Halogenatome ausgewählt sind aus der Gruppe Fluor, Chlor oder Brom, Iod. Die entsprechenden Halogenatome können sich dabei als Substituenten an einem oder mehreren verschiedenen Kohlenstoffatomen der geradkettigen oder verzweigten Kohlenwasserstoffkette befinden. Teilfluoriertes Haloalkyl schließt auch die vollständige Substitution der geradkettigen oder verzweigten Kette durch Halogen unter Beteiligung von mindestens einem Fluoratom ein.

"Haloalkoxy" ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Der hier beispielhaft genannte Ausdruck "(C₁-C₄)-Alkyl" bedeutet eine Kurzschreibweise für geradkettiges oder verzweigtes Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)-Alkyl", umfassen entsprechend auch geradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung.

Der Begriff "Alkenyl" schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl. Alkenyl bedeutet z.B. Vinyl, welches ggf. durch weitere Alkylreste substituiert sein kann, z B. (aber nicht beschränkt auf) (C₂-C₆)-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Der Begriff "Alkinyl" schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl. (C₂-C₆)-Alkinyl bedeutet z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Di-methyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Der Begriff "Cycloalkyl" bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl, das gegebenenfalls weiter substituiert ist, bevorzugt durch Wasserstoff, Alkyl, Alkoxy, Cyano, Nitro, Alkylthio, Haloalkylthio, Halogen, Alkenyl, Alkinyl, Haloalkyl, AMino, Alkylamino, Bisalkylamino, Alkocycarbonyl, Hydroxycarbonyl, Arylalkoxycarbonyl, Aminocarbonyl, Alkylaminocarbonyl, Cycloalkylaminocarbonyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfasst, wie beispielsweise Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl und Adamantan-2-yl, aber auch Systeme wie z. B. 1,1'-Bi(cyclopropyl)-1-yl, 1,1'-Bi(cyclopropyl)-2-yl. Der Ausdruck "(C₃-C₇)-Cycloalkyl" bedeutet eine Kurzschreibweise für Cycloalkyl mit drei bis 7 Kohlenstoffatomen entsprechend der Bereichsangabe für C-Atome.

Im Falle von substituiertem Cycloalkyl werden auch spirocyclische aliphatische Systeme umfasst, wie beispielsweise Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl.

"Cycloalkenyl" bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Der Begriff "Alkyliden", z. B. auch in der Form (C₁-C₁₀)-Alkyliden, bedeutet den Rest eines geradkettigen oder verzweigten offenkettigen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H_{5.} Cycloalkyliden bedeutet ein carbocyclischer Rest, der über eine Zweifachbindung gebunden ist.

"Alkoxyalkyl" steht für einen über eine Alkylgruppe gebundenen Alkoxyrest und "Alkoxyalkoxy" bedeutet einen über ein Sauerstoffatom gebundenen Alkoxyalkylrest, z.B. (aber nicht beschränkt auf) Methoxymethoxy, Methoxyethoxy, Ethoxyethoxy, Methoxy-n-propyloxy.

"Alkylthioalkyl" steht für einen über eine Alkylgruppe gebundenen Alkylthiorest und "Alkylthioalkylthio" bedeutet einen über ein Sauerstoffatom gebundenen Alkylthioalkylrest.

"Arylalkoxyalkyl" steht für einen über eine Alkylgruppe gebundenen Aryloxyrest und "Heteroaryloxyalkyl" bedeutet einen über eine Alkylgruppe gebundenen Heteroaryloxyrest.

"Haloalkoxyalkyl" steht für einen gebundenen Haloalkoxyrest und "Haloalkylthioalkyl" bedeutet einen über eine Alkylgruppe gebundenen Haloalkylthiorest.

"Arylalkyl" steht für einen über eine Alkylgruppe gebundenen Arylrest, "Heteroarylalkyl" bedeutet einen über eine Alkylgruppe gebundenen Heteroarylrest, und "Heterocyclylalkyl" bedeutet einen über eine Alkylgruppe gebundenen Heterocyclylrest.

"Cycloalkylalkyl" steht für einen über eine Alkylgruppe gebundenen Cycloalkylrest, z. B. (aber nicht beschränkt auf) Cyclopropylmethyl, Cyclobutylmethyl, Cyclopentylmethyl, Cyclohexylmethyl, 1-Cyclopropyleth-1-yl, 2-Cyclopropyleth-1-yl, 1-Cyclopropylprop-1-yl, 3-Cyclopropylprop-1-yl.

Erfindungsgemäß steht "Haloalkylthio" - in Alleinstellung oder als Bestandteil einer chemischen Gruppe - für geradkettiges oder verzweigtes S-Halogenalkyl, vorzugsweise mit 1 bis 8, oder mit 1 bis 6 Kohlenstoffatomen, wie (C₁-C₈)-, (C₁-C₆)- oder (C₁-C₄)-Haloalkylthio, z.B. (aber nicht beschränkt auf) Trifluormethylthio, Pentafluorethylthio, Difluormethyl, 2,2-Difluoreth-1-ylthio, 2,2,2-Difluoreth-1-ylthio, 3,3,3-prop-1-ylthio.

"Halocycloalkyl" und "Halocycloalkenyl" bedeuten durch gleiche oder verschiedene Halogenatome, wie z. B. F, Cl und Br, oder durch Haloalkyl, wie z. B. Trifluormethyl oder Difluormethyl teilweise oder vollständig substituiertes Cycloalkyl oder Cycloalkenyl , z.B. 1-Fluorcycloprop-1-yl, 2-Fluorcycloprop-1-yl, 2,2-Difluorcycloprop-1-yl, 1-Fluorcyclobut-1-yl, 1-Trifluormethylcycloprop-1-yl, 2-Trifluormethylcycloprop-1-yl, 1-Chlor-cycloprop-1-yl, 2-Chlorcycloprop-1-yl, 2,2-Dichlorcycloprop-1-yl, 3,3-Difluorcyclobutyl,

Erfindungsgemäß steht "Trialkylsilyl" - in Alleinstellung oder als Bestandteil einer chemischen Gruppe - für geradkettiges oder verzweigtes Si-Alkyl, vorzugsweise mit 1 bis 8, oder mit 1 bis 6 Kohlenstoffatomen, wie Tri-[(C₁-C₈)-, (C₁-C₆)- oder (C₁-C₄)-alkyl]silyl, z.B. (aber nicht beschränkt auf) Trimethylsilyl, Triethylsilyl, Tri-(n-propyl)silyl, Tri-(iso-propyl)silyl, Tri-(n-butyl)silyl, Tri-(1-methylprop-1-yl)silyl, Tri-(2-methylprop-1-yl)silyl, Tri(1, 1-Dimethyleth-1-yl)silyl, Tri(2,2-Dimethyleth-1-yl)silyl.

Wenn die Verbindungen durch Wasserstoffverschiebung Tautomere bilden können, welche strukturell formal nicht durch die allgemeine Formel **(I)** erfasst würden, so sind diese Tautomere gleichwohl von der Definition der erfindungsgemäßen Verbindungen der allgemeinen Formel **(I)** umfasst, sofern nicht ein bestimmtes Tautomer Gegenstand der Betrachtung ist. So können beispielsweise viele Carbonylverbindungen sowohl in der Ketoform wie auch in der Enolform vorliegen, wobei beide Formen durch die Definition der Verbindung der allgemeinen Formel **(I)** umfasst werden.

Die Verbindungen der allgemeinen Formel **(I)** können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der allgemeinen Formel (I) umfasst. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden erhalten. Die chromatographische Trennung kann sowohl im analytischen Maßstab zur Feststellung des Enantiomerenüberschusses bzw. des Diastereomerenüberschusses, wie auch im präparativen Maßstab zur Herstellung von Prüfmustern für die biologische Ausprüfung erfolgen. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel **(I)** umfasst, jedoch nicht mit ihrer spezifischen Stereoform angegeben sind, sowie deren Gemische.

Sofern die Verbindungen als Feststoffe erhalten werden, kann die Reinigung auch durch Umkristallisieren oder Digerieren erfolgen. Sofern einzelne Verbindungen **(I)** nicht auf den nachstehend beschriebenen Wegen zufriedenstellend zugänglich sind, können sie durch Derivatisierung anderer Verbindungen **(I)** hergestellt werden.

Als Isolierungs-, Reinigungs- und Stereoisomerenauftrennungsverfahren von Verbindungen der allgemeinen Formel **(I)** kommen Methoden in Frage, die dem Fachmann aus analogen Fällen allgemein bekannt sind, z.B. durch physikalische Verfahren wie Kristallisation, Chromatographieverfahren, vor allem Säulenchromatographie und HPLC (Hochdruckflüssigchromatographie), Destillation, gegebenenfalls unter reduziertem Druck, Extraktion und andere Verfahren, können gegebenfalls verbleibende Gemische in der Regel durch chromatographische Trennung, z.B. an chiralen Festphasen, getrennt werden. Für präparative Mengen oder im industriellen Maßstab kommen Verfahren in Frage wie Kristallisation, z.B. diastereomerer Salze, die aus den Diastereomerengemischen mit optisch aktiven Säuren und gegebenenfalls bei vorhandenen sauren Gruppen mit optisch aktiven Basen erhalten werden können.

Synthese von substituierten Pyrazole der allgemeinen Formel **(I)**

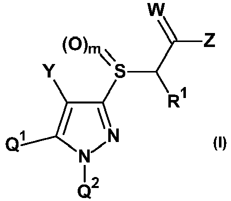

Die erfindungsgemäßen substituierten Pyrazole der allgemeinen Formel (**I**) können ausgehend von bekannten Verfahren hergestellt werden. Die eingesetzten und untersuchten Syntheserouten gehen dabei von kommerziell erhältlichen oder leicht herstellbaren Aminen, von entsprechend substituierten Aldehyden und von kommerziell erhältlichen Chemikalien wie Malonsäurederivaten und Nitromethan aus. Die Gruppierungen Q¹, Q², Y, W, R¹, R², R³, R⁴, R⁵, R⁶, R⁷, R⁸, R⁹, m, n und o der allgemeinen Formel (**I**) haben in den nachfolgenden Schemata die zuvor definierten Bedeutungen, sofern nicht beispielhafte, aber nicht einschränkende, Definitionen erfolgen.

Die Synthese der erfindungsgemäßen Verbindungen der allgemeinen Formel (**Ia**) erfolgt über eine Amidkupplung von einer Säure der allgemeinen Formel (**II**) mit einem Amin der allgemeinen Formel (**III**) in Gegenwart eines Amidkupplungsreagenzes wie zum Beispiel T3P, Dicyclohexylcarbodiimid, *N-*(3-Dimethylaminopropyl)-*N'*-ethylcarbodiimid, *N*,*N'*-Cabonyldiimidazol, 2-Chlor-1,3-dimethyl-imidazolium chlorid oder 2-Chlor-1-methylpyridinium iodid (siehe Chemistry of Peptide Synthsis, Ed. N. Leo Benoiton, Taylor & Francis, 2006, ISBN-10: 1-57444-454-9). Polymergebundene Reagenzien wie zum Beispiel polymergebundenes Dicyclohexylcarbodiimid sind auch für diese Kupplungs-reaktion geeignet. Die Reaktion findet bevorzugt in dem Temperaturbereich zwischen 0 °C und 80 °C, in einem adäquaten Lösungsmittel wie zum Beispiel Dichlormethan, Acetonitril, *N,N*-Dimethyl-formamid oder Ethylacetat und in Gegenwart eine Base wie zum Beispiel Triethylamin, *N*,*N*-Diisopropylethylamin oder 1,8-Diazabicyclo[5.4.0]undec-7-cen statt (siehe Schema 1). Für die T3P Peptidkupplungsbedingungen siehe Organic Process Research & Development 2009, 13, 900-906.

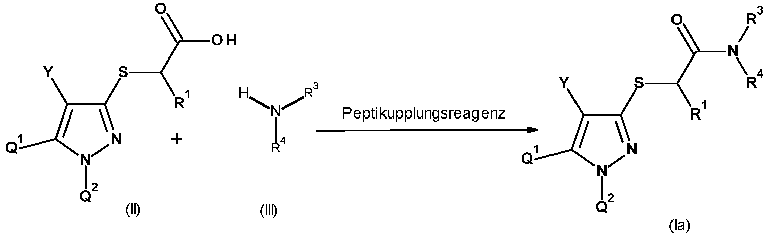

### Schema 1

Die Synthese der Säure der allgemeinen Formel (**II**) lässt sich durch Verseifung der Verbindung der allgemeinen Formel (**IV**) nach oder lassen sich analog dem Fachmann bekannten Methoden herstellen (Schema 2). Die Verseifung lässt sich in Gegenwart einer Base oder einer Lewis-Säure durchführen. Die Base kann ein Hydroxid-Salz von einem Alkali-Metall (wie zum Beispiel Lithium, Natrium oder Kalium) sein, und die Verseifungsreaktion findet bevorzugt in dem Temperaturbereich zwischen Raumtemperatur und 120 °C statt. 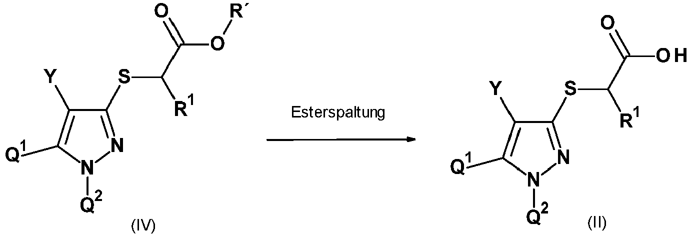 Mit R' = (C₁-C₄)-Alkyl.

### Schema 2

Die Synthese der Verbindung der allgemeinen Formel (**IV**) lässt sich durch Alkylierung der Verbindung der allgemeinen Formel (**V**) mit einem Halogenid der allgemeinen Formel (**VI**) in Gegenwart einer Base nach oder lassen sich analog dem Fachmann bekannten Methoden herstellen (siehe Schema 3). Die Base kann ein Carbonat-Salz von einem Alkali-Metall (wie zum Beispiel Lithium, Natrium, Kalium oder Caesium) sein, und die Verseifungsreaktion findet bevorzugt in dem Temperaturbereich zwischen Raumtemperatur und 150 °C in einem adäquaten Lösungsmittel wie zum Beispiel Dichlormethan, Acetonitril, *N,N*-Dimethylformamid oder Ethylacetat statt. Siehe J. Med. Chem. 2011, 54(16), 5820-5835 und WO2010/010154. 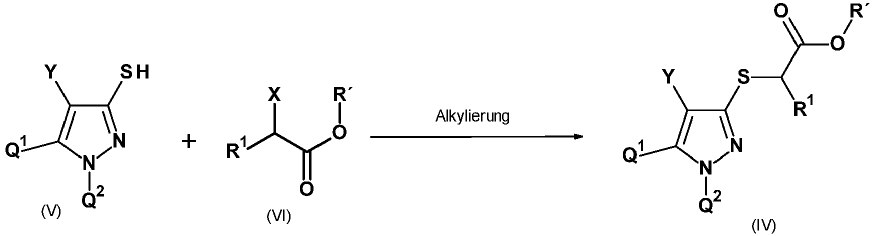
Mit R' = (C₁-C₄)-Alkyl.
Mit X = Halogen.

### Schema 3

In Schema 4 wird die Synthese der Verbindung der allgemeinen Formel (**V**) durch Reaktion eines Pyrazolones der allgemeinen Formel (**VII**) in Gegenwart eines Schwefelungsreagenzes wie zum Beispiel Phosphorpentasulfid oder Lawesson-Reagenz in einem adäquaten Lösungsmittel wie zum Beispiel Toluol.

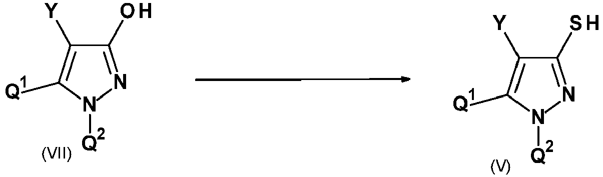

### Schema 4

In Schema 5 wird die Synthese der Verbindung der allgemeinen Formel (**VII**) durch Reaktion eines Pyrazoles der allgemeinen Formel (**VIII**) mit einem Halogensuccinimid der allgemeinen Formel (**IX**) in einem adäquaten Lösungsmittel wie zum Beispiel DMF beschrieben. 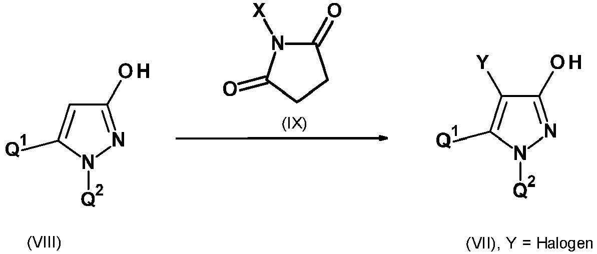 Mit X = Halogen.

### Schema 5

Die 3-Hydroxypyrazole (**VIII**) können analog literaturbekannter Methoden aus substituierten 3-Azinylpropinsäurederivaten und Phenyhydrazinen (Schema 8; z. B.: Adv. Synth. Catal. 2014, 356, 3135-3147) oder aus substituierten Azinylacrylsäurederivaten und Phenylhydrazinen (Schema 8; z. B.: J. Heterocyclic Chem., 49, 130 (2012)) hergestellt werden.

Die Synthese der Verbindungen der allgemeinen Formel (**X**) erfolgt über eine Amidkupplung von einer Säure der allgemeinen Formel (**XII**) mit einem Arylhydrazin oder Hetarylhydrazin der allgemeinen Formel (**XI**) in Gegenwart eines Amidkupplungsreagenzes wie zum Beispiel T3P, Dicyclohexylcarbodiimid, *N*-(3-Dimethylaminopropyl)-*N'*-ethylcarbodiimid, *N*,*N'*-Cabonyldiimidazol, 2-Chlor-1,3-dimethyl-imidazolium chlorid oder 2-Chlor-1-methylpyridinium iodid (siehe Chemistry of Peptide Synthesis, Ed. N. Leo Benoiton, Taylor & Francis, 2006, ISBN-10: 1-57444-454-9). Polymergebundene Reagenzien wie zum Beispiel polymergebundenes Dicyclohexylcarbodiimid sind auch für diese Kupplungsreaktion geeignet. Die Reaktion findet bevorzugt in dem Temperaturbereich zwischen 0 °C und 80 °C, in einem adäquaten Lösungsmittel wie zum Beispiel Dichlormethan, Tetrahydrofuran, Acetonitril, *N*,*N-*Dimethylformamid oder Ethylacetat und in Gegenwart eine Base wie zum Beispiel Triethylamin, *N*,*N*-Diisopropylethylamin oder 1,8-Diazabicyclo[5.4.0]undec-7-cen statt (siehe Schema 8). Für die T3P Peptidkupplungsbedingungen siehe Organic Process Research & Development 2009,13, 900-906.

Die Synthese der 3-Hydroxypyrazole der allgemeinen Formel (**VIII**) erfolgt durch Reaktion der Verbindungen der allgemeinen Formel (**X**) in Gegenwart eines Kupferhalogenides wie zum Beispiel Kupfer(I)iodid, Kupfer(I)bromid oder einer Säure wie Methansulfonsäure. Die Reaktion findet bevorzugt in dem Temperaturbereich zwischen 0 °C und 120 °C, in einem adäquaten Lösungsmittel wie zum Beispiel 1,2-Dichlorethan, Acetonitril, *N,N*-Dimethylformamid, n-Propanol oder Ethylacetat statt. Die Reaktion findet bevorzugt in *N,N*-Dimethylformamid statt.

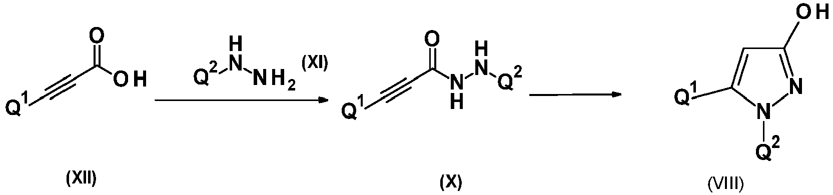

### Schema 6

Alternativ lassen sich Verbindungen der allgemeinen Formel (**VIII**) durch eine eine Amidkupplung von einer Säure der allgemeinen Formel (**XIV**) mit einem Arylhydrazin oder Hetarylhydrazin der allgemeinen Formel (**XI**) in Gegenwart eines Amidkupplungsreagenzes wie zum Beispiel T3P, Dicyclohexylcarbodiimid, *N-*(3-Dimethylaminopropyl)-*N'*-ethylcarbodiimid, *N*,*N'*-Cabonyldiimidazol, 2-Chlor-1,3-dimethyl-imidazolium chlorid oder 2-Chlor-1-methylpyridinium iodid. Die Reaktion findet bevorzugt in dem Temperaturbereich zwischen 0 °C und 80 °C, in einem adäquaten Lösungsmittel wie zum Beispiel Dichlormethan, Acetonitril, *N*,*N*-Dimethylformamid oder Ethylacetat und in Gegenwart eine Base wie zum Beispiel Triethylamin, *N*,*N*-Diisopropylethylamin oder 1,8-Diazabicyclo[5.4.0]undec-7-cen statt (siehe Schema 7).

Die Synthese der 3-Hydroxypyrazole der allgemeinen Formel (**VIII**) erfolgt durch Reaktion der Verbindungen der allgemeinen Formel (**XIII**) in Gegenwart eines Eisenhalogenides wie zum Beispiel Eisen(III)chlorid. Die Reaktion findet bevorzugt in dem Temperaturbereich zwischen 0 °C und 120 °C, in einem adäquaten Lösungsmittel wie zum Beispiel 1,2-Dichlorethan, Acetonitril, *N,N*-Dimethylformamid oder Ethylacetat statt.

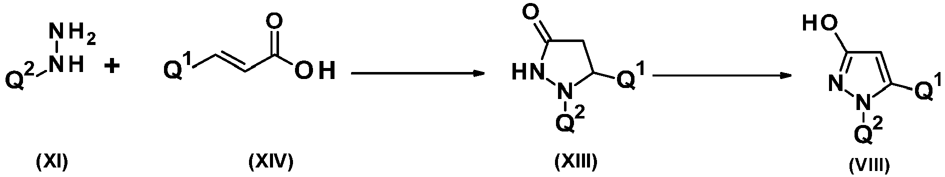

### Schema 7

Verbindungen der allgemeinen Formel (**XVI**) lassen sich durch eine N-Arylierung von einer 3-Hydroxypyrazole der allgemeinen Formel (**XVIII**) mit einem Arylhalogenid der allgemeinen Formel (**XVII**) in Gegegenwart eines Kupferhalogenides wie zum Beispiel Kupfer(I)iodid. Die Reaktion findet bevorzugt in dem Temperaturbereich zwischen 0 °C und 120 °C, in einem adäquaten Lösungsmittel wie zum Beispiel Acetonitril oder *N,N*-Dimethylformamid und in Gegenwart eine Base wie zum Beispiel Triethylamin, Cäsium Carbonat (siehe Schema 8). Die Verbindungen der allgemeinen Formeln (**XVIII**) können nach analog dem Fachmann bekannten Methoden hergestellt werden (Chem. Med. Chem. 2015, 10, 1184-1199).

Die Synthese der 5-Iodpyrazole der allgemeinen Formel (**XV**) erfolgt durch Reaktion der Verbindungen der allgemeinen Formel (**XVI**) in Gegenwart einer Base wie zum Beispiel Lithiumdiisopropylamid und Iod. Die Reaktion (Schema 8) findet bevorzugt in dem Temperaturbereich zwischen -78 °C und -60°C, in einem adäquaten Lösungsmittel wie zum Beispiel Diethylether und Tetrahydrofuran. 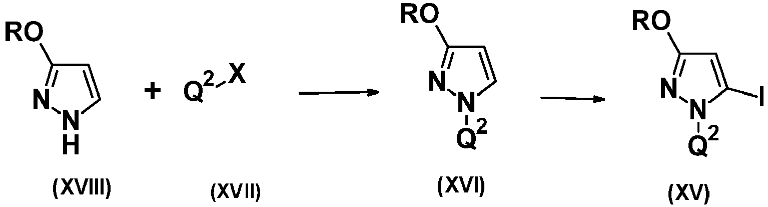
Mit X = Chlor, Brom und Iod.
Mit R = Bn (Benzyl).

### Schema 8

Eine Verbindung der allgemeinen Formel (**XIX**) lässt sich durch Reaktion von einer Verbindung der Formel (**XV**) in einem geeigneten Lösungsmittel mit einem Q¹-M (**XX**) unter Zusatz einer adäquaten Menge eines Übergangsmetallkatalysators, insbesondere Palladium-Katalysatoren wie zum Beispiel Palladiumdiacetat oder Bis(triphenylphosphin)palladium(ll)dichlorid oder um Nickelkatalysatoren wie zum Beispiel Nickel(ll)acetylacetonat oder Bis(triphenylphosphin)nickel(ll) chlorid vorzugsweise bei erhöhter Temperatur in einem organischen Lösungsmittel wie 1,2-Dimethoxyethan darstellen (Schema 9).

Der Rest "M" steht beispielsweise für Mg-X, Zn-X, Sn((C₁-C₄)Alkyl)₃, Lithium, Kupfer oder B(OR^{b})(OR^{c}), wobei die Reste R^{b} und R^{c} unabhängig voneinander beispielsweise Wasserstoff, (C₁-C₄)-Alkyl, oder, wenn die Reste R^{b} und R^{c} miteinander verbunden sind, gemeinsam Ethylen oder Propylen bedeuten. Allgemein eignen sich Methoden von Kreuzkupplungen, die in R. D. Larsen, Organometallics in Process Chemistry 2004 Springer Verlag, die in I. Tsuji, Palladium Reagents and Catalysts 2004 Wiley, die in M. Belier, C. Bolm, Transition Metals for Organic Synthesis 2004 VCH-Wiley beschrieben werden. Weitere geeignete Synthesemethoden sind in Chem. Rev. 2006, 106, 2651; Platinum Metals Review, 2009, 53, 183; Platinum Metals Review 2008, 52, 172 und Acc. Chem. Res. 2008, 41, 1486 beschrieben.

Die Synthese der 3-Hydroxypyrazole der allgemeinen Formel (**VIII**) lässt sich durch Reaktion einer Verbindung der allgemeinen Formel (**XIX**) in Gegegenwart einer Brönsted-Säure wie zum Beispiel Trifluormethansulfonsäure herstellen. Die Reaktion findet bevorzugt in dem Temperaturbereich zwischen 0 °C und 120 °C, in einem adäquaten Lösungsmittel wie zum Beispiel Dichlormethan statt (Schema 9). 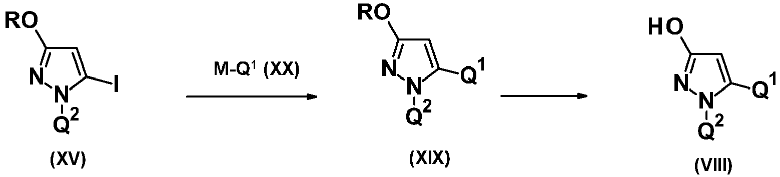 Mit R = Bn (Benzyl).

### Schema 9

Die Synthese der Verbindung der allgemeinen Formel (**XXII**) lässt sich durch Alkylierung der Verbindung der allgemeinen Formel (**XXIII**) mit einem Halogenid der allgemeinen Formel (VI) in Gegenwart einer Base nach oder lassen sich analog dem Fachmann bekannten Methoden herstellen (siehe Schema 10). Die Base kann ein Carbonat-Salz von einem Alkali-Metall (wie zum Beispiel Lithium, Natrium, Kalium oder Cäsium) sein, und die Reaktion findet bevorzugt in dem Temperaturbereich zwischen Raumtemperatur und 150 °C in einem adäquaten Lösungsmittel wie zum Beispiel Dichlormethan, Acetonitril, *N,N*-Dimethylformamid oder Ethylacetat statt.

Verbindungen der allgemeinen Formel (**XXI**) lassen sich durch eine Diazotierung oder Sandmeyer Reaktion mit der Verbindung der allgemeinen Formel (**XXII**) mit den üblichen organischen und anorganischen Nitrite wie beispielsweise 1,1-Dimethylethylnitrit, tert-Butylnitrit oder Isoamylnitrit in Gegenwart verwendbare Reagenzien wie beispielsweise Gemische aus Kupfer(1)- und Kupfer(II)bromide/chlorid oder Iod (Schema 10). Die Reaktion findet bevorzugt in dem Temperaturbereich zwischen Raumtemperatur und 0 und 120°C in einem adäquaten Lösungsmittel wie zum Beispiel Dichlormethan, Acetonitril, *N,N*-Dimethylformamid oder Diiodmethan statt. In Schema 10 wird die Synthese der Verbindung der allgemeinen Formel (**XXI**) durch Reaktion eines Pyrazoles der allgemeinen Formel (**XXII**) mit einem Halogensuccinimid der allgemeinen Formel (**IX**) in einem adäquaten Lösungsmittel wie zum Beispiel DMF beschrieben.

Eine Verbindung der allgemeinen Formel (**IV**) lässt sich durch Reaktion von einer Verbindung der Formel (**XXI**) in einem geeigneten Lösungsmittel mit einem Q¹-M (**XX**) unter Zusatz einer adäquaten Menge eines Übergangsmetallkatalysators, insbesondere Palladium-Katalysatoren wie zum Beispiel Palladiumdiacetat oder Bis(triphenylphosphin)palladium(ll)dichlorid oder um Nickelkatalysatoren wie zum Beispiel Nickel(ll)acetylacetonat oder Bis(triphenylphosphin)nickel(ll) chlorid vorzugsweise bei erhöhter Temperatur in einem organischen Lösungsmittel wie 1,2-Dimethoxyethan darstellen (Schema 9). Der Rest "M" steht beispielsweise für Mg-X, Zn-X, Sn((C₁-C₄)Alkyl)₃, Lithium, Kupfer oder B(OR^{b})(OR^{c}), wobei die Reste R^{b} und R^{c} unabhängig voneinander beispielsweise Wasserstoff, (C₁-C₄)-Alkyl, oder, wenn die Reste R^{b} und R^{c} miteinander verbunden sind, gemeinsam Ethylen oder Propylen bedeuten. Allgemein eignen sich Methoden von Kreuzkupplungen, die in R. D. Larsen, Organometallics in Process Chemistry 2004 Springer Verlag, die in I. Tsuji, Palladium Reagents and Catalysts 2004 Wiley, die in M. Belier, C. Bolm, Transition Metals for Organic Synthesis 2004 VCH-Wiley beschrieben werden. Weitere geeignete Synthesemethoden sind in Chem. Rev. 2006, 106, 2651; Platinum Metals Review, 2009, 53, 183; Platinum Metals Review 2008, 52, 172 und Acc. Chem. Res. 2008, 41, 1486 beschrieben. 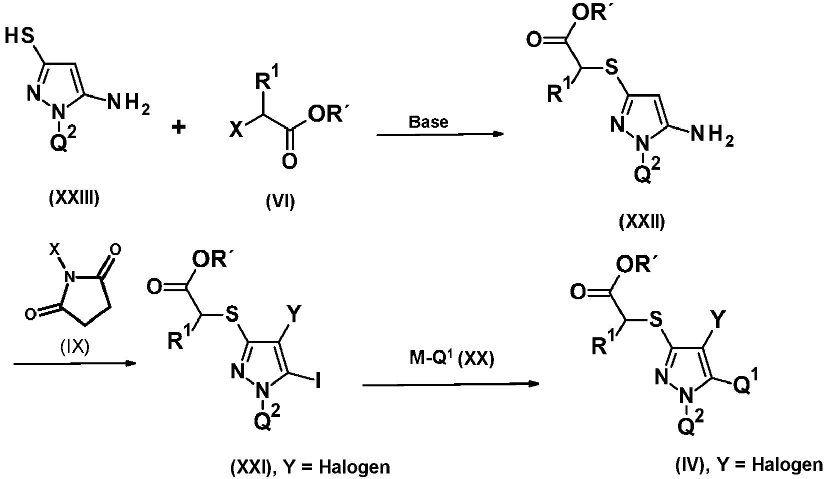
Mit R' = (C₁-C₄)-Alkyl.
Mit X = Halogen.

### Schema 10

In Schema 11 wird die Synthese der Verbindung der allgemeinen Formel (**XXIII**) durch Reaktion eines Pyrazolones der allgemeinen Formel (**XXIV**) in Gegenwart eines Schwefelungsreagenzes wie zum Beispiel Phosphorpentasulfid oder Lawesson-Reagenz in einem adäquaten Lösungsmittel wie zum Beispiel Toluol. Die Verbindungen der allgemeinen Formeln (**XXIV**) sind kommerziell erhältlich oder analog dem Fachmann bekannten Methoden herstellen.

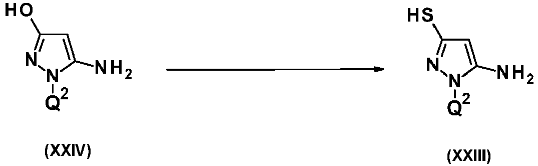

### Schema 11

Die Synthese der Verbindung der allgemeinen Formel (**IV**) lässt sich durch Reaktion eines 3-Aminopyrazoles der allgemeinen Formel (**XXVI**) mit einem Disulfid der allgemeinen Formel (**XXV**) in Gegenwart eines organischen Nitrites wie beispielsweise 1,1-Dimethylethylnitrit, tert-Butylnitrit oder Isoamylnitrit in Gegenwart von einem Metall wie zum Beispiel Kupfer statt (siehe Schema 12). Die Reaktion findet bevorzugt in dem Temperaturbereich zwischen Raumtemperatur und 120°C in einem adäquaten Lösungsmittel wie zum Beispiel Dichlormethan, Acetonitril, *N,N*-Dimethylformamid oder 1,2-Dichlorethan statt. 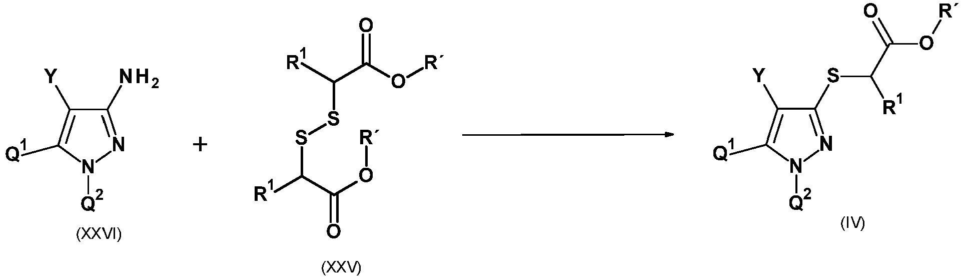
Mit R' = (C₁-C₄)-Alkyl.
Mit X = Halogen.

### Schema 12

Die Synthese der Säure der allgemeinen Formel (**XXVI**) lässt sich durch Reaktion der Verbindung der allgemeinen Formel (**XXVII**) in Gegenwart einer Lewis-Säure wie zum Beispiel Trifluoressigsäure nach oder lassen sich analog dem Fachmann bekannten Methoden herstellen (Schema 13). Die Reaktion findet bevorzugt in dem Temperaturbereich zwischen Raumtemperatur und 140 °C statt. 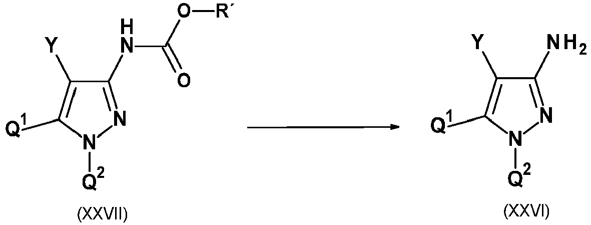 Mit R' = (C₁-C₄)-Alkyl.

### Schema 13

Die Synthese der Verbindungen der allgemeinen Formel (**XXVII**) erfolgt über eine Curtius-Reaktion von einer Säure der allgemeinen Formel (**XXIX**) mit einem Azid der allgemeinen Formel (**XXVIII**). Die Reaktion findet bevorzugt in dem Temperaturbereich zwischen 0 °C und 100 °C, in einem adäquaten Lösungsmittel wie zum Beispiel tert.-Butanol und in Gegenwart eine Base wie zum Beispiel Triethylamin, *N*,*N*-Diisopropylethylamin oder 1,8-Diazabicyclo[5.4.0]undec-7-cen statt (siehe Schema 14).

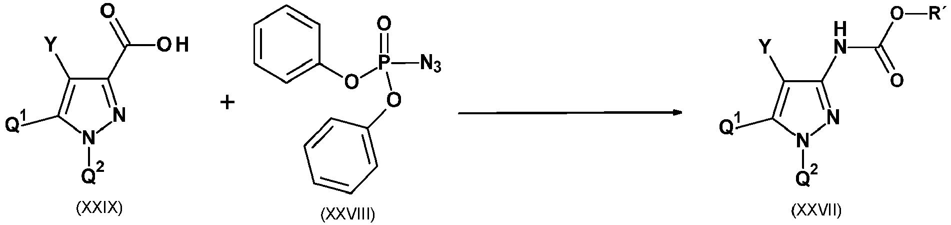

### Schema 14

Die Synthese der Säure der allgemeinen Formel (**XXIX**) lässt sich durch Verseifung der Verbindung der allgemeinen Formel (**XXX**) nach oder lassen sich analog dem Fachmann bekannten Methoden herstellen (Schema 15). Die Verseifung lässt sich in Gegenwart einer Base oder einer Lewis-Säure durchführen. Die Base kann ein Hydroxid-Salz von einem Alkali-Metall (wie zum Beispiel Lithium, Natrium oder Kalium) sein, und die Verseifungsreaktion findet bevorzugt in dem Temperaturbereich zwischen Raumtemperatur und 120 °C statt. 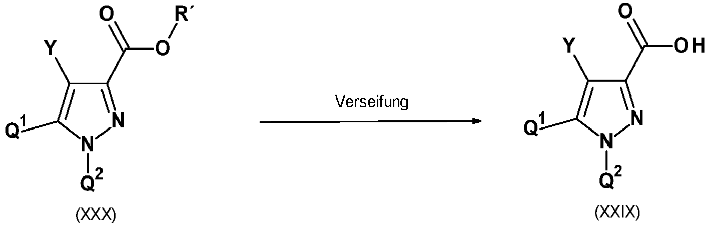 Mit R' = (C₁-C₄)-Alkyl.

### Schema 15

In Schema 16 wird die Synthese der Verbindung der allgemeinen Formel (**XXX**) durch Reaktion eines Pyrazoles der allgemeinen Formel (**XXXI**) mit einem Halogensuccinimid der allgemeinen Formel (**IX**) in einem adäquaten Lösungsmittel wie zum Beispiel DMF beschrieben. 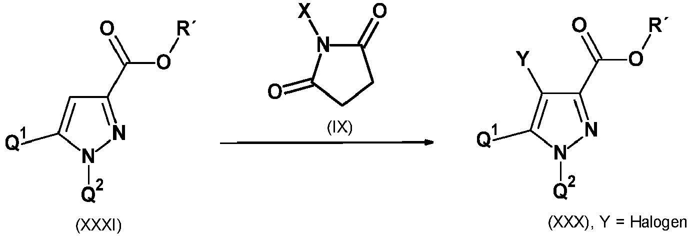 Mit X = Halogen.

### Schema 16

Die Synthese der Verbindungen der allgemeinen Formel (**XXXI**) erfolgt über eine Kondensation von eines Diketoesters der allgemeinen Formel (**XXXIII**) mit einem Arylhydrazin oder Hetarylhydrazin der allgemeinen Formel (**XI**) in Gegenwart einer Broensted-Säure wie zum Beispiel Essigsäure oder Chlorwasserstoff in einem adäquaten Lösungsmittel wie zum Beispiel Methanol, Ethanol, Isopropanol, n-Butanol, Tetrahydrofuran, Dioxan, Toluol oder Chlorbenzol (Schema 17). Die Reaktion findet bevorzugt in dem Temperaturbereich zwischen 0 °C und 150 °C statt. Die Verbindungen der allgemeinen Formeln (**XI**) und (**XXXIII**) sind kommerziell erhältlich oder lassen sich analog dem Fachmann bekannten Methoden herstellen. 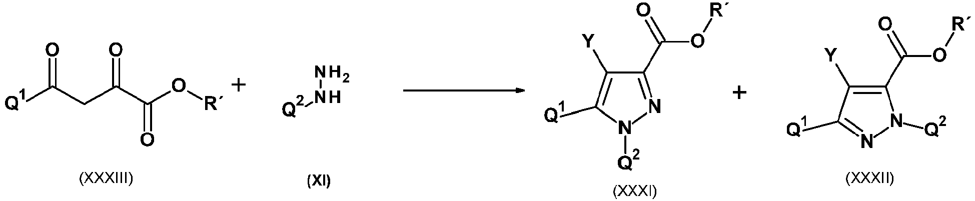 Mit R' = (C₁-C₄)-Alkyl.

### Schema 17

Eine Verbindung der allgemeinen Formel (**XXXIV**) lässt sich beispielsweise durch Reaktion von einer Verbindung der Formel (**VII**) in einem geeigneten Lösungsmittel mit einem Metallcynid M-CN (**XXXV**) unter Zusatz einer adäquaten Menge eines Übergangsmetallkatalysators, insbesondere Palladium-Katalysatoren wie Palladium(0)tetrakis(triphenylphosphin) oder Palladiumdiacetat oder Bis(triphenylphosphin)-palladium(ll)dichlorid oder um Nickelkatalysatoren wie Nickel(ll)acetylacetonat oder Bis(triphenylphosphin)nickel(ll)chlorid vorzugsweise bei erhöhter Temperatur in einem organischen Lösungsmittel wie zum Beispiel 1,2-Dimethoxyethan oder N,N-Dimethylformamid darstellen (Schema 18). Der Rest "M" steht beispielsweise für Magnesium, Zink, Lithium oder Natrium. Allgemein eignen sich Methoden von Kreuzkupplungen, die in R. D. Larsen, Organometallics in Process Chemistry 2004 Springer Verlag, die in I. Tsuji, Palladium Reagents and Catalysts 2004 Wiley, die in M. Belier, C. Bolm, Transition Metals for Organic Synthesis 2004 VCH-Wiley beschrieben werden. Weitere geeignete Synthesemethoden sind in Chem. Rev. 2006, 106, 2651; Platinum Metals Review, 2009, 53, 183; Platinum Metals Review 2008, 52, 172 und Acc. Chem. Res. 2008, 41, 1486 beschrieben.

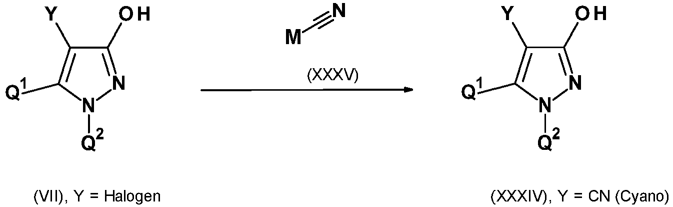

### Schema 18

Verbindungen der allgemeinen Formeln (**XXXVI**) und (**XXXVII**) lassen sich durch Reaktion von einer Verbindung der Formel (**IV**) in Gegenwart eines Oxidationsmittels wie zum Beispiel mCPBA (3-Chlorperbenzoesäure) in einem adäquaten Lösungsmittel wie zum Beispiel Dichlormethan oder 1,2-Dichlorethan herstellen (Schema 19). Die Reaktion findet bevorzugt in dem Temperaturbereich zwischen -30 °C und 100 °C statt. 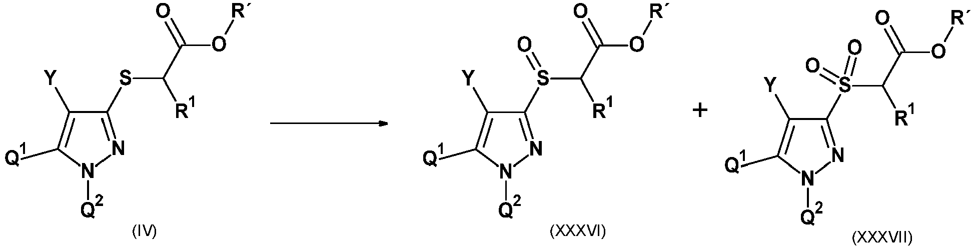 Mit R' = (C₁-C₄)-Alkyl.

### Schema 19

In Schema 20 wird die Synthese der Verbindung der allgemeinen Formel (**IV**) durch Reaktion eines Pyrazoles der allgemeinen Formel (**XXXVIII**) mit einem Halogensuccinimid der allgemeinen Formel (**IX**) in einem adäquaten Lösungsmittel wie zum Beispiel DMF beschrieben. 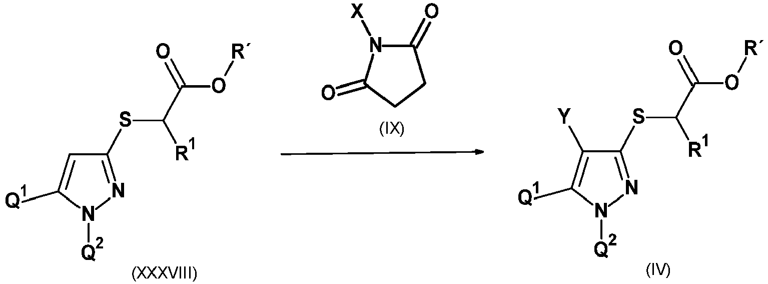 Mit R' = (C₁-C₄)-Alkyl.

### Schema 20

Ausgewählte detaillierte Synthesebeispiele für die erfindungsgemäßen Verbindungen der allgemeinen Formel (1) sind im Folgenden aufgeführt. Die angegebenen Beispielnummern entsprechen den in den nachstehenden Tabellen 1.1 bis 1.49 genannten Nummerierungen. Die ¹H-NMR-, ¹³C-NMR- und ¹⁹F-NMR-spektroskopischen Daten, die für die in den nachfolgenden Abschnitten beschriebenen chemischen Beispiele angegeben sind, (400 MHz bei ¹H-NMR und 150 MHz bei ¹³C-NMR und 375 MHz bei ¹⁹F-NMR, Lösungsmittel CDCl₃, CD₃OD oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Bei Diastereomerengemischen werden entweder die jeweils signifikanten Signale beider Diastereomere oder das charakteristische Signal des Hauptdiastereomers angegeben. Die verwendeten Abkürzungen für chemische Gruppen haben beispielsweise die nachfolgenden Bedeutungen: Me = CH₃, Et = CH₂CH₃, t-Hex = C(CH₃)₂CH(CH₃)₂, t-Bu = C(CH₃)₃, n-Bu = unverzweigtes Butyl, n-Pr = unverzweigtes Propyl, i-Pr = verzweigtes Propyl, c-Pr = Cyclopropyl, c-Hex = Cyclohexyl.

### Synthesebeispiele:

### Synthesebeispiel No. 1-005:

### Synthesestufe1: Ethyl-(3Z)-4-(3,4-difluorphenyl)-4-hydroxy-2-oxobut-3-enoat

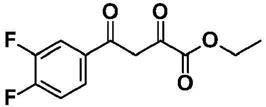

Eine 1 M Lithiumtrimethyl-N-(trimethylsilyl)silanaminid-Lösung in THF (33,63 ml, 33,63 mmol, 1,05 equiv) wurde in Diethylether (80 ml) unter Stickstoffatmosphäre gelöst und mit einem Trockeneisbad auf -78 °C gekühlt. Zu der Lösung tropfte man innerhalb von 10 min. Eine Lösung von 1-(3,4-Difluorphenyl)ethanon (5,0 g, 35,02 mmol, 1,0 equiv.) in Diethylether (20 ml) zu. Das resultierende Reaktionsgemisch wurde 3 h bei -78 °C und danach bei Raumtemperaturüber Nacht gerührt. Anschließend kühlte man die Suspension mit einem Eisbad auf 0 bis 4 °C ab, versetzte diese mit 1M Salzsäure und rührte 30 min bei Raumtemperatur. Das Reaktionsgemisch wurde dreimal mit jeweils 100 ml Ethylacetat extrahiert. Die organische Phase trocknete man über Magnesiumsulfat und entfernte das Lösungsmittel im Vakuum. Das gewünschte Produkt konnte Ethyl-(3Z)-4-(3,4-difluorphenyl)-4-hydroxy-2-oxobut-3-enoat in Form eines weißen Feststoffes isoliert werden (8,00 g, 97% der Theorie). ¹H-NMR (400 MHz, CDCl₃ δ, ppm) 15.05 (bs, 1H), 7.87-7.77 (m, 2H), 7.33-7.29 (m, 1H), 7.00 (s, 1H), 4.41 (q, 2H), 1.42 (t, 3H).

### Synthesestufe 2: Ethyl-5-(3,4-difluorphenyl)-1-(2-fluorphenyl)-1H-pyrazol-3-carboxylat

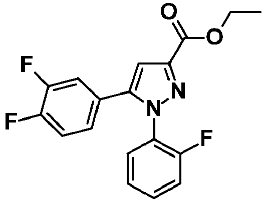

Ethyl-(3Z)-4-(3,4-difluorphenyl)-4-hydroxy-2-oxobut-3-enoat (2,0 g, 7,81 mmol, 1,0 equiv) und (2-Fluorphenyl)hydrazinhydrochlorid (1:1) (1,90 g, 11,71 mmol, 1,5 equiv) wurden in 1,25 M Chlorwasserstoff in Ethanol (30 ml) suspendiert und 4 h zum Sieden erhitzt. Das Reaktionsgemisch wurde mit einer gesättigten Natriumhydrogencarbonat-Lösung neutraliert und anschließend dreimal mit jeweils 50 ml Dichlormethan extrahiert. Die organische Phase trocknete man über Magnesiumsulfat und entfernte das Lösungsmittel im Vakuum. Durch abschließende säulenchromatographische Reinigung (Gradient Essigester/Heptan) des resultierenden Rohproduktes konnte Ethyl-5-(3,4-difluorphenyl)-1-(2-fluorphenyl)-1H-pyrazol-3-carboxylat in Form eines gelben Öls isoliert werden (3,02 g, 98% der Theorie). ¹H-NMR (400 MHz, CDCl₃ δ, ppm) 7.57-7.53 (m, 1H), 7.47-7.41 (m, 1H), 7.29-7.25 (m, 1H), 7.13-7.01 (m, 4H), 6.97-6.93 (m, 1H), 4.46 (q, 2H), 1.43 (t, 3H).

### Synthesestufe 3: Ethyl-4-brom-5-(3,4-difluorphenyl)-1-(2-fluorphenyl)-1H-pyrazol-3-carboxylat

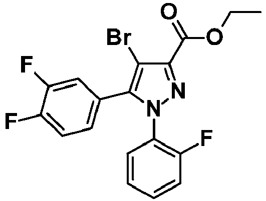

Ethyl-5-(3,4-difluorphenyl)-1-(2-fluorphenyl)-1H-pyrazol-3-carboxylat (2,38 g, 6,87 mmol, 1,0 equiv) wurde in Essigsäure (20 ml) gelöst und bei Raumtemperatur mit Brom (1,10 g, 6,87 mmol, 1,0 equiv) versetzt. Das resultierende Reaktionsgemisch wurde 2 h bei Raumtemperatur gerührt und dann erneut mit Brom (0,55 g, 3,43 mmol, 0,5 equiv) versetzt. Die Reaktion wurde bei Raumtemperatur über Nacht gerührt und anschließend mit Eiswasser (150 ml) versetzt, wobei ein Niederschlag entstand. Der Niederschlag wurde in Dichlormethan (100 ml) gelöst und mit gesättigter Natriumthiosulfat-Lösung extrahiert. Die organische Phase trocknete man über Magnesiumsulfat und entfernte das Lösungsmittel im Vakuum. Durch abschließende säulenchromatographische Reinigung (Gradient Essigester/Heptan) des resultierenden Rohproduktes konnte Ethyl-4-brom-5-(3,4-difluorphenyl)-1-(2-fluorphenyl)-1H-pyrazol-3-carboxylat in Form eines weißen Feststoffes isoliert werden (2,4 g, 78% der Theorie). ¹H-NMR (400 MHz, CDCl₃ δ, ppm) 7.51-7.45 (m, 1H), 7.44-7.39 (m, 1H), 7.27-7.22 (m, 1H), 7.17-6.98 (m, 4H), 4.49 (q, 2H), 1.44 (t, 3H).

### Synthesestufe 4: 4-Brom-5-(3,4-difluorphenyl)-1-(2-fluorphenyl)-1H-pyrazol-3-carbonsäure

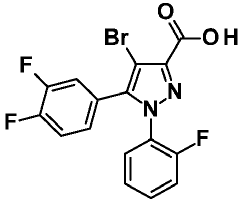

Ethyl-4-brom-5-(3,4-difluorphenyl)-1-(2-fluorphenyl)-1H-pyrazol-3-carboxylat (2,08 g, 4,88 mmol, 1,0 equiv) und Lithiumhydroxid (350,6 mg, 14,64 mmol, 3,0 equiv) wurden in einer Mischung aus THF und Wasser (18 ml, THF:Wasser = 7:2) gelöst und 6 h zum Sieden erhitzt. Nach dem Abkühlen säuerte man die Lösung mit 2 M Salzsäure anund extrahierte zweimal mit Ethylacetat (50 ml). Die organische Phase trocknete man über Magnesiumsulfat und entfernte das Lösungsmittel im Vakuum. 4-Brom-5-(3,4-difluorphenyl)-1-(2-fluorphenyl)-1H-pyrazol-3-carbonsäure wurde in Form eines weißen Feststoffes isoliert (1,51 g, 77% der Theorie). ¹H-NMR (400 MHz, CDCl₃ δ, ppm) 7.52-7.42 (m, 1H), 7.29-7.26 (m, 1H), 7.48 (m, 1H), 7.19-7.08 (m, 3H), 7.03-7.00 (m, 1H).

### Synthesestufe 5: tert-Butyl-[4-brom-5-(3,4-difluorphenyl)-1-(2-fluorphenyl)-1H-pyrazol-3-yl]carbamat

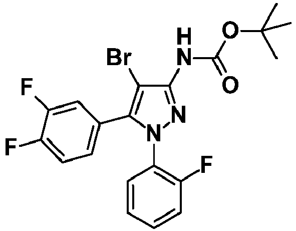

4-Brom-5-(3,4-difluorphenyl)-1-(2-fluorphenyl)-1H-pyrazol-3-carbonsäure (1,02 g, 2,57 mmol, 1,0 equiv) und Triethylamin (0,90 ml, 6,42 mmol, 2,5 equiv) wurden in tert.-Butanol (15 ml) gelöst und bei Raumtemperatur mit Diphenylphosphorazidat (0,55 ml, 2,57 mmol, 1,0 equiv) versetzt. Die Reaktion wurde 4 h bei 60 °C gerührt, wobei eine Gasentwicklung auftrat. Nach dem Abkühlen auf Raumtemperatur versetzte man die Reaktionslösung mit einer gesättigten Natriumhydrogencarbonat-Lösung (10 ml) und extrahierte zweimal mit Ethylacetat (50 ml). Die organische Phase trocknete man über Magnesiumsulfat und entfernte das Lösungsmittel im Vakuum. Durch abschließende säulenchromatographische Reinigung (Gradient Essigester/Heptan) des resultierenden Rohproduktes konnte tert-Butyl-[4-brom-5-(3,4-difluorphenyl)-1-(2-fluorphenyl)-1H-pyrazol-3-yl]carbamat in Form eines weißen Feststoffes isoliert werden (625 mg, 51% der Theorie). ¹H-NMR (400 MHz, CDCl₃ δ, ppm) 7.52-7.48 (m, 1H), 7.38-7.32 (m, 1H), 7.21 (dt, 1H), 7.15-7.09 (m, 2H), 7.05-6.97 (m, 2H), 6.48 (bs, 1H), 1.56 (s, 9H).

### Synthesestufe 6: 4-Brom-5-(3,4-difluorphenyl)-1-(2-fluorphenyl)-1H-pyrazol-3-amin

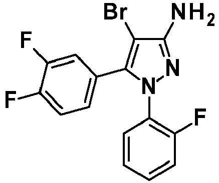

tert-Butyl-[4-brom-5-(3,4-difluorphenyl)-1-(2-fluorphenyl)-1H-pyrazol-3-yl]carbamat (625 mg, 1,34 mmol, 1,0 equiv) und Trifluoressigsäure (0,21 ml, 2,67 mmol, 2,0 equiv) wurden in Dichlormethan (20 ml) gelöst und 2 h zum Sieden erhitzt. Nach dem Abkühlen verdünnte man das Reaktionsgemisch mit gesättigter Natriumhydrogencarbonat-Lösung (10 ml) und extrahierte danach zweimal mit DCM (50 ml). 4-Brom-5-(3,4-difluorphenyl)-1-(2-fluorphenyl)-1H-pyrazol-3-amin wurde in Form eines weißen Feststoffes isoliert werden (480 mg, 99% der Theorie). ¹H-NMR (400 MHz, CDCl₃ δ, ppm) 7. 96 (bs, 2H), 7.46-7.36 (m, 2H), 7.26-7.23 (m, 1H), 7.18-7.10 (m, 3H), 7.08-7.00 (m, 1H).

### Synthesestufe 7: Ethyl-{[4-brom-5-(3,4-difluorphenyl)-1-(2-fluorphenyl)-1H-pyrazol-3-yl]sulfanyl} acetat (Beispiel No. 1-002)

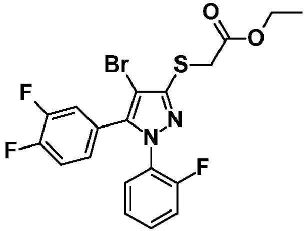

4-Brom-5-(3,4-difluorphenyl)-1-(2-fluorphenyl)-1H-pyrazol-3-amin (223 mg, 0,61 mmol, 1,0 equiv), Diethyl-2,2'-disulfandiyldiacetat (0,22 ml, 1,21 mmol, 2 equiv) und Kupfer-Pulver (7,70 mg, 0,12 mmol, 0,2 equiv) wurden in 1,2-Dichlorethan (10 ml) suspendier und 15 min auf 60 °C erhitzt. Anschließend tropfte man tert-Butylnitrit (0,22 ml, 1,21 mmol, 2,0 equiv) zu und erhitzte das Reaktionsgemisch 4 h auf 80 °C. Nach dem Abkühlen verdünnte man das Reaktionsgemisch mit Wasser (10 ml) und Dichlormethan (30 ml). extrahierte danach zweimal mit DCM (50 ml). Die Phasen trennte man mit einem Phasenseparator. Durch abschließende säulenchromatographische Reinigung (Gradient Essigester/Heptan) des resultierenden Rohproduktes konnte Ethyl-{[4-brom-5-(3,4-difluorphenyl)-1-(2-fluorphenyl)-1H-pyrazol-3-yl]sulfanyl}acetat in Form eines weißen Feststoffes isoliert werden (129 mg, 44% der Theorie). ¹H-NMR (400 MHz, CDCl₃ δ, ppm) 7.44-7.34 (m, 2H), 7.26-7.20 (m, 1H), 7.13-7.02 (m, 3H), 6.99-6.96 (m, 1H), 4.22 (q, 2H), 3.89 (s, 2H), 1.24 (t, 3H).

### Synthesestufe 8: {[4-Brom-5-(3,4-difluorphenyl)-1-(2-fluorphenyl)-1H-pyrazol-3-yl]sulfanyl}essigsäure (Beispiel No. 1-005)

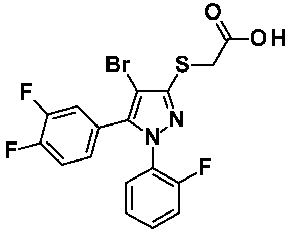

Ethyl-{[4-brom-5-(3,4-difluorphenyl)-1-(2-fluorphenyl)-1H-pyrazol-3-yl]sulfanyl}acetat (83 mg, 0,18 mmol, 1,0 equiv) und Lithiumhydroxid (12,6 mg, 0,53 mmol, 3,0 equiv) wurden in einer Mischung aus THF und Wasser (9 ml, THF:Wasser = 7:2) gelöst und 6 h zum Sieden erhitzt. Nach dem Abkühlen säuerte man die Lösung mit 2 M Salzsäure anund extrahierte zweimal mit Ethylacetat (50 ml). Die organische Phase trocknete man über Magnesiumsulfat und entfernte das Lösungsmittel im Vakuum. {[4-Brom-5-(3,4-difluorphenyl)-1-(2-fluorphenyl)-1H-pyrazol-3-yl]sulfanyl}essigsäure wurde in Form eines weißen Feststoffes isoliert (56 mg, 68% der Theorie). ¹H-NMR (400 MHz, CDCl₃ δ, ppm) 7.45-7.35 (m, 2H), 7.27-7.22 (m, 1H), 7.18-7.09 (m, 3H), 7.01-6.97 (m, 3H), 3.86 (s, 2H).

### Synthesebeispiel No. 1-008:

### Synthesestufe1: Ethyl-{[4-brom-1-(2,3 -difluorphenyl)-5 -phenyl- 1H-pyrazol-3 -yl] sulfonyl} acetat

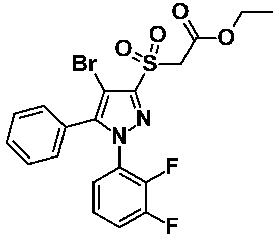

Ethyl-{[4-brom-1-(2,3-difluorphenyl)-5-phenyl-1H-pyrazol-3-yl]sulfanyl}acetat (115 mg, 0,25 mmol, 1.0 equiv) wurde in Dichlormethan (5 ml) gelöst. Die Lösung versetzte man mit 3-Chlorbenzencarboperoxosäure (114 mg, 0,51 mmol, 2.0 equiv, 77%ig) zu. Das resultierende Reaktionsgemisch wurde bei Raumtemperaturüber Nacht gerührt. Das Reaktionsgemisch wurde mit Dichlormethan (30 ml) verdünnt und mit einer ges. Natriumhydrogencarbonat-Lösung extrahiert. Die organische Phase trocknete man über Magnesiumsulfat und entfernte das Lösungsmittel im Vakuum. Durch abschließende säulenchromatographische Reinigung (Gradient Essigester/Heptan) des resultierenden Rohproduktes konnte Ethyl-{[4-brom-1-(2,3-difluorphenyl)-5-phenyl-1H-pyrazol-3-yl]sulfonyl}acetat in Form eines farblosen Feststoffes isoliert werden (90 mg, 55% der Theorie, 80%ig). ¹H-NMR (400 MHz, CDCl₃ δ, ppm) 7.71-7.63 (m, 2H), 7.58-7.32 (m, 6H), 4.76 (s, 2H), 4.12 (q, 2H), 1.13 (t, 3H).

### Synthesebeispiel No. II-001:

### Synthesestufe 1: 1H-Pyrazol-3-ol

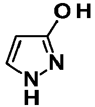

Methyl-(2E)-3-methoxyacrylat (20,00 g, 172,24 mmol, 1.0 equiv) wurde in Methanol (15 ml) gelöst und tropfenweise mit Hydrazinhydrat (1:1) (8,38 ml, 172,24 mmol, 1 equiv) versetzt, wobei sich das Reaktionsgemisch zum Sieden erhitzte. Anschließend erhitzte man das Reaktionsgemisch 2 h zum Sieden und engte dann im Vakuum ein. Das Rohprodukt wurde ohne weitere Aufreinigung in der nächsten Synthesestufe eingesetzt. 1H-Pyrazol-3-ol wurde in Form eines gelben Öls isoliert (15,30 g, 95% der Theorie). ¹H-NMR (400 MHz, CDCl₃ δ, ppm) 9.85 (bs, 1H), 7.35 (d, 1H), 5.43 (d, 1H).

### Synthesestufe 2: 1-(3-Hydroxy-1H-pyrazol-1-yl)ethanon

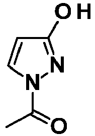

1H-Pyrazol-3-ol (15,30 g, 181,97 mmol, 1,0 equiv) wurde in Pyridin (100 ml) gelöst. Zu dieser Lösung tropfte man innerhalb von 30 min ein Gemisch aus Essigsäureanhydrid (19,00 ml, 201,37 mmol) und Pyridin (10 ml). Das resultierende Reaktionsgemisch wurde 2 h auf eine Temperatur von 100 °C erhitzt. Nach dem Abkühlen auf Raumtemperatur entfernte man das Lösungsmittel im Vakuum. Der Rückstand wurde mit Diethylether (50 ml) verrührt und kristallisierte dabei aus. 1-(3-Hydroxy-1H-pyrazol-1-yl)ethanon konnte in Form eines leicht gelben Feststoffes isoliert werden (18,00 g, 78% der Theorie). ¹H-NMR (400 MHz, CDCl₃ δ, ppm) 10.98 (bs, 1H), 8.12 (d, 1H), 6.01 (d, 2H), 2.47 (s, 3H).

### Synthesestufe 3: 3-(Benzyloxy)-1H-pyrazol

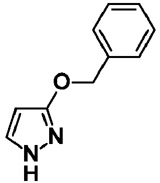

1-(3-Hydroxy-1H-pyrazol-1-yl)ethanon (18,00 g, 142,73 mmol, 1,0 equiv), (Brommethyl)benzol (16,98 ml, 142,73 mmol, 1,0 equiv) und Kaliumcarbonat (19,73 g, 142,73 mmol, 1,0 equiv) wurden in Butan-2-on (200 ml) suspendiert und 3 h zum Sieden erhitzt. Nach dem Abkühlen wurde das Reaktionsgemisch filtriert und das Filtrat im Vakuum eingeengt. Der Rückstand wurde in THF (150 ml) und Methanol (150 ml) gelöst und mit einer 6 M Natronlauge (24 ml) versetzt. Das Reaktionsgemisch wurde 1 h bei Raumtemperatur gerührt und anschließend das Lösungsmittel im Vakuum eingeengt. Der Rückstand wurde in Dichlormethan (150 ml) gelöst und zweimal mit Wasser (50 ml) gewaschen. Die organische Phase trocknete man über Magnesiumsulfat und entfernte das Lösungsmittel im Vakuum. Das Rohprodukt wurde ohne weitere Aufreinigung in der nächsten Synthesestufe eingesetzt. 3-(Benzyloxy)-1H-pyrazol wurde in Form eines gelben Öls isoliert (19,82 g, 75% der Theorie). ¹H-NMR (400 MHz, CDCl₃ δ, ppm) 10.55 (bs, 1H), 7.43-7.23 (m, 6H), 5.74 (d, 1H), 5.20 (s, 2H).

### Synthesestufe 4: 3-(Benzyloxy)-1-(2-fluorphenyl)-1H-pyrazol

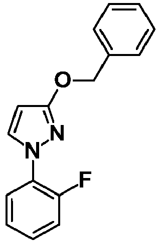

3-(Benzyloxy)-1H-pyrazol (2,74 g, 15,73 mmol, 1,0 equiv), 1-Fluor-2-iodbenzol (1,83 ml, 15,73 mmol, 1 equiv), Kupfer(I)iodid (299 mg, 1,57 mmol, 0,10 equiv), Kaliumcarbonat (5,43 g, 39,31 mmol, 2,50 equiv) und N,N'-Dimethylethan-1,2-diamin (0,87 ml, 7,88 mmol, 0,10 equiv) wurden in Toluol (25 ml) suspendiert und 5 h auf 115 °C erhitzt. Man entfernt das Lösungsmittel im Vakuum und extrahierte den Rückstand mit Dichlormethan (50 ml) und Wasser (20 ml). Die organische Phase trocknete man über Magnesiumsulfat und entfernte das Lösungsmittel im Vakuum. Durch abschließende säulenchromatographische Reinigung (Gradient Essigester/Heptan) des resultierenden Rohproduktes konnte 3-(Benzyloxy)-1-(2-fluorphenyl)-1H-pyrazol in Form eines gelben Feststoffes isoliert werden (2,09 g, 49% der Theorie, 80%ig). ¹H-NMR (400 MHz, CDCl₃ δ, ppm) 7.91-7.85 (m, 2H), 7.51-7.49 (m, 2H), 7.42-7.32 (m, 3H), 7.25-7.16 (m, 3H), 5.95 (d, 1H), 5.32 (s, 2H).

### Synthesestufe 5: 3-(Benzyloxy)-1-(2-fluorphenyl)-5-iod-1H-pyrazol

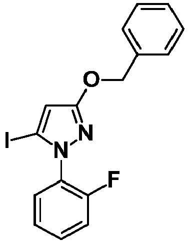

3-(Benzyloxy)-1-(2-fluorphenyl)-1H-pyrazol (4.81 g, 17,92 mmol, 1,0 equiv) wurde in THF (140 ml) gelöst und mit einem Kältebad (Trockeneis / Aceton) auf -78 °C gekühlt. Zu dieser gekühlten Lösung tropfte man innerhalb von 10 min eine 2 M Lithiumdiisopropylamid-Lösung in THF zu und rührte 30 min bei dieser Temperatur nach. Anschließend tropfte man Iod (4,55 g, 17,92 mmol, 1 equiv) gelöst in THF (10 ml) innerhalb von 10 min zu. Danach wurde das Reaktiongemisch über Nacht auf Raum-temperatur erwärmt und mit Wasser (300 ml) und Ethylacetat (250 ml) extrahiert. Die organische Phase wurde mit einer gesättigten Natriumthiosulfat-Lösung (75 ml) gewaschen. Anschließend trocknete man die organische Phase über Magnesiumsulfat und entfernte das Lösungsmittel im Vakuum. Durch abschließende säulenchromatographische Reinigung (Gradient Essigester/Heptan) des resultierenden Rohproduktes konnte 3-(Benzyloxy)-1-(2-fluorphenyl)-5-iod-1H-pyrazol in Form eines braunen Öls isoliert werden (5,20 g, 73% der Theorie). ¹H-NMR (400 MHz, CDCl₃ δ, ppm) 7.90-7.84 (m, 2H), 7.52-7.47 (m, 2H), 7.41-7.31 (m, 2H), 7.25-7.15 (m, 3H), 5.94 (s, 1H), 5.31 (s, 2H).

### Synthesestufe 6: 5-[3-(Benzyloxy)-1-(2-fluorphenyl)-1H-pyrazol-5-yl]-2-fluorpyridin

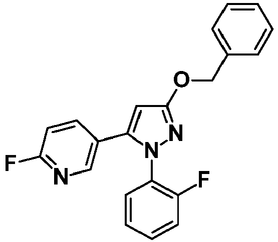

3-(Benzyloxy)-1-(2-fluorphenyl)-5-iod-1H-pyrazol (2,20 g, 5,58 mmol, 1,0 equiv), (6-Fluorpyridin-3-yl)boronsäure (1,18 mg, 8,37 mmol, 1,5 equiv) und Caesiumcarbonat (7,27 lg, 22,32 mmol, 4 equiv) wurden in einem Lösungsmittelgemisch bestehend aus Toluol (56 ml), Ethanol (16 ml) und Wasser (8 ml) suspendiert und dreimal nacheinander entgast und mit Argon belüftet. Die Suspension versetzt man mit Bis(di-tert.-butyl-(4-dimethylaminophenyl)-phosphino)- palladium(II)chlorid. Anschließend erhitzte man das Reaktionsgemisch 2 h zum Sieden. Man entfernt das Lösungsmittel im Vakuum und extrahierte den Rückstand mit Dichlormethan (750 ml) und Wasser (30 ml). Die organische Phase trocknete man über Magnesiumsulfat und entfernte das Lösungsmittel im Vakuum. Durch abschließende säulenchromatographische Reinigung (Gradient Essigester/Heptan) des resultierenden Rohproduktes konnte 5-[3-(Benzyloxy)-1-(2-fluorphenyl)-1H-pyrazol-5-yl]-2-fluorpyridin in Form eines gelben Feststoffes isoliert werden (1,01 g, 45% der Theorie). ¹H-NMR (400 MHz, DMSO-d⁶ δ, ppm) 8.15 (d, 1H), 7.81-7.77 (m, 1H), 7.63-7.59 (m, 1H), 7.54-7.48 (m, 3H), 7.43-7.30 (m, 5H), 7.22-7.19 (m, 1H), 6.44 (s, 1H), 5.24 (s, 2H).

### Synthesestufe 7: 5-[3-(Benzyloxy)-4-brom-1-(2-fluorphenyl)-1H-pyrazol-5-yl]-2-fluorpyridin

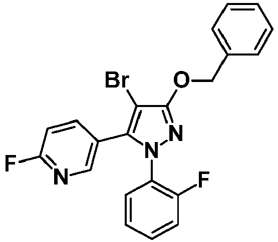

5-[3-(Benzyloxy)-1-(2-fluorphenyl)-1H-pyrazol-5-yl]-2-fluorpyridin (870 mg, 1,92 mmol, 1,0 equiv, 80%ig) wurde in DMF (10 ml) gelöst und bei Raumtemperatur mit 1-Brompyrrolidin-2,5-dion (0,51 g, 2,87 mmol, 1,5 equiv) versetzt. Das resultierende Reaktionsgemisch wurde über Nacht bei Raumtemperatur gerührt und anschließend mit Eiswasser (50 ml) versetzt, wobei ein Niederschlag entstand. Der Niederschlag wurde in Dichlormethan (100 ml) gelöst und mit gesättigter Natriumthiosulfat-Lösung extrahiert. Die organische Phase trocknete man über Magnesiumsulfat und entfernte das Lösungsmittel im Vakuum. Durch abschließende säulenchromatographische Reinigung (Gradient Essigester/Heptan) des resultierenden Rohproduktes konnte 5-[3-(Benzyloxy)-4-brom-1-(2-fluorphenyl)-1H-pyrazol-5-yl]-2-fluorpyridin in Form eines weißen Feststoffes isoliert werden (847 mg, 99% der Theorie). ¹H-NMR (400 MHz, DMSO-d⁶ δ, ppm) 8.20 (d, 1H), 7.96-7.91 (m, 1H), 7.61 (dt, 1H), 7.52-7.27 (m, 9H), 5.33 (s, 2H).

### Synthesestufe 8: 4-Brom-1-(2-fluorphenyl)-5-(6-fluorpyridin-3-yl)-1H-pyrazol-3-ol

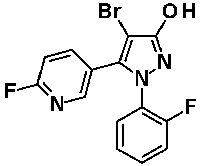

5-[3-(Benzyloxy)-4-brom-1-(2-fluorphenyl)-1H-pyrazol-5-yl]-2-fluorpyridin (811 mg, 1,83 mmol, 1,0 equiv) wurde in Dichlormethan (25 ml) gelöst und bei Raumtemperatur mit Trifluormethansulfonsäure (0,33 ml, 3,67 mmol, 2,0 equiv) versetzt. Das resultierende Reaktionsgemisch wurde 2 h bei Raumtemperatur gerührt und anschließend mit einer gesättigter Natriumhydrogencarbonat-Lösung (20 ml) extrahiert. Die wässrige Phase extrahierte man zweimal mit Dichlormethan (25 ml). Die vereinigten organische Phase trocknete man über Magnesiumsulfat und entfernte das Lösungsmittel im Vakuum. Das Rohprodukt wurde ohne weitere Aufreinigung in der nächsten Synthesestufe eingesetzt. 4-Brom-1-(2-fluorphenyl)-5-(6-fluorpyridin-3-yl)-1H-pyrazol-3-ol wurde in Form eines weißen Feststoffs isoliert (700 mg, 92% der Theorie, 90%ig). ¹H-NMR (400 MHz, CDCl₃ δ, ppm) 9.51 (bs, 1H), 8.12 (d, 1H), 7.76 (dt, 1H), 7.48-7.43 (m, 1H), 7.41-7.38 (m, 1H), 7.27-7.24 (m, 1H), 7.08-7.06 (m, 1H), 6.96-6.94 (m, 1H).

### Synthesestufe 8: 4-Brom-1-(2-fluorphenyl)-5-(6-fluorpyridin-3-yl)-1H-pyrazol-3-thiol

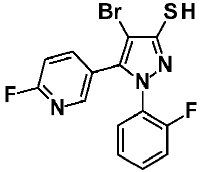

4-Brom-1-(2-fluorphenyl)-5-(6-fluorpyridin-3-yl)-1H-pyrazol-3-ol (930 mg, 2,64 mmol, 1,0 equiv) wurde in Toluol (20 ml) suspendiert und bei Raumtemperatur mit Phosphorpentasulfid (2,35 g, 5,28 mmol, 2,0 equiv) versetzt. Das resultierende Reaktionsgemisch wurde 6 h zum Sieden erhitzt (Gasentwicklung). Nach dem Abkühlen auf Raumtemperatur filtrierte man den Niederschlag ab und rührte diesen mit Toluol (50 ml) aus. Die Toluol-Phasen wurden vereinigt und das Lösungsmittel im Vakuum entfernt. Das Rohprodukt wurde ohne weitere Aufreinigung in der nächsten Synthesestufe eingesetzt. 4-Brom-1-(2-fluorphenyl)-5-(6-fluorpyridin-3-yl)-1H-pyrazol-3-thiol wurde in Form eines gelben Feststoffs isoliert (515 mg, 52% der Theorie). ¹H-NMR (400 MHz, CDCl₃ δ, ppm)

### Synthesestufe 9: Ethyl-{[4-brom-1-(2-fluorphenyl)-5-(6-fluorpyridin-3-yl)-1H-pyrazol-3-yl]sulfanyl}acetat (Beispiel No. 11-001)

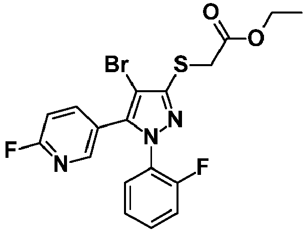

4-Brom-1-(2-fluorphenyl)-5-(6-fluorpyridin-3-yl)-1H-pyrazol-3-thiol (515 mg, 1,40 mmol, 1,0 equiv) und Natriumacetat (172 mg, 2,10 mmol, 1,5 equiv) wurden in Etanol (10 ml) suspendiert und anschließend mit Ethylbromacetat (0,17 ml, 1,54 mmol, 1,1 equiv) versetzt. Das resultierende Reaktionsgemisch wurde 3 h zum Sieden erhitzt. Nach dem Abkühlen auf Raumtemperatur entfernte das Lösungsmittel im Vakuum und extrahierte den Rückstand mit Dichlormethan (50 ml) und Wasser (10 ml). rührte diesen mit Toluol (50 ml) aus. Die organische Phase trocknete man über Magnesiumsulfat und entfernte das Lösungsmittel im Vakuum. Durch abschließende säulenchromatographische Reinigung (Gradient Essigester/Heptan) des resultierenden Rohproduktes konnte Ethyl-{[4-brom-1-(2-fluorphenyl)-5-(6-fluorpyridin-3-yl)-1H-pyrazol-3-yl]sulfanyl}acetat in Form eines weißen Feststoffes isoliert werden (21 mg, 3% der Theorie). ¹H-NMR (400 MHz, CDCl₃, δ, ppm) 8.10 (d, 1H), 7.72 (dt, 1H), 7.48-7.42 (m, 1H), 7.41-7.37 (m, 1H), 7.27-7.23 (m, 1H), 7.07-7.03 (m, 1H), 6.97-6.92 (m, 1H), 4.22 (q, 2H), 3.90 (s, 2H), 1.25 (t, 3H).

In Analogie zu den oben angeführten und an entsprechender Stelle rezitierten Herstellungsbeispielen und unter Berücksichtigung der allgemeinen Angaben zur Herstellung von substituierten Pyrazole erhält man die nachfolgend genannten Verbindungen:

**Tabelle I:**

| | | | | | | |
|---|---|---|---|---|---|---|
| 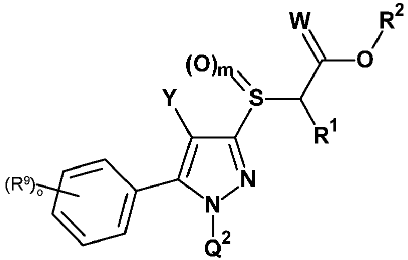 | | | | | | |

| Beispielnummer | R¹ | R² | Y | Q² | m | (R⁹)ₒ |
|---|---|---|---|---|---|---|
| I-001 | H | Et | Cl | 2-F-Ph | 0 | 3,4-DiF |
| I-002 | H | Et | Br | 2-F-Ph | 0 | 3,4-DiF |
| I-003 | H | Et | Br | 2-F-Ph | 0 | 3-Cl-5-F |
| I-004 | H | Et | Br | 2,3-DiF-Ph | 0 | H |
| I-005 | H | H | Br | 2-F-Ph | 0 | 3,4-DiF |
| I-006 | H | H | Br | 2,3-DiF-Ph | 0 | H |
| I-007 | H | Et | Br | 2-F-Ph | 1 | 3,4-DiF |
| I-008 | H | Et | Br | 2,3-DiF-Ph | 2 | H |
| I-009 | H | H | Br | 2-F-Ph | 0 | 3-Cl-5-F |
| I-010 | H | Et | Br | 2,3-DiF-Ph | 1 | H |
| I-011 | H | Et | Br | 2-F-Ph | 0 | 4-I |
| I-012 | H | Et | CN | 2-F-Ph | 0 | 3,4-DiF |
| I-013 | H | Et | Br | 2,5-DiF | 0 | 3,4-DiF |
| I-014 | H | H | Cl | 2-F-Ph | 0 | 3,4-DiF |
| I-015 | Me | Et | Br | 2-F-Ph | 0 | 3,4-DiF |
| I-016 | H | Et | Br | 2,4-DiF-Ph | 0 | 4-F |
| I-017 | H | H | Br | 2-F-Ph | 0 | 4-I |
| I-018 | H | Et | CF₃ | 2-F-Ph | 0 | 3,4-DiF |
| I-019 | H | Et | Br | 4-F-Ph | 0 | 4-F |
| I-020 | H | Et | Br | 2,5-DiF-Ph | 0 | 4-F |
| I-021 | H | Et | Br | 2,4-DiCl-Ph | 0 | 4-F |
| I-022 | Me | H | Br | 2-F-Ph | 0 | 3,4-DiF |
| I-023 | H | Et | Br | 2-H₃CO-Ph | 0 | 4-F |
| I-024 | H | H | NO₂ | 2-F-Ph | 0 | 3,4-DiF |
| I-025 | H | Et | Br | 2,4-DiCl-Ph | 0 | 2,4-DiF |
| I-026 | H | Et | Br | 2,4-DiCl-Ph | 0 | 3,4-DiF |
| I-027 | H | Et | Br | 2,4-DiF-Ph | 0 | 3,4-DiF |

**Tabelle II:**

| | | | | | | |
|---|---|---|---|---|---|---|
| 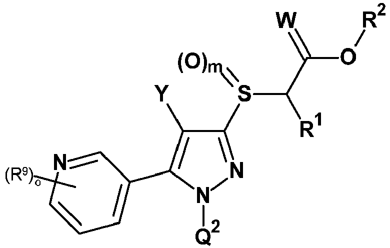 | | | | | | |

| Beispielnummer | R¹ | R² | Y | Q² | m | (R⁹)ₒ |
|---|---|---|---|---|---|---|
| II-001 | H | Et | Br | 2-F-Ph | 0 | 6-F |
| II-002 | H | Et | Br | 2-F-Ph | 0 | 5-F |
| II-003 | H | Et | Br | 2-F-Ph | 2 | 5-F |
| II-004 | H | Et | Br | 2-F-Ph | 0 | 6-OH |
| II-005 | H | H | Br | 2-F-Ph | 0 | 5-F |
| II-006 | H | H | Br | 2-F-Ph | 0 | 6-OH |
| II-007 | H | Et | Br | 2-F-Ph | 0 | 5-Cl |
| II-008 | H | H | Br | 2-F-Ph | 0 | 5-Cl |
| II-009 | H | Et | Br | 2-F-Ph | 2 | 6-OH |
| II-010 | H | Et | Br | 2-F-Ph | 1 | 6-OH |
| II-011 | H | Et | Br | 2-F-Ph | 1 | 5-F |
| II-012 | H | Et | Br | 2-F-Ph | 0 | 6-Cl |
| II-013 | H | Et | Br | 2-F-Ph | 1 | 5-Cl |
| II-014 | H | Et | Br | 2-F-Ph | 0 | H |
| II-015 | H | Et | Br | 2-F-Ph | 0 | 6-Br |
| II-016 | H | H | Br | 2-F-Ph | 0 | 6-Cl |
| II-017 | H | H | Br | 2-F-Ph | 0 | H |

**Tabelle III:**

| 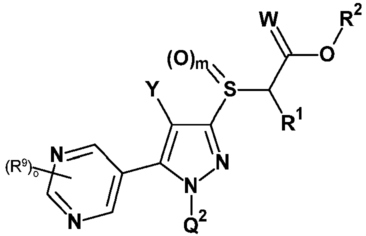 | | | | | | |
|---|---|---|---|---|---|---|
| Beispielnummer | R¹ | R² | Y | Q² | m | (R⁹)ₒ |
| III-001 | H | Et | Br | 2-F-Ph | 0 | H |
| III-002 | H | H | Br | Ph | 0 | H |
| III-003 | H | Et | Br | Ph | 0 | H |
| III-004 | H | Et | Br | 2,3-DiF-Ph | 0 | H |
| III-005 | H | H | Br | 2,3-DiF-Ph | 0 | H |

**Tabelle IV:**

| | | | | | | |
|---|---|---|---|---|---|---|
| 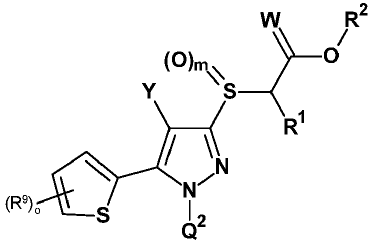 | | | | | | |

| Beispielnummer | R¹ | R² | Y | Q² | m | (R⁹)ₒ |
|---|---|---|---|---|---|---|
| IV-001 | H | Et | Br | 2-F-Ph | 0 | 4-Cl |

**Tabelle V:**

| 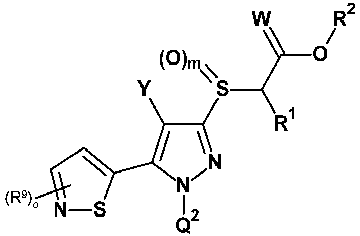 | | | | | | |
|---|---|---|---|---|---|---|
| Beispielnummer | R¹ | R² | Y | Q² | m | (R⁹)ₒ |
| V-001 | H | Et | Br | 2-F-Ph | 0 | 4-Me |
| V-002 | H | H | Br | 2-F-Ph | 0 | 4-Me |

**Tabelle VI:**

| 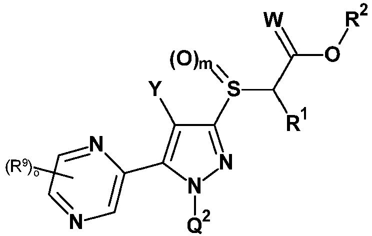 | | | | | | |
|---|---|---|---|---|---|---|
| Beispielnummer | R¹ | R² | Y | Q² | m | (R⁹)ₒ |
| VI-001 | H | Et | Br | 2-F-Ph | 0 | H |
| VI-002 | H | H | Br | 2-F-Ph | 0 | H |

**Tabelle VII:**

| 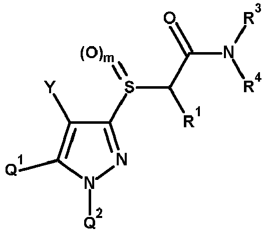 | | | | | | | |
|---|---|---|---|---|---|---|---|
| Beispielnummer | R¹ | Y | Q¹ | Q² | m | R³ | R⁴ |
| VII-001 | H | Cl | 3,4-DiF-Ph | 2-F-Ph | 0 | H | CH₂CO₂CH₃ |
| VII-002 | H | Cl | 3,4-DiF-Ph | 2-F-Ph | 0 | Me | CH₂CO₂CH₃ |
| VII-003 | H | Br | 3,4-DiF-Ph | 2-F-Ph | 0 | H | CH₂CO₂CH₂CH₃ |
| VII-004 | H | NO₂ | 3,4-DiF-Ph | 2-F-Ph | 0 | H | CH₂CO₂CH₃ |
| VII-005 | H | Br | Ph | 2-F-Ph | 0 | H | CH₂CO₂CH₃ |
| VII-006 | H | Br | 3-Pyridyl | 2-F-Ph | 0 | H | CH₂CH₂CO₂CH₃ |
| VII-007 | H | Br | 3-Pyridyl | 2-F-Ph | 0 | H | CH₂CO₂CH₃ |
| VII-008 | H | Br | 3-Pyridyl | 2-F-Ph | 0 | Me | CH₂CO₂CH₃ |
| VII-009 | H | Br | 5-F-3-Pyridyl | 2-F-Ph | 0 | H | CH₂CO₂CH₃ |
| VII-010 | H | Br | 5-F-3-Pyridyl | 2-F-Ph | 0 | H | CH₂CH₂CO₂CH₃ |
| VII-011 | H | Br | 3,4-DiF-Ph | 2-F-Ph | 0 | Me | CH₂CO₂CH₃ |

**Tabelle VIII:**

| 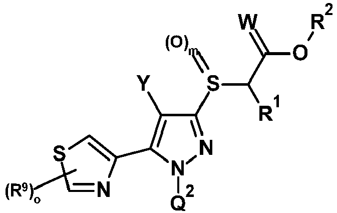 | | | | | | |
|---|---|---|---|---|---|---|
| Beispielnummer | R¹ | R² | Y | Q² | m | (R⁹)ₒ |
| VIII-001 | H | Et | Br | 2-F-Ph | 0 | 2-Cl |

### Spektroskopische Daten ausgewählter Tabellenbeispiele:

Ausgewählte detaillierte Synthesebeispiele für die erfindungsgemäßen Verbindungen der allgemeinen Formeln (I) sind im Folgenden aufgeführt. Die ¹H-NMR-, ¹³C-NMR- und ¹⁹F-NMR-spektroskopischen Daten, die für die in den nachfolgenden Abschnitten beschriebenen chemischen Beispiele angegeben sind, (400 MHz bei ¹H-NMR und 150 MHz bei ¹³C-NMR und 375 MHz bei ¹⁹F-NMR Lösungsmittel CDCl₃, CD₃OD oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Bei Diastereomerengemischen werden entweder die jeweils signifikanten Signale beider Diastereomere oder das charakteristische Signal des Hauptdiastereomers angegeben. Die verwendeten Abkürzungen für chemische Gruppen haben beispielsweise die nachfolgenden Bedeutungen: Me = CH₃, Et = CH₂CH₃, t-Hex = C(CH₃)₂CH(CH₃)₂, t-Bu = C(CH₃)₃, n-Bu = unverzweigtes Butyl, n-Pr = unverzweigtes Propyl, i-Pr = verzweigtes Propyl, c-Pr = Cyclopropyl, c-Hex = Cyclohexyl.

Die nachfolgend aufgeführten spektroskopischen Daten ausgewählter Tabellenbeispiele wurden über klassische ¹H-NMR-Interpretation oder über NMR-Peak-Listenverfahren ausgewertet.

### Klassische ¹H-NMR-Interpretation

### Synthesebeispiel No. I-001

¹H-NMR (400 MHz, CDCl₃, δ, ppm) 7.42 (m, 2H), 7.25 (m, 1H), 7.12 (m, 2H), 7.07 (m, 1H), 4.20 (q, 2H), 3.87 (s, 2H), 1.24 (t, 3H).

### Synthesebeispiel No. I-003

¹H-NMR(400 MHz, CDCl₃, δ, ppm) 8.46 (d, 1H), 8.27 (d, 1H), 7.47 (m, 1H), 7.39 (m, 2H), 7.26 (m, 1H), 7.05 (m, 1H), 4.22 (q, 2H), 3.90 (s, 2H), 1.26 (t, 3H).

### Synthesebeispiel No. I-004

¹H-NMR (400 MHz, CDCl₃, δ, ppm) 7.35 (m, 3H), 7.26(m, 2H), 7.12 (m, 3H), 4.22 (q, 2H), 3.90 (s, 2H), 1.26 (t, 3H).

### Synthesebeispiel No. I-006

¹H-NMR (400 MHz, DMSO-d⁶, δ, ppm) 7.57 (m, 1H), 7.45-7.28 (m, 7H), 3.96 (s, 2H).

### Synthesebeispiel No. I-007

¹H-NMR (400 MHz, CDCl₃, δ, ppm) 7.44 (m, 2H), 7.26 (m, 1H), 7.12 (m, 3H), 6.99 (m, 1H), 4.40 (s, 2H), 4.26 (q, 2H), 1.27 (t, 3H).

### Synthesebeispiel No. I-009

¹H-NMR (400 MHz, CDCl₃, δ, ppm) 10.80 (br s,1H), 7.42 (m, 2H), 7.25 (m, 1H), 7.11 (m, 3H), 3.87 (s, 2H).

### Synthesebeispiel No. 1-010

¹H-NMR (400 MHz, CDCl₃, δ, ppm) 7.64 (m, 1H), 7.47 (m, 4H), 7.33 (m, 3H), 4.55 (q, 2H), 4.15 (m, 2H), 1.16 (t, 3H).

### Synthesebeispiel No. II-002

¹H-NMR (400 MHz, CDCl₃, δ, ppm) 8.45 (d, 1H), 8.27 (d, 1H), 7.46 (m, 1H), 7.42-7.36 (m, 2H), 7.27 (m, 1H), 7.05 (m, 1H), 4.22 (q, 2H), 3.90 (s, 2H), 1.25 (t, 3H).

### Synthesebeispiel No. III-002

¹H-NMR (400 MHz, CDCl₃, δ, ppm) 9.23 (s, 1H), 8.67 (s, 2H), 7.40 (m, 3H), 7.18 (m, 2H), 3.90 (s, 2H).

### Synthesebeispiel No. III-003

¹H-NMR (400 MHz, DMSO-d⁶, δ, ppm) 9.23 (s, 1H), 8.77 (s, 2H), 7.43 (m, 3H), 7.29 (m, 2H), 4.13 (q, 2H), 4.09 (s, 2H), 1.16 (t, 3H).

### Synthesebeispiel No. IV-001

¹H-NMR(400 MHz, CDCl₃, δ, ppm) 7.44 (m, 2H), 7.26 (m, 1H), 7.16 (m, 1H), 6.86 (d, 1H), 6.81 (d, 1H), 4.19 (q, 2H), 3.86 (s, 2H), 1.23 (t, 3H).

### Synthesebeispiel No. V-001

¹H-NMR (400 MHz, CDCl₃, δ, ppm) 7.43 (m, 1H), 7.31 (m, 1H), 7.29 (m, 1H), 7.18 (m, 1H), 7.10 (s, 1H), 4.20 (q, 2H), 3.87 (s, 2H), 2.25 (s, 3H), 1.24 (t, 3H).

### Synthesebeispiel No. V-002

¹H-NMR (400 MHz, CDCl₃, δ, ppm) 7.53 (m, 1H), 7.43 (m, 1H), 7.32 (m, 1H), 7.21 (m, 1H), 7.13 (s, 1H), 3.86 (s, 2H), 2.46 (s, 3H).

### Synthesebeispiel No. II-003

¹H-NMR (400 MHz, CDCl₃, δ, ppm) 8.50 (d, 1H), 8.28 (s, 1H), 7.53-7.39 (m, 3H), 7.28 (m, 1H), 7.09 (m, 1H), 4.41 (s, 2H), 4.24 (m, 2H), 1.27 (t, 3H).

### Synthesebeispiel No. II-004

¹H-NMR(400 MHz, CDCl₃, δ, ppm) 11.09 (brs, 1H), 8.25 (s, 1H), 7.57-7.51 (m, 2H), 7.46-7.42 (m, 1H), 7.38 (dd, 1H), 7.31-7.25 (m, 2H), 4.07 (q, 2H), 4.01 (s, 2H), 1.10 (t, 3H).

### Synthesebeispiel No. 1-011

¹H-NMR (400 MHz, CDCl₃, δ, ppm) 7.80 (d, 2H), 7.57-7.48 (m, 2H), 7.34-7.28 (m, 2H), 7.05 (d, 2H), 4.09 (q, 2H), 3.97 (s, 2H), 1.11 (t, 3H).

### Synthesebeispiel No. II-005

¹H-NMR (400 MHz, CDCl₃, δ, ppm) 8.50 (d, 1H), 8.29 (d, 1H), 7.47-7.39 (m, 3H), 7.27 (m, 1H), 7.10-7.05 (m, 1H), 3.91 (s, 2H).

### Synthesebeispiel No. II-006

¹H-NMR(400 MHz, CDCl₃, δ, ppm) 11.09 (brs, 1H), 8.29 (d, 1H), 7.56-7.53 (m, 2H), 7.50-7.44 (m, 2H), 7.36-7.27 (m, 2H), 3.96 (s, 2H).

### Synthesebeispiel No. II-007

¹H-NMR(400 MHz, CDCl₃, δ, ppm) 8.54 (s, 1H), 8.31 (s, 1H), 7.66 (s, 1H), 7.49-7.36 (m, 2H), 7.27-7.23 (m, 1H), 7.08-7.03 (m, 1H), 4.21 (q, 2H), 3.90 (s, 2H), 1.26 (t, 3H).

### Synthesebeispiel No. I-012

¹H-NMR (400 MHz, CDCl₃, δ, ppm) 7.48-7.43 (m, 2H), 7.27-7.26 (m, 1H), 7.20-7.07 (m, 4H), 4.21 (q, 2H), 3.93 (s, 2H), 1.26 (t, 3H).

### Synthesebeispiel No. II-008

¹H-NMR(400 MHz, CDCl₃, δ, ppm) 8.54 (s, 1H), 8.31 (s, 1H), 7.66 (s, 1H), 7.49-7.36 (m, 2H), 7.27-7.23 (m, 1H), 7.08-7.03 (m, 1H), 3.92 (s, 2H).

### Synthesebeispiel No. II-009

¹H-NMR (400 MHz, CDCl₃, δ, ppm) 11.26 (s, 1H), 8.71 (m, 2H), 8.09 (s, 1H), 7.66-7.59 (m, 1H), 7.50-7.43 (m, 1H), 7.37-7.24 (m, 2H), 7.08-7.03 (m, 1H), 4.77 (s, 2H), 3.95 (q, 2H), 0.95 (t, 3H).

### Synthesebeispiel No. II-010

¹H-NMR (400 MHz, CDCl₃, δ, ppm) 11.26 (s, 1H), 8.59 (d, 1H), 8.01 (d, 1H), 7.91 (d, 1H), 7.61-7.56 (m, 1H), 7.48-7.43 (m, 1H), 7.32-7.24 (m, 2H), 4.25-4.22 (d, 1H), 4.03-3.96 (m, 3H), 1.04 (t, 3H).

### Synthesebeispiel No. I-013

¹H-NMR(400 MHz, CDCl₃, δ, ppm) 7.46-7.35 (m, 2H), 7.27-7.18 (m, 1H), 7.17-7.02 (m, 2H), 6.97-6.92 (m, 1H), 4.22 (q, 2H), 3.89 (s, 2H), 1.24 (t, 3H).

### Synthesebeispiel No. II-011

¹H-NMR (400 MHz, CDCl₃, δ, ppm) 8.51 (s, 1H), 8.28 (s, 1H), 7.53-7.38 (m, 3H), 7.32-7.26 (m, 1H), 7.12-7.07 (m, 1H), 4.40 (d, 2H), 4.25 (q, 2H), 1.27 (t, 3H).

### Synthesebeispiel No. II-014

¹H-NMR (400 MHz, CDCl₃, δ, ppm) 7.80 (d, 2H), 7.57-7.48 (m, 2H), 7.34-7.28 (m, 2H), 7.05 (d, 2H), 3.97 (s, 2H).

### Synthesebeispiel No. III-004

¹H-NMR (400 MHz, CDCl₃, δ, ppm) 9.22 (s, 1H), 8.67 (s, 2H), 7.25-7.22 (m, 3H), 4.22 (q, 2H), 3.91 (s, 2H), 1.26 (t, 3H).

### Synthesebeispiel No. I-015

¹H-NMR (400 MHz, CDCl₃, δ, ppm) 7.42-7.36 (m, 2H), 7.22 (m, 1H), 7.16-6.95 (m, 4H), 4.21-4.13 (m, 3H), 1.62 (d, 3H), 1.22 (t, 3H).

### Synthesebeispiel No. VI-001

¹H-NMR (400 MHz, CDCl₃, δ, ppm) 8.94 (s, 1H), 8.68 (m, 1 H), 8.59 (m, 1H), 7.55-7.46 (m, 2H), 7.34-7.27 (m, 2H), 4.10 (q, 2H), 4.01 (s, 2H), 1.12 (t, 3H).

### Synthesebeispiel No. II-012

¹H-NMR (400 MHz, CDCl₃, δ, ppm) 8.24 (m, 1H), 7.60 (dd, 1H), 7.46 (dt, 1H), 7.37 (m, 1H), 7.33 (m, 1H), 7.23 (m, 1H), 7.05 (dt, 1H), 4.21 (q, 2H), 3.89 (s, 2H), 1.25 (t, 3H).

### Synthesebeispiel No. I-016

¹H-NMR (400 MHz, CDCl₃, δ, ppm) 7.43 (m, 1H), 7.25-6.95 (m, 3H), 6.84 (m, 1H), 4.38 (s, 2H), 4.25 (q, 2H), 1.27 (t, 3H).

### Synthesebeispiel No. II-013

¹H-NMR (400 MHz, CDCl₃, δ, ppm) 8.59 (m, 1H), 8.32 (m, 1H), 7.66 (m, 1H), 7.53-7.44 (m, 2H), 7.30 (m, 1H), 7.10 (m, 1H), 4.40 (s, 2H), 4.25 (q, 2H), 1.27 (t, 3H).

### Synthesebeispiel No. I-019

¹H-NMR(400 MHz, CDCl₃, δ, ppm) 7.25-7.22 (m, 2H), 7.17-7.13 (m, 2H), 7.10-7.05 (m, 2H), 7.01-6.97 (m, 2H), 4.22 (q, 2H), 3.89 (s, 2H), 1.26 (t, 3H).

### Synthesebeispiel No. I-017

¹H-NMR (400 MHz, CDCl₃, δ, ppm) 12.81 (bs, 1H), 7.78 (d, 2H), 7.59-7.48 (m, 2H), 7.33-7.29 (m, 2H), 7.07 (d, 2H), 3.92 (s, 2H).

### Synthesebeispiel No. I-020

¹H-NMR (400 MHz, CDCl₃, δ, ppm) 7.25-7.23 (m, 2H), 7.20-7.16 (m, 1H), 7.07-6.95 (m, 4H), 4.22 (q, 2H), 3.89 (s, 2H), 1.26 (t, 3H).

### Synthesebeispiel No. I-021

¹H-NMR (400 MHz, CDCl₃, δ, ppm) 7.41 (m, 1H), 7.27 (m, 1H), 7.24-7.20 (m, 2H), 7.04-7.00 (m, 2H), 4.20 (q, 2H), 3.88 (s, 2H), 1.25 (t, 3H).

### Synthesebeispiel No. I-022

¹H-NMR (400 MHz, d⁶-DMSO, δ, ppm) 7.71-7.12 (m, 7H), 4.15 (m, 1H), 1.52 (d, 3H).

### Synthesebeispiel No. I-023

¹H-NMR (400 MHz, CDCl₃, δ, ppm) 7.39-7.30 (m, 2H), 7.24-7.20 (m, 2H), 7.02-6.95 (m, 3H), 6.79 (d, 1H), 4.20 (q, 2H), 3.87 (s, 2H), 3.43 (s, 3H), 1.25 (t, 3H).

### Synthesebeispiel No. I-024

¹H-NMR (400 MHz, d⁶-DMSO, δ, ppm) 12.85 (bs, 1H), 7.75 (m, 1H), 7.64 (m, 1H), 7.58-7.45 (m, 2H), 7.37-7.28 (m, 3H), 4.02 (s, 2H).

### Synthesebeispiel No. II-015

¹H-NMR (400 MHz, CDCl₃, δ, ppm) 8.22 (m, 1H), 7.49-7.38 (m, 4H), 7.26 (m, 1H, 7.05 (dt, 1H), 4.20 (q, 2H), 3.89 (s, 2H), 1.24 (t, 3H).

### Synthesebeispiel No. I-016

¹H-NMR (400 MHz, CDCl₃, δ, ppm) 8.26 (m, 1H), 7.60 (dd, 1H), 7.45-7.40 (m, 2H), 7.35 (d, 1H), 7.26 (m, 1H), 7.10 (m, 1H), 3.89 (s, 2H).

### Synthesebeispiel No. I-025

¹H-NMR (400 MHz, CDCl₃, δ, ppm) 7.38 (m, 1H), 7.31-7.21 (m, 3H), 6.90 (m, 1H), 6.80 (m, 1H), 4.20 (q, 2H), 3.88 (s, 2H), 1.24 (t, 3H).

### Synthesebeispiel No. I-026

¹H-NMR (400 MHz, CDCl₃, δ, ppm) 7.42 (m, 1H), 7.32-7.26 (m, 2H), 7.15-7.08 (m, 2H9, 6.96-6.93 (m, 1H), 4.19 (q, 2H), 3.87 (s, 2H), 1.24 (t, 3H).

### Synthesebeispiel No. I-027

¹H-NMR (400 MHz, CDCl₃, δ, ppm) 7.39 (m, 1H), 7.17-7.08 (m, 2H), 6.98-6.93 (m, 2H), 6.81 (m, 1H), 4.20 (q, 2H), 3.88 (s, 2H), 1.25 (t, 3H).

### Synthesebeispiel No. VII-001

¹H-NMR (400 MHz, CDCl₃, δ, ppm) 7.55 (bs, 1H), 7.47-7.39 (m, 2H), 7.25 (m, 1H), 7.17-7.06 (m, 3H), 6.99 (m, 1H), 4.05 (d, 2H), 3.81 (s, 2H), 3.71 (s, 3H).

### Synthesebeispiel No. I-014

¹H-NMR (400 MHz, CDCl₃, δ, ppm) 7.46-7.39 (m, 2H), 7.25 (m, 1H), 7.14-7.10 (m, 3H), 6.97 (m, 1H), 3.86 (s, 2H).

### Synthesebeispiel No. VII-002

¹H-NMR (400 MHz, CDCl₃, δ, ppm) 7.47-7.36 (m, 2H), 7.25 (m, 1H), 7.15-7.04 (m, 3H), 6.97 (m, 1H), 4.16 (s, 2H), 4,10 (s, 2H), 3.73 (s, 3H), 3.18 (s, 3H).

### Synthesebeispiel No. VIII-001

¹H-NMR (400 MHz, CDCl₃, δ, ppm) 7.53-7.42 (m, 2H), 7.28 (m, 1H), 6.85 (d, 1H), 4.20 (q, 2H), 3.80 (s, 2H), 1.25 (t, 3H).

### Synthesebeispiel No. VII-003

¹H-NMR (400 MHz, CDCl₃, δ, ppm) 7.52 (bt, 1H), 7.47-7.36 (m, 2H), 7.23 (m, 1H), 7.18-7.04 (m, 3H), 7.00 (m, 1H), 4.17 (q, 2H), 4.03 (d, 2H), 3.82 (s, 2H), 1.25 (t, 3H).

### Synthesebeispiel No. VII-004

¹H-NMR (400 MHz, CDCl₃, δ, ppm) 7.47-7.41 (m, 2H), 7.25-7.02 (m, 6H), 4.05 (d, 2H), 3.89 (s, 2H), 3.72 (s, 3H).

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| |
| VI-002: ¹H-NMR(400.6 MHz, d₆-DMSO): |
| δ= 12.8357 (0.6); 8.9379 (3.3); 8.9343 (3.4); 8.6831 (3.2); 8.6772 (3.8); 8.6002 (2.7); 8.5963 (2.8); 8.5939 (2.5); 8.5901 (2.4); 7.5822 (0.6); 7.5779 (0.7); 7.5628 (1.3); 7.5584 (1.5); 7.5434 (0.8); 7.5390 (0.8); 7.5006 (0.6); 7.4958 (0.6); 7.4879 (0.6); 7.4835 (0.6); 7.4756 (0.5); 7.4673 (0.5); 7.3431 (0.8); 7.3406 (1.0); 7.3242 (1.2); 7.3210 (1.6); 7.3107 (1.0); 7.3078 (0.8); 7.3018 (0.8); 7.2900 (0.8); 7.2842 (1.0); 7.2811 (0.8); 7.2633 (0.7); 7.2602 (0.6); 5.7568 (16.0); 3.9748 (10.0); 3.3213 (18.7); 2.6744 (0.8); 2.6698 (1.1); 2.6652 (0.8); 2.5404 (1.1); 2.5236 (3.3); 2.5189 (4.3); 2.5101 (60.8); 2.5056 (133.6); 2.5010 (187.3); 2.4964 (129.6); 2.4918 (58.7); 2.4642 (0.8); 2.3327 (0.8); 2.3280 (1.1); 2.3235 (0.8); 1.9885 (0.7); 1.1689 (0.5); 0.0103 (0.6); 0.0080 (4.4); 0.0064 (1.2); 0.0056 (1.3); 0.0047 (1.7); 0.0039 (2.4); -0.0002 (151.4); -0.0050 (2.1); -0.0058 (1.8); -0.0067 (1.6); -0.0085 (4.4); -0.0106 (0.8); -0.0114 (0.7); -0.0122 (0.6) |
| VII-005: ¹H-NMR(400.6 MHz, CDCl3): |
| δ= 7.3896 (0.6); 7.3866 (0.7); 7.3797 (0.6); 7.3768 (1.0); 7.3724 (3.8); 7.3699 (1.4); 7.3674 (1.8); 7.3623 (0.5); 7.3578 (0.9); 7.3539 (2.2); 7.3391 (0.8); 7.3315 (0.6); 7.2773 (2.3); 7.2735 (2.3); 7.2604 (19.9); 7.2533 (1.5); 7.2015 (0.7); 7.1830 (0.6); 7.1592 (0.5); 7.1453 (0.7); 7.1416 (0.8); 7.1306 (0.7); 7.1287 (0.8); 7.1223 (1.1); 7.1115 (0.6); 7.1079 (0.6); 7.0999 (0.6); 7.0874 (0.6); 5.2998 (6.8); 4.0637 (3.9); 4.0505 (3.9); 3.8259 (8.2); 3.7125 (16.0); 2.0453 (0.6); 1.5502 (4.2); 1.2595 (0.5); 0.0079 (0.8); -0.0002 (29.7); -0.0085 (0.9) |
| II-017: ¹H-NMR(400.6 MHz, d₆-DMSO): |
| δ= 12.8229 (0.9); 8.5977 (3.0); 8.5936 (3.3); 8.5856 (3.3); 8.5815 (3.2); 8.4763 (3.0); 8.4742 (3.3); 8.4706 (3.4); 8.4685 (3.2); 7.7351 (1.6); 7.7309 (1.9); 7.7296 (1.8); 7.7252 (1.6); 7.7153 (1.9); 7.7111 (2.1); 7.7097 (2.3); 7.7054 (1.8); 7.6479 (1.0); 7.6437 (1.2); 7.6285 (2.0); 7.6242 (2.2); 7.6092 (1.2); 7.6048 (1.3); 7.5378 (0.6); 7.5334 (0.6); 7.5251 (0.7); 7.5189 (1.0); 7.5145 (0.9); 7.5061 (1.0); 7.5018 (1.0); 7.4983 (1.0); 7.4938 (0.8); 7.4855 (0.8); 7.4810 (0.8); 7.4662 (2.1); 7.4640 (2.1); 7.4541 (2.0); 7.4519 (2.0); 7.4464 (1.8); 7.4442 (1.9); 7.4343 (1.9); 7.4320 (1.9); 7.3629 (1.2); 7.3602 (1.5); 7.3440 (2.0); 7.3409 (2.4); 7.3209 (2.2); 7.3175 (1.3); 7.2997 (1.2); 7.2946 (1.6); 7.2913 (1.3); 7.2736 (1.1); 7.2705 (1.0); 4.0377 (1.0); 4.0199 (1.0); 3.9568 (16.0); 3.3233 (77.7); 2.6745 (1.0); 2.6699 (1.4); 2.6653 (1.0); 2.5405 (5.3); 2.5237 (4.0); 2.5190 (5.7); 2.5103 (72.3); 2.5057 (159.3); 2.5011 (224.2); 2.4965 (157.2); 2.4919 (71.2); 2.4698 (0.7); 2.3328 (0.9); 2.3282 (1.3); 2.3236 (0.9); 1.9885 (4.8); 1.9083 (1.1); 1.6520 (1.7); 1.3550 (3.2); 1.2353 (0.6); 1.1921 (1.5); 1.1744 (3.0); 1.1566 (1.4); 0.1458 (0.5); 0.0103 (0.6); 0.0080 (5.0); 0.0064 (1.2); 0.0056 (1.2); 0.0048 (1.5); 0.0039 (2.0); 0.0023 (6.3); -0.0002 (174.8); -0.0050 (3.1); -0.0058 (2.6); -0.0067 (2.1); -0.0085 (5.3); -0.0106 (1.1); -0.0114 (1.0); -0.0122 (0.9); -0.0138 (0.5); -0.0146 (0.5); - 0.1493 (0.5) |
| III-005: ¹H-NMR(400.6 MHz, d₆-DMSO): |
| δ= 12.8568 (0.7); 9.2554 (7.2); 8.8381 (16.0); 7.6143 (0.7); 7.5956 (0.6); 7.4214 (0.7); 7.4175 (1.0); 7.4023 (0.8); 7.3846 (0.5); 7.3806 (0.6); 7.3719 (0.5); 7.3643 (0.6); 7.3599 (0.7); 7.3511 (0.6); 7.3467 (0.6); 6.8702 (0.6); 5.7567 (1.4); 3.9818 (9.2); 3.3199 (73.7); 2.6790 (0.6); 2.6745 (1.3); 2.6698 (1.8); 2.6651 (1.3); 2.6605 (0.6); 2.5404 (6.8); 2.5326 (1.4); 2.5280 (1.6); 2.5236 (5.4); 2.5189 (6.6); 2.5101 (98.2); 2.5056 (220.8); 2.5010 (313.5); 2.4964 (221.6); 2.4918 (101.7); 2.4713 (0.6); 2.4663 (0.7); 2.4614 (0.8); 2.4567 (0.8); 2.3373 (0.6); 2.3326 (1.4); 2.3280 (1.9); 2.3234 (1.4); 2.3188 (0.7); 2.1826 (1.0); 1.9885 (1.5); 1.9081 (0.8); 1.3549 (8.7); 1.2352 (1.9); 1.1744 (1.0); 0.1457 (0.7); 0.0317 (0.5); 0.0080 (6.1); 0.0063 (1.2); 0.0055 (1.3); 0.0046 (1.6); -0.0002 (226.6); -0.0051 (4.1); -0.0059 (3.4); -0.0068 (3.0); -0.0085 (7.2); -0.0139 (0.8); -0.0179 (0.5); - 0.1494 (0.7) |
| VII-006: ¹H-NMR(400.6 MHz, CDCl3): |
| δ= 8.6037 (1.0); 8.5996 (1.0); 8.5916 (1.0); 8.5875 (1.0); 8.5004 (1.3); 8.4964 (1.3); 7.6568 (0.7); 7.6523 (0.9); 7.6469 (0.7); 7.6370 (0.8); 7.6324 (1.0); 7.6271 (0.7); 7.4838 (0.5); 7.4795 (0.6); 7.4645 (1.1); 7.4604 (1.2); 7.4457 (0.9); 7.4414 (0.9); 7.3832 (0.5); 7.3208 (0.8); 7.3189 (0.8); 7.3086 (0.8); 7.3067 (0.8); 7.3010 (0.7); 7.2991 (0.7); 7.2888 (0.7); 7.2868 (0.7); 7.2613 (18.0); 7.2550 (0.7); 7.2518 (0.7); 7.2333 (1.0); 7.0887 (0.6); 7.0855 (0.6); 7.0678 (0.6); 7.0641 (1.0); 7.0604 (0.7); 7.0428 (0.6); 7.0396 (0.6); 5.3002 (6.6); 4.1309 (0.7); 4.1131 (0.7); 3.7656 (8.2); 3.5734 (16.0); 3.5581 (0.9); 3.5427 (2.4); 3.5273 (2.5); 3.5117 (1.0); 2.5239 (1.8); 2.5085 (3.1); 2.4931 (1.7); 2.0454 (3.1); 1.5705 (1.0); 1.2774 (0.9); 1.2596 (1.8); 1.2417 (0.8); 0.0080 (0.7); -0.0002 (27.4); -0.0085 (0.8) |
| VII-007: ¹H-NMR(400.6 MHz, CDCl3): |
| δ= 8.6051 (0.9); 8.6010 (1.0); 8.5929 (1.0); 8.5888 (1.0); 8.4964 (1.2); 8.4926 (1.2); 7.6601 (0.6); 7.6556 (0.8); 7.6503 (0.6); 7.6403 (0.7); 7.6350 (0.9); 7.6304 (0.7); 7.4842 (0.5); 7.4799 (0.6); 7.4649 (0.9); 7.4607 (1.0); 7.4461 (0.6); 7.4418 (0.7); 7.3235 (0.7); 7.3214 (0.7); 7.3113 (0.7); 7.3093 (0.7); 7.3037 (0.6); 7.3017 (0.6); 7.2916 (0.6); 7.2605 (49.9); 7.2372 (0.6); 7.2186 (1.0); 7.0829 (0.6); 7.0797 (0.6); 7.0621 (0.6); 7.0582 (1.0); 7.0544 (0.6); 7.0370 (0.6); 7.0337 (0.5); 5.3001 (4.9); 4.0637 (3.8); 4.0504 (3.8); 3.8357 (8.0); 3.7110 (16.0); 2.0454 (2.1); 1.5474 (2.9); 1.2775 (0.6); 1.2597 (1.2); 1.2418 (0.6); 0.0080 (2.3); -0.0002 (75.2); -0.0085 (2.1) |
| VII-008: ¹H-NMR(400.6 MHz, CDCl3): |
| δ= 8.5875 (0.8); 8.5839 (0.8); 8.5754 (0.9); 8.5718 (0.8); 8.4805 (1.0); 8.4761 (1.3); 7.6535 (0.6); 7.6480 (0.9); 7.6436 (0.8); 7.6337 (0.7); 7.6282 (1.0); 7.6238 (0.8); 7.4739 (0.8); 7.4697 (0.8); 7.4553 (0.6); 7.4508 (0.6); 7.3633 (0.5); 7.3089 (0.8); 7.2968 (0.8); 7.2951 (0.8); 7.2892 (0.8); 7.2769 (0.7); 7.2753 (0.7); 7.2622 (12.6); 7.2449 (0.7); 7.2432 (0.8); 7.2237 (1.1); 7.0597 (0.6); 7.0566 (0.6); 7.0389 (0.6); 7.0352 (0.9); 7.0315 (0.6); 5.3002 (10.6); 4.2356 (1.6); 4.1673 (6.1); 4.1351 (8.0); 4.1309 (1.8); 4.1129 (1.2); 3.9867 (1.8); 3.7333 (16.0); 3.1909 (12.9); 3.0185 (2.9); 2.0451 (6.0); 1.2773 (1.6); 1.2595 (3.4); 1.2417 (1.6); -0.0002 (18.5); -0.0085 (0.6) |
| VII-009: ¹H-NMR(400.6 MHz, CDCl3): |
| δ= 8.4704 (2.1); 8.4635 (2.1); 8.2948 (1.1); 8.2910 (2.0); 8.2873 (1.1); 7.5201 (0.6); 7.5161 (0.6); 7.5010 (1.0); 7.4968 (1.2); 7.4822 (1.0); 7.4780 (1.0); 7.4207 (1.0); 7.4164 (1.2); 7.4139 (1.2); 7.4095 (0.9); 7.4058 (0.6); 7.3989 (1.1); 7.3943 (1.0); 7.3921 (1.0); 7.3875 (0.8); 7.3851 (0.5); 7.2690 (0.7); 7.2610 (26.4); 7.2494 (1.0); 7.1008 (0.7); 7.0976 (0.6); 7.0799 (0.6); 7.0761 (1.0); 7.0724 (0.7); 7.0548 (0.6); 7.0516 (0.6); 5.3002 (5.6); 4.0627 (3.9); 4.0495 (3.9); 3.8378 (8.0); 3.7142 (16.0); 2.0454 (1.2); 1.5671 (2.7); 1.2774 (0.6); 1.2597 (1.4); 0.8989 (0.6); 0.8820 (2.0); 0.8642 (0.8); 0.0080 (1.1); -0.0002 (39.8); - 0.0085 (1.1) |
| VII-010: ¹H-NMR(400.6 MHz, CDCl3): |
| δ= 8.4700 (1.9); 8.4631 (2.0); 8.2975 (1.0); 8.2937 (1.8); 8.2901 (1.0); 7.5105 (0.5); 7.4955 (0.8); 7.4913 (0.9); 7.4768 (0.6); 7.4724 (0.6); 7.4215 (1.2); 7.4170 (1.3); 7.4147 (1.1); 7.4103 (1.3); 7.4064 (0.7); 7.4045 (0.7); 7.4023 (0.8); 7.3995 (1.2); 7.3951 (1.2); 7.3926 (1.1); 7.3899 (0.8); 7.3882 (1.0); 7.3857 (0.6); 7.2859 (0.5); 7.2843 (0.6); 7.2827 (0.6); 7.2813 (0.5); 7.2620 (14.8); 7.1077 (0.6); 7.1045 (0.6); 7.0869 (0.6); 7.0831 (1.0); 7.0794 (0.6); 7.0618 (0.5); 7.0586 (0.5); 5.3004 (4.8); 4.1309 (0.8); 4.1131 (0.8); 3.7682 (7.8); 3.5761 (16.0); 3.5562 (0.9); 3.5409 (2.2); 3.5256 (2.2); 3.5101 (0.9); 2.5218 (1.7); 2.5063 (2.7); 2.4911 (1.6); 2.0454 (3.8); 1.2774 (1.1); 1.2596 (2.3); 1.2418 (1.1); 0.0080 (0.6); -0.0002 (22.3); -0.0085 (0.6) |
| VII-011: ¹H-NMR(400.6 MHz, CDCl3): |
| δ= 7.4393 (0.7); 7.4351 (0.8); 7.4207 (0.6); 7.4162 (0.6); 7.4025 (0.5); 7.3879 (0.6); 7.3835 (0.5); 7.2620 (8.0); 7.2447 (0.7); 7.2255 (1.1); 7.1314 (0.6); 7.1259 (1.0); 7.1220 (0.6); 7.1185 (0.5); 7.1127 (0.6); 7.1103 (0.7); 7.1050 (1.3); 7.1015 (1.0); 7.0914 (0.6); 7.0858 (0.6); 7.0801 (1.1); 7.0619 (0.5); 7.0581 (0.8); 7.0546 (0.5); 6.9874 (0.6); 6.9830 (0.5); 5.3003 (4.2); 4.2298 (1.5); 4.1623 (5.6); 4.1181 (7.0); 3.9701 (1.7); 3.7315 (16.0); 3.1842 (11.4); 3.0133 (2.7); 1.5743 (12.3); 1.2593 (0.7); 1.2563 (0.7); -0.0002 (10.7) |

Gegenstand der vorliegenden Erfindung ist weiterhin die Verwendung einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (I.001) bis (VII.002) und/oder deren Salze, jeweils wie oben definiert,
als Herbizid und/oder Pflanzenwachstumsregulator, vorzugsweise in Kulturen von Nutz- und/oder Zierpflanzen.

Gegenstand der vorliegenden Erfindung ist ferner ein Verfahren zur Bekämpfung von Schadpflanzen und/oder zur Wachstumsregulierung von Pflanzen, dadurch gekennzeichnet, dass eine wirksame Menge
- einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (I.001) bis (VII.002) und/oder deren Salze, jeweils wie oben definiert, oder
- eines erfindungsgemäßen Mittels, wie nachstehend definiert,
auf die (Schad)Pflanzen, (Schad)Pflanzensamen, den Boden, in dem oder auf dem die (Schad)Pflanzen wachsen, oder die Anbaufläche appliziert wird.

Gegenstand der vorliegenden Erfindung ist auch ein Verfahren zur Bekämpfung von unerwünschten Pflanzen, vorzugsweise in Nutzpflanzenkulturen, dadurch gekennzeichnet, dass eine wirksame Menge
- einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (I.001) bis (VII.002) und/oder deren Salze, jeweils wie oben definiert, oder
- eines erfindungsgemäßen Mittels, wie nachstehend definiert,
auf unerwünschte Pflanzen (z.B. Schadpflanzen wie mono- oder dikotyle Unkräuter oder unerwünschte Kulturpflanzen), das Saatgut der unerwünschten Pflanzen (d.h. Pflanzensamen, z.B. Körner, Samen oder vegetative Vermehrungsorgane wie Knollen oder Sprossteile mit Knospen), den Boden, in dem oder auf dem die unerwünschte Pflanzen wachsen, (z.B. den Boden von Kulturland oder Nicht-Kulturland) oder die Anbaufläche (d.h. Fläche, auf der die unerwünschte Pflanzen wachsen werden) appliziert wird.

Gegenstand der vorliegenden Erfindung ist ferner auch Verfahren zur Bekämpfung zur Wachstumsregulierung von Pflanzen, vorzugsweise von Nutzpflanzen, dadurch gekennzeichnet, dass eine wirksame Menge
- einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der allgemeinen Formeln (I.001) bis (VII.002) und/oder deren Salze, jeweils wie oben definiert, oder
- eines erfindungsgemäßen Mittels, wie nachstehend definiert,
die Pflanze, das Saatgut der Pflanze (d.h. Pflanzensamen, z.B. Körner, Samen oder vegetative Vermehrungsorgane wie Knollen oder Sprossteile mit Knospen), den Boden, in dem oder auf dem die Pflanzen wachsen, (z.B. den Boden von Kulturland oder Nicht-Kulturland) oder die Anbaufläche (d.h. Fläche, auf der die Pflanzen wachsen werden) appliziert wird.

Dabei können die erfindungsgemäßen Verbindungen bzw. die erfindungsgemäßen Mittel z.B. im Vorsaat-(ggf. auch durch Einarbeitung in den Boden), Vorauflauf- und/oder Nachauflaufverfahren ausgebracht werden. Im einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Vorzugsweise werden in einem erfindungsgemäßen Verfahren zur Bekämpfung von Schadpflanzen oder zur Wachstumsregulierung von Pflanzen eine oder mehrere Verbindungen der allgemeinen Formel (I) und/oder deren Salze zur Bekämpfung von Schadpflanzen oder zur Wachstumsregulierung in Kulturen von Nutzpflanzen oder Zierpflanzen eingesetzt, wobei die Nutzpflanzen oder Zierpflanzen in einer bevorzugten Ausgestaltung transgene Pflanzen sind.

Die erfindungsgemäßen Verbindungen der allgemeinen Formel (I) und/oder deren Salze eignen sich zur Bekämpfung der folgenden Gattungen von monokotylen und dikotylen Schadpflanzen:
Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Dikotyle Schadpflanzen der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen der Schadpflanzen (Ungräser und/oder Unkräuter) auf die Erdoberfläche appliziert (Vorauflaufverfahren), so wird entweder das Auflaufen der Ungras- bzw. Unkrautkeimlinge vollständig verhindert oder diese wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen und/oder deren Salze in wirtschaftlich bedeutenden transgenen Kulturen von Nutz und Zierpflanzen, z.B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen auch als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz-und Zierpflanzen, z.B. von Getreide wie Weizen, Gerste, Roggen, Hafer, Triticale, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden.

Zahlreiche molekularbiologische Techniken, mit denen neue transgene Pflanzen mit veränderten Eigenschaften hergestellt werden können, sind dem Fachmann bekannt. Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) und/oder deren Salze als Herbizide zur Bekämpfung von Schadpflanzen in Kulturen von Nutz- oder Zierpflanzen, gegebenenfalls in transgenen Kulturpflanzen.

Bevorzugt ist die Verwendung in Getreide, dabei vorzugsweise Mais, Weizen, Gerste, Roggen, Hafer, Hirse, oder Reis, im Vor- oder Nachauflauf.

Bevorzugt ist auch die Verwendung in Soja im Vor- oder Nachauflauf.

Die erfindungsgemäße Verwendung zur Bekämpfung von Schadpflanzen oder zur Wachstumsregulierung von Pflanzen schließt auch den Fall ein, bei dem der Wirkstoff der allgemeinen Formel (I) oder dessen Salz erst nach der Ausbringung auf der Pflanze, in der Pflanze oder im Boden aus einer Vorläufersubstanz ("Prodrug") gebildet wird.

Gegenstand der Erfindung ist auch die Verwendung einer oder mehrerer Verbindungen der allgemeinen Formel (I) oder deren Salzen bzw. eines erfindungsgemäßen Mittels (wie nachstehend definiert) (in einem Verfahren) zur Bekämpfung von Schadpflanzen oder zur Wachstumsregulierung von Pflanzen, dadurch gekennzeichnet, dass man eine wirksame Menge einer oder mehreren Verbindungen der allgemeinen Formel (I) oder deren Salzen auf die Pflanzen (Schadpflanzen, ggf. zusammen mit den Nutzpflanzen) Pflanzensamen, den Boden, in dem oder auf dem die Pflanzen wachsen, oder die Anbaufläche appliziert.

Gegenstand der Erfindung ist auch ein herbizides und/oder pflanzenwachstumsregulierendes Mittel, dadurch gekennzeichnet, dass das Mittel
(a) eine oder mehrere Verbindungen der allgemeinen Formel (I) und/oder deren Salze enthält wie oben definiert, vorzugsweise in einer der als bevorzugt bzw. besonders bevorzugt gekennzeichneten Ausgestaltung, insbesondere eine oder mehrere Verbindungen der Formeln (I.001) bis (VII.002) und/oder deren Salze, jeweils wie oben definiert,
   und
(b) ein oder mehrere weitere Stoffe ausgewählt aus den Gruppen (i) und/oder (ii):
   (i) ein oder mehrere weitere agrochemisch wirksame Stoffe, vorzugsweise ausgewählt aus der Gruppe bestehend aus Insektiziden, Akariziden, Nematiziden, weiteren Herbiziden (d.h. solche, die nicht der oben definierten allgemeinen Formel (I) entsprechen), Fungiziden, Safenern, Düngemitteln und/oder weiteren Wachstumsregulatoren,
   (ii) ein oder mehrere im Pflanzenschutz übliche Formulierungshilfsmittel.

Die weiteren agrochemischen wirksamen Stoffe des Bestandteils (i) eines erfindungsgemäßen Mittels sind dabei vorzugsweise ausgewählt aus der Gruppe der Stoffe, die in "The Pesticide Manual", 16th edition, The British Crop Protection Council und the Royal Soc. of Chemistry, 2012 genannt sind.

Ein erfindungsgemäßes herbizides oder pflanzenwachstumsregulierendes Mittel, umfasst vorzugsweise ein, zwei, drei oder mehr im Pflanzenschutz übliche Formulierungshilfsmittel (ii) ausgewählt aus der Gruppe bestehend aus Tensiden, Emulgatoren, Dispergiermitteln, Filmbildnern, Verdickungsmitteln, anorganischen Salzen, Stäubemitteln, bei 25 °C und 1013 mbar festen Trägerstoffen, vorzugsweise adsorptionsfähigen, granulierten Inertmaterialien, Netzmitteln, Antioxidationsmitteln, Stabilisatoren, Puffersubstanzen, Antischaummitteln, Wasser, organischen Lösungsmitteln, vorzugsweise bei 25 °C und 1013 mbar mit Wasser in jedem beliebigen Verhältnis mischbare organische Lösungsmittel.

Die erfindungsgemäßen Verbindungen der allgemeinen Formel (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der allgemeinen Formel (I) und/oder deren Salze enthalten.

Die Verbindungen der allgemeinen Formel (I) und/oder deren Salze können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen und die Formulierungshilfsmittel wie Inertmaterialien, Tenside, Lösungsmittel und weitere Zusatzstoffe sind dem Fachmann bekannt, und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulaten siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen, vorzugsweise herbizide oder pflanzenwachstumsregulierende Mittel der vorliegenden Erfindung enthalten vorzugsweise eine Gesamtmenge von 0,1 bis 99 Gew.-%, bevorzugt 0,5 bis 95 Gew.-%, weiter bevorzugt 1 bis 90 Gew.-%, insbesondere bevorzugt 2 bis 80 Gew.-%, an Wirkstoffen der allgemeinen Formel (I) und deren Salzen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der allgemeinen Formel (I) oder deren Salze können als solche oder in Form ihrer Zubereitungen (Formulierungen) mit anderen pestizid wirksamen Stoffen, wie z.B. Insektiziden, Akariziden, Nematiziden, Herbiziden, Fungiziden, Safenern, Düngemitteln und/oder Wachstumsregulatoren kombiniert eingesetzt werden, z.B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen der allgemeinen Formel (I) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. in Weed Research 26 (1986) 441-445 oder "The Pesticide Manual", 16th edition, The British Crop Protection Council and the Royal Soc. of Chemistry, 2012 und der dort zitierten Literatur beschrieben sind.

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen der allgemeinen Formel (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z.B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der allgemeinen Formel (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Äußere Bedingungen wie Temperatur, Feuchtigkeit etc. beeinflussen zu einem gewissen Teil die Aufwandmenge der Verbindungen der allgemeinen Formel (I) und/oder deren Salze. Die Aufwandmenge kann dabei innerhalb weiter Grenzen variieren. Für die Anwendung als Herbizid zur Bekämpfung von Schadpflanzen liegt die Gesamtmenge an Verbindungen der allgemeinen Formel (I) und deren Salze vorzugsweise im Bereich von 0,001 bis 10,0 kg/ha, bevorzugt im Bereich von 0,005 bis 5 kg/ha, weiter bevorzugt im Bereich von 0,01 bis 1,5 kg/ha, insbesondere bevorzugt im Bereich von 0,05 bis 1 kg/ha. Dies gilt sowohl für die Anwendung im Vorauflauf oder im Nachauflauf.

Bei der Anwendung von Verbindungen der allgemeinen Formel (I) und/oder deren Salzen als Pflanzenwachstumsregulator, beispielsweise als Halmverkürzer bei Kulturpflanzen, wie sie oben genannt worden sind, vorzugsweise bei Getreidepflanzen wie Weizen, Gerste, Roggen, Triticale, Hirse, Reis oder Mais, liegt die Gesamt-Aufwandmenge vorzugsweise im Bereich von 0,001 bis 2 kg/ha, vorzugsweise im Bereich von 0,005 bis 1 kg/ha, insbesondere im Bereich von 10 bis 500 g/ha, ganz besonders bevorzugt im Bereich von 20 bis 250 g/ha. Dies gilt sowohl für die Anwendung im Vorauflauf oder im Nachauflauf.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Als Kombinationspartner für die erfindungsgemäßen Verbindungen der allgemeinen Formel (I) in erfindungsgemäßen Mitteln (z.B. Mischungsformulierungen oder im Tank-Mix) sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 16th edition, The British Crop Protection Council und the Royal Soc. of Chemistry, 2012 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit ihrem "common name" in der englischsprachigen Variante gemäß International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomeren Formen wie Stereoisomere und optische Isomere umfaßt, auch wenn diese nicht explizit erwähnt sind.

Beispiele für solche herbiziden Mischungspartner sind:
Acetochlor, acifluorfen, acifluorfen-sodium, aclonifen, alachlor, allidochlor, alloxydim, alloxydimsodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlorpotassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenac-sodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl, - dimethylammonium, -diolamin, -ethyl, 2-ethylhexyl, -isobutyl, -isooctyl, -isopropylammonium, - potassium, -triisopropanolammonium und -trolamine, 2,4-DB, 2,4-DB-butyl, -dimethylammonium, isooctyl, -potassium und -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyrsodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, -dimethylammonium und -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosateammonium, -isopropylammonium, -diammonium, -dimethylammonium, -potassium, -sodium und - trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuron-methyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(Dimethoxyphosphoryl)-ethyl-(2,4-dichlorphenoxy)acetat, imazamethabenz, Imazamethabenz-methyl, imazamox, imazamox-ammonium, imazapic, imazapic-ammonium, imazapyr, imazapyr-isopropylammonium, imazaquin, imazaquin-ammonium, imazethapyr, imazethapyrimmonium, imazosulfuron, indanofan, indaziflam, iodosulfuron, iodosulfuron-methyl-sodium, ioxynil, ioxynil-octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, ketospiradox, lactofen, lenacil, linuron, MCPA, MCPA-butotyl, -dimethylammonium, -2-ethylhexyl, -isopropylammonium, -potassium und - sodium, MCPB, MCPB-methyl, -ethyl und -sodium, mecoprop, mecoprop-sodium, und -butotyl, mecoprop-P, mecoprop-P-butotyl, -dimethylammonium, -2-ethylhexyl und -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, neburon, nicosulfuron, nonanoic acid (Pelargonsäure), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuron-ethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, , SYN-523, SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trifluoressigsäure), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazone-methyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, sowie die folgenden Verbindungen:

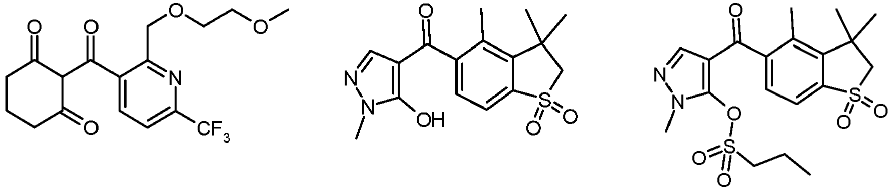

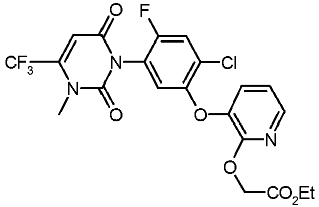

Beispiele für Pflanzenwachstumsregulatoren als mögliche Mischungspartner sind:
Acibenzolar, acibenzolar-S-methyl, 5-Aminolävulinsäure, ancymidol, 6-benzylaminopurine, Brassinolid, Catechin, chlormequat chloride, cloprop, cyclanilide, 3-(Cycloprop-1-enyl)propionsäure, daminozide, dazomet, n-decanol, dikegulac, dikegulac-sodium, endothal, endothal-dipotassium, -disodium, und mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, Jasmonsäure, Jasmonsäuremethylester, maleic hydrazide, mepiquat chloride, 1-methylcyclopropene, 2-(1-naphthyl)acetamide, 1-naphthylacetic acid, 2- naphthyloxyacetic acid, nitrophenolate-mixture, 4-Oxo-4[(2-phenylethyl)amino]buttersäure, paclobutrazol, N-phenylphthalamic acid, prohexadione, prohexadione-calcium, prohydrojasmone, Salicylsäure, Strigolacton, tecnazene, thidiazuron, triacontanol, trinexapac, trinexapac-ethyl, tsitodef, uniconazole, uniconazole-P.

Ebenfalls als Kombinationspartner für die erfindungsgemäßen Verbindungen der allgemeinen Formel (I) kommen beispielsweise die folgenden Safener in Frage:
S1) Verbindungen aus der Gruppe heterocyclischer Carbonsäurederivate:
S1^{a}) Verbindungen vom Typ der Dichlorphenylpyrazolin-3-carbonsäure (S1^{a}), vorzugsweise Verbindungen wie
1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
S1^{b}) Derivate der Dichlorphenylpyrazolcarbonsäure (S1^{b}), vorzugsweise Verbindungen wie 1-(2,4-Dichlorphenyl)-5-methylpyrazol-3-carbonsäureethylester (S1-2), 1-(2,4-Dichlorphenyl)-5-isopropylpyrazol-3-carbonsäureethylester (S1-3), 1-(2,4-Dichlorphenyl)-5-(1,1-dimethyl-ethyl)pyrazol-3-carbonsäureethylester (S1-4) und verwandte Verbindungen, wie sie in EP-A-333131 und EP-A-269806 beschrieben sind;
S1^{c}) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
S1^{d}) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen, wie sie in EP-A-174562 und EP-A-346620 beschrieben sind;
S1^{e}) Verbindungen vom Typ der 5-Benzyl- oder 5-Phenyl-2-isoxazolin-3- carbonsäure, oder der 5,5-Diphenyl-2-isoxazolin-3-carbonsäure(S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-3-carbonsäureethylester (S1-11) ("Isoxadifen-ethyl") oder -n-propylester (S1-12) oder 5-(4-Fluorphenyl)-5-phenyl-2-isoxazolin-3-carbonsäureethylester (S1-13), wie sie in der Patentanmeldung WO-A-95/07897 beschrieben sind.

S2) Verbindungen aus der Gruppe der 8-Chinolinoxyderivate (S2):
S2^{a}) Verbindungen vom Typ der 8-Chinolinoxyessigsäure (S2^{a}), vorzugsweise
(5-Chlor-8-chinolinoxy)essigsäure-(1-methylhexyl)-ester ("Cloquintocet-mexyl") (S2-1),
(5-Chlor-8-chinolinoxy)essigsäure-(1,3-dimethyl-but-1-yl)-ester (S2-2),
(5-Chlor-8-chinolinoxy)essigsäure-4-allyl-oxy-butylester (S2-3),
(5-Chlor-8-chinolinoxy)essigsäure-1-allyloxy-prop-2-ylester (S2-4),
(5-Chlor-8-chinolinoxy)essigsäureethylester (S2-5),
(5-Chlor-8-chinolinoxy)essigsäuremethylester (S2-6),
(5-Chlor-8-chinolinoxy)essigsäureallylester (S2-7),
(5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86750, EP-A-94349 und EP-A-191736 oder EP-A-0 492 366 beschrieben sind, sowie (5-Chlor-8-chinolinoxy)essigsäure (S2-10), deren Hydrate und Salze, beispielsweise deren Lithium-, Natrium-Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
S2^{b}) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.

S3) Wirkstoffe vom Typ der Dichloracetamide (S3), die häufig als Vorauflaufsafener (bodenwirksame Safener) angewendet werden, wie z. B. "Dichlormid" (N,N-Diallyl-2,2-dichloracetamid) (S3-1), "R-29148" (3-Dichloracetyl-2,2,5-trimethyl-1,3-oxazolidin) der Firma Stauffer (S3-2), "R-28725" (3-Dichloracetyl-2,2,-dimethyl-1,3-oxazolidin) der Firma Stauffer (S3-3), "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5), "DKA-24" (N-Allyl-N-[(allylaminocarbonyl)methyl]-dichloracetamid) der Firma Sagro-Chem (S3-6), "AD-67" oder "MON 4660" (3-Dichloracetyl-1-oxa-3-aza-spiro[4,5]decan) der Firma Nitrokemia bzw. Monsanto (S3-7), "TI-35" (1-Dichloracetyl-azepan) der Firma TRI-Chemical RT (S3-8), "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF, "Furilazol" oder "MON 13900" ((RS)-3-Dichloracetyl-5-(2-furyl)-2,2-dimethyloxazolidin) (S3-10), sowie dessen (R)-Isomer (S3-11).
S4) Verbindungen aus der Klasse der Acylsulfonamide (S4):
S4^{a}) N-Acylsulfonamide der Formel (S4^{a}) und deren Salze wie sie in der WO-A-97/45016 beschrieben sind, 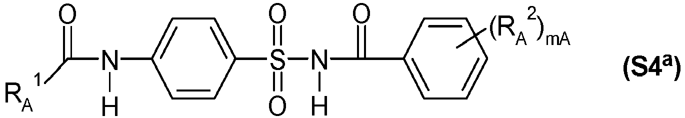 worin
R_{A}¹ (C₁-C₆)Alkyl, (C₃-C₆)Cycloalkyl, wobei die 2 letztgenannten Reste durch v_{A} Substituenten aus der Gruppe Halogen, (C₁-C₄)Alkoxy, (C₁-C₆)Haloalkoxy und (C₁-C₄)Alkylthio und im Falle cyclischer Reste auch durch (C₁-C₄)Alkyl und (C₁-C₄)Haloalkyl substituiert sind;
R_{A}² Halogen, (C₁-C₄)Alkyl, (C₁-C₄)Alkoxy, CF₃,
m_{A} 1 oder 2;
v_{A} ist 0, 1, 2 oder 3 bedeuten;
S4^{b}) Verbindungen vom Typ der 4-(Benzoylsulfamoyl)benzamide der Formel (S4^{b}) und deren Salze, wie sie in der WO-A-99/16744 beschrieben sind, 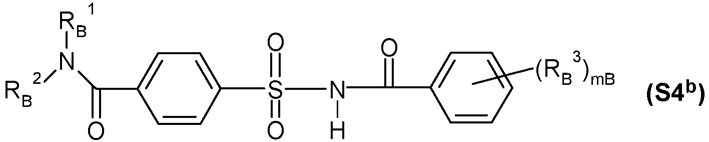
worin
R_{B}¹, R_{B}² unabhängig voneinander Wasserstoff, (C₁-C₆)Alkyl, (C₃-C₆)Cycloalkyl, (C₃-C₆)Alkenyl, (C₃-C₆)Alkinyl,
R_{B}³ Halogen, (C₁-C₄)Alkyl, (C₁-C₄)Haloalkyl oder (C₁-C₄)Alkoxy und
m_{B} 1 oder 2 bedeuten,
z.B. solche worin
R_{B}¹ = Cyclopropyl, R_{B}² = Wasserstoff und (R_{B}³) = 2-OMe ist ("Cyprosulfamide", S4-1),
R_{B}¹= Cyclopropyl, R_{B}² = Wasserstoff und (R_{B}³) = 5-Cl-2-OMe ist (S4-2),
R_{B}¹= Ethyl, R_{B}² = Wasserstoff und (R_{B}³) = 2-OMe ist (S4-3),
R_{B}¹ = Isopropyl, R_{B}² = Wasserstoff und (R_{B}³) = 5-Cl-2-OMe ist (S4-4) und
R_{B}¹ = Isopropyl, R_{B}² = Wasserstoff und (R_{B}³) = 2-OMe ist (S4-5);
S4^{c}) Verbindungen aus der Klasse der Benzoylsulfamoylphenylharnstoffe der Formel (S4^{c}), wie sie in der EP-A-365484 beschrieben sind, 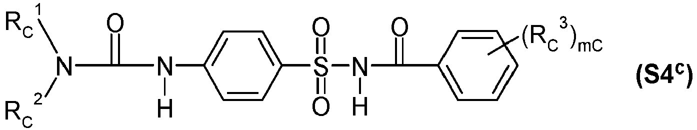
worin
R_{C}¹, R_{C}² unabhängig voneinander Wasserstoff, (C₁-C₈)Alkyl, (C₃-C₈)Cycloalkyl, (C₃-C₆)Alkenyl, (C₃-C₆)Alkinyl,
R_{C}³ Halogen, (C₁-C₄)Alkyl, (C₁-C₄)Alkoxy, CF₃ und
m_{C} 1 oder 2 bedeuten;
beispielsweise
1-[4-(N-2-Methoxybenzoylsulfamoyl)phenyl]-3-methylharnstoff,
1-[4-(N-2-Methoxybenzoylsulfamoyl)phenyl]-3,3-dimethylharnstoff,
1-[4-(N-4,5-Dimethylbenzoylsulfamoyl)phenyl]-3-methylharnstoff;
S4^{d}) Verbindungen vom Typ der N-Phenylsulfonylterephthalamide der Formel (S4^{d}) und deren Salze, die z.B. bekannt sind aus CN 101838227, 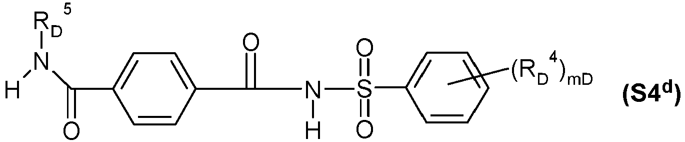
worin
R_{D}⁴ Halogen, (C₁-C₄)Alkyl, (C₁-C₄)Alkoxy, CF₃,
m_{D} 1 oder 2;
R_{D}⁵ Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl bedeutet.

S5) Wirkstoffe aus der Klasse der Hydroxyaromaten und der aromatisch-aliphatischen Carbonsäurederivate (S5), z.B. 3,4,5 -Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen aus der Klasse der Diphenylmethoxyessigsäurederivate (S7), z.B. Diphenylmethoxyessigsäuremethylester (CAS-Reg.Nr. 41858-19-9) (S7-1), Diphenylmethoxyessigsäureethylester oder Diphenylmethoxyessigsäure wie sie in der WO-A-98/38856 beschrieben sind.
S8) Verbindungen der Formel (S8), wie sie in der WO-A-98/27049 beschrieben sind, 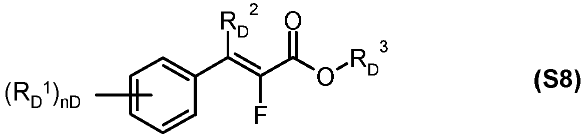 worin die Symbole und Indizes folgende Bedeutungen haben:
R_{D}¹ ist Halogen, (C₁-C₄)Alkyl, (C₁-C₄)Haloalkyl, (C₁-C₄)Alkoxy, (C₁-C₄)Haloalkoxy,
R_{D}² ist Wasserstoff oder (C₁-C₄)Alkyl,
R_{D}³ ist Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze,
n_{D} ist eine ganze Zahl von 0 bis 2.

S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr.: 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b}),
wie sie in der WO-A-2007/023719 und WO-A-2007/023764 beschrieben sind,
worin 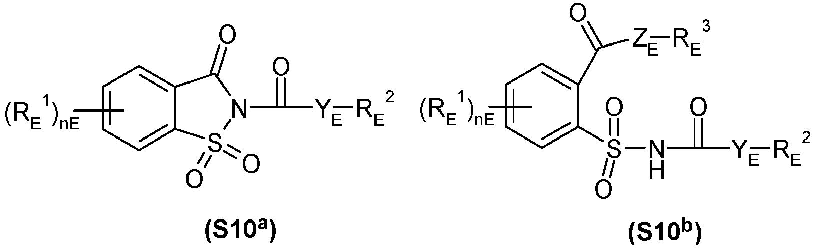
R_{E}¹ Halogen, (C₁-C₄)Alkyl, Methoxy, Nitro, Cyano, CF₃, OCF₃
Y_{E}, Z_{E} unabhängig voneinander O oder S,
n_{E} eine ganze Zahl von 0 bis 4,
R_{E}² (C₁-C₁₆)Alkyl, (C₂-C₆)Alkenyl, (C₃-C₆)Cycloalkyl, Aryl; Benzyl, Halogenbenzyl,
R_{E}³ Wasserstoff oder (C₁-C₆)Alkyl bedeuten.

S11) Wirkstoffe vom Typ der Oxyimino-Verbindungen (S11), die als Saatbeizmittel bekannt sind, wie z. B.
"Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
"Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
"Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.

S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
"Naphthalic anhydrid" (1,8-Naphthalindicarbonsäureanhydrid) (S13-1), das als Saatbeiz-Safener für Mais gegen Schäden von Thiocarbamatherbiziden bekannt ist,
"Fenclorim" (4,6-Dichlor-2-phenylpyrimidin) (S13-2), das als Safener für Pretilachlor in gesätem Reis bekannt ist,
"Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
"CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
"MG 191" (CAS-Reg.Nr. 96420-72-3) (2-Dichlormethyl-2-methyl-1,3-dioxolan) (S13-5) der Firma Nitrokemia, das als Safener für Mais bekannt ist,
"MG 838" (CAS-Reg.Nr. 133993-74-5) (2-propenyl 1-oxa-4-azaspiro[4.5]decan-4-carbodithioat) (S13-6) der Firma Nitrokemia
"Disulfoton" (O,O-Diethyl S-2-ethylthioethyl phosphordithioat) (S13-7),
"Dietholate" (O,O-Diethyl-O-phenylphosphorothioat) (S13-8),
"Mephenate" (4-Chlorphenyl-methylcarbamat) (S13-9).

S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist,
"Daimuron" oder "SK 23" (1-(1-Methyl-1-phenylethyl)-3-p-tolyl-harnstoff), das als Safener für Reis gegen Schäden des Herbizids Imazosulfuron bekannt ist,
"Cumyluron" = "JC-940" (3-(2-Chlorphenylmethyl)-1-(1-methyl-1-phenyl-ethyl)harnstoff, siehe JP-A-60087270), das als Safener für Reis gegen Schäden einiger Herbizide bekannt ist,
"Methoxyphenon" oder "NK 049" (3,3'-Dimethyl-4-methoxy-benzophenon), das als Safener für Reis gegen Schäden einiger Herbizide bekannt ist,
"CSB" (1-Brom-4-(chlormethylsulfonyl)benzol) von Kumiai, (CAS-Reg.Nr. 54091-06-4), das als Safener gegen Schäden einiger Herbizide in Reis bekannt ist.

S15) Verbindungen der Formel (S15) oder deren Tautomere, 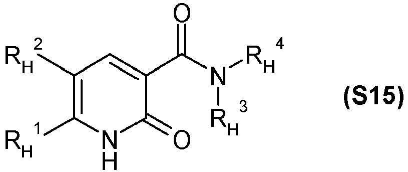 wie sie in der WO-A-2008/131861 und WO-A-2008/131860 beschrieben sind,
worin
- R_{H}¹: einen (C₁-C₆)Haloalkylrest bedeutet und
- R_{H}²: Wasserstoff oder Halogen bedeutet und
- R_{H}³, R_{H}⁴: unabhängig voneinander Wasserstoff, (C₁-C₁₆)Alkyl, (C₂-C₁₆)Alkenyl oder (C₂-C₁₆)Alkinyl,

wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
bedeutet oder
R_{H}³ (C₁-C₄)-Alkoxy, (C₂-C₄)Alkenyloxy, (C₂-C₆)Alkinyloxy oder (C₂-C₄)Haloalkoxy bedeutet und
R_{H}⁴ Wasserstoff oder (C₁-C₄)-Alkyl bedeutet oder
R_{H}³ und R_{H}⁴ zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.

S16) Wirkstoffe, die vorrangig als Herbizide eingesetzt werden, jedoch auch Safenerwirkung auf Kulturpflanzen aufweisen, z. B.

| | |
|---|---|
| (2,4-Dichlorphenoxy)essigsäure | (2,4-D), |
| (4-Chlorphenoxy)essigsäure, | |
| (R,S)-2-(4-Chlor-o-tolyloxy)propionsäure | (Mecoprop), |
| 4-(2,4-Dichlorphenoxy)buttersäure | (2,4-DB), |
| (4-Chlor-o-tolyloxy)essigsäure | (MCPA), |
| 4-(4-Chlor-o-tolyloxy)buttersäure, | |
| 4-(4-Chlorphenoxy)buttersäure, | |
| 3,6-Dichlor-2-methoxybenzoesäure | (Dicamba), |
| 1-(Ethoxycarbonyl)ethyl-3,6-dichlor-2-methoxybenzoat (Lactidichlor-ethyl). | |

Bevorzugte Safener in Kombination mit den erfindungsgemäßen Verbindungend der Formel (I) und/oder deren Salze, insbesondere mit den Verbindungen der Formeln (I.1) bis (I.182) und/oder deren Salze sind: Cloquintocet-mexyl, Cyprosulfamid, Fenchlorazol-ethylester, Isoxadifen-ethyl, Mefenpyr-diethyl, Fenclorim, Cumyluron, S4-1 und S4-5, und besonders bevorzugte Safener sind: Cloquintocet-mexyl, Cyprosulfamid, Isoxadifen-ethyl und Mefenpyr-diethyl.

### Biologische Beispiele:

### 1. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 l/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen).

### Unerwünschte Pflanzen / Weeds:

| | | | |
|---|---|---|---|
| ALOMY: | *Alopecurus myosuroides* | SETVI: | *Setaria viridis* |
| AMARE: | *Amaranthus retroflexus* | KCHSC: | *Bassia scoparia* |
| CYPES: | *Cyperus esculentus* | ECHCG: | *Echinochloa crus-galli* |
| LOLRI: | *Lolium rigidum* | STEME: | *Stellaria media* |
| VERPE: | *Veronica persica* | MATIN: | *Tripleurospermum inodorum* |
| POAAN: | *Poa annua* | ABUTH: | *Abutylon threophrasti* |
| DIGSA: | *Digitaria sanguinalis* | | |

### 1. Vorauflaufwirksamkeit

Wie die Ergebnisse aus den Tabellen 1.1 bis 1.10 zeigen, weisen erfindungsgemäße Verbindungen eine gute herbizide Vorauflaufwirksamkeit gegen ein breites Spektrum von Ungräsern und Unkräutern auf.

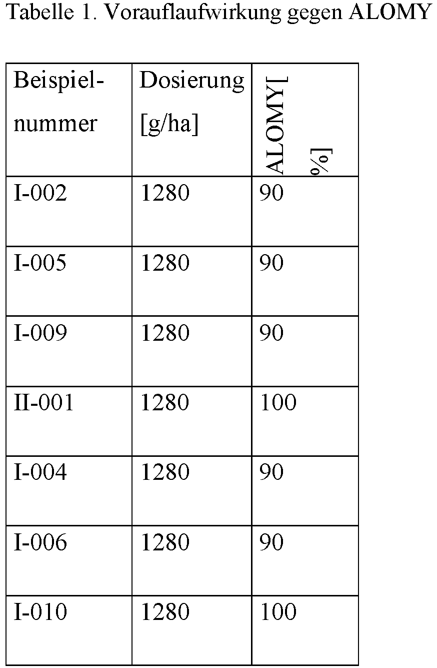

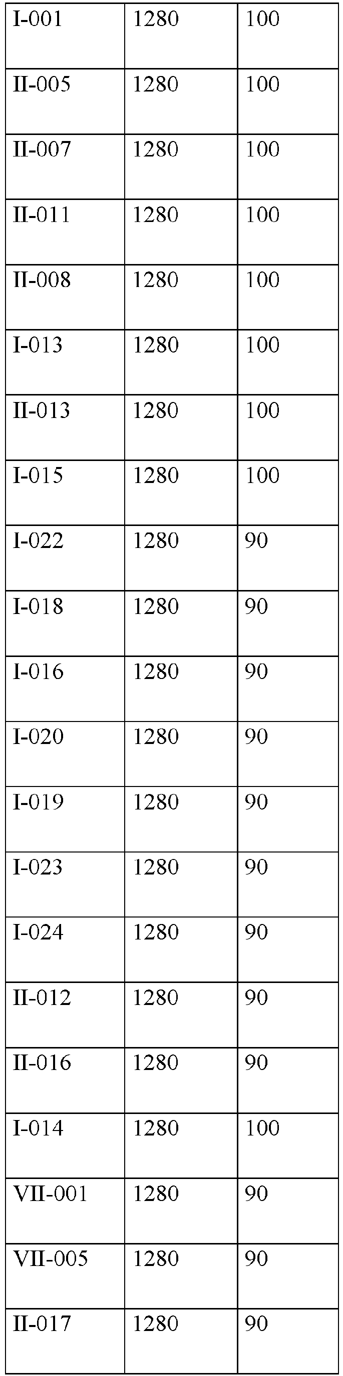

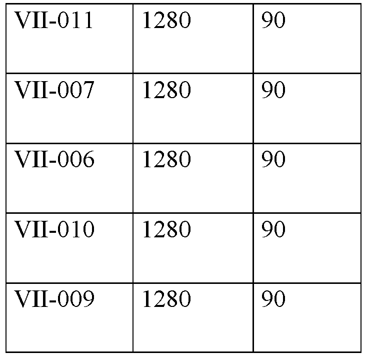

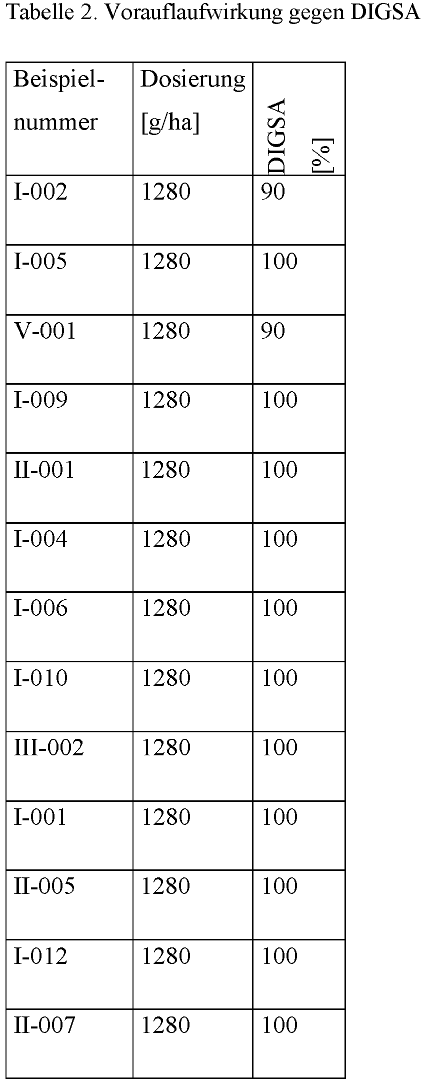

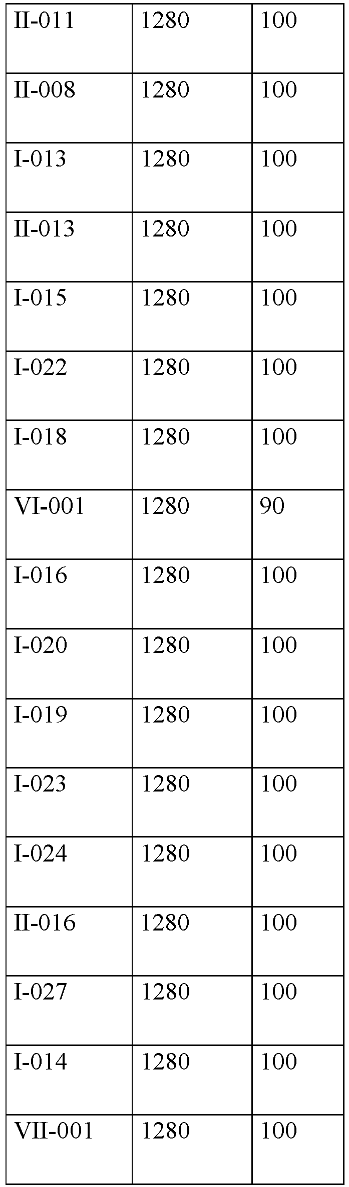

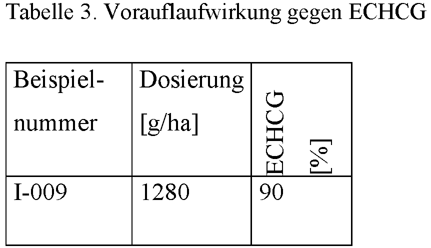

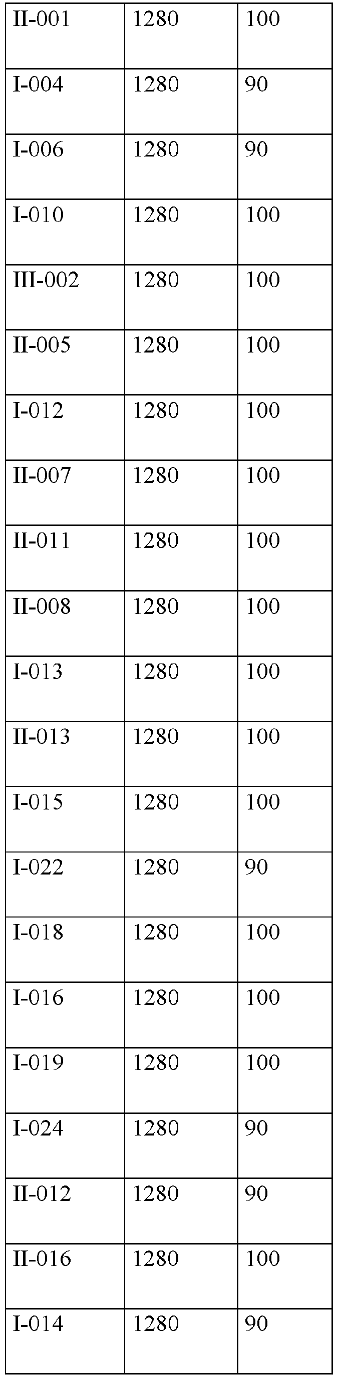

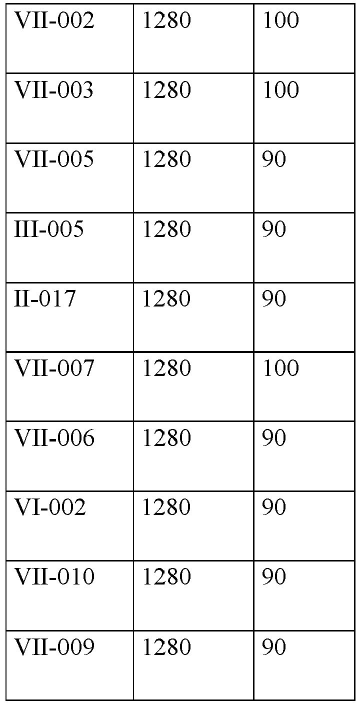

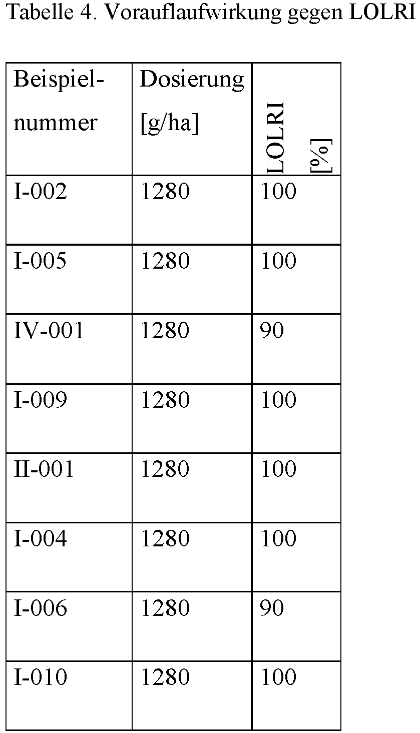

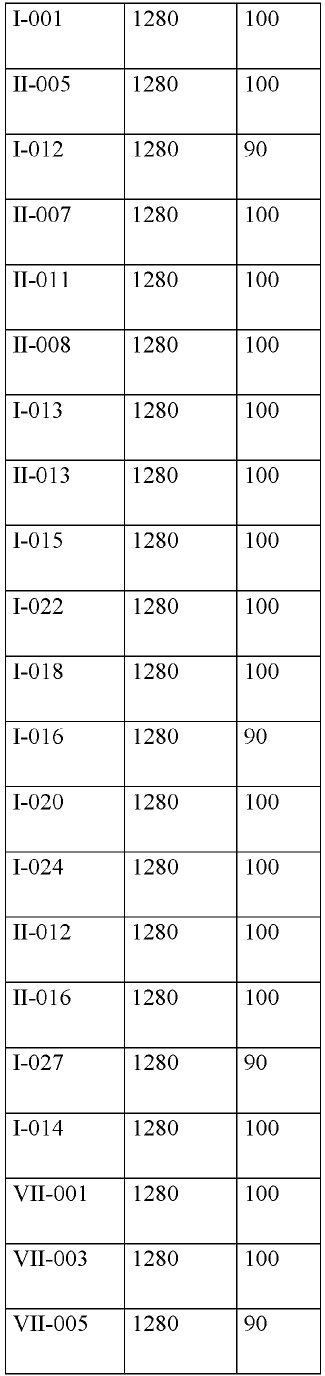

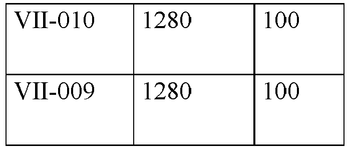

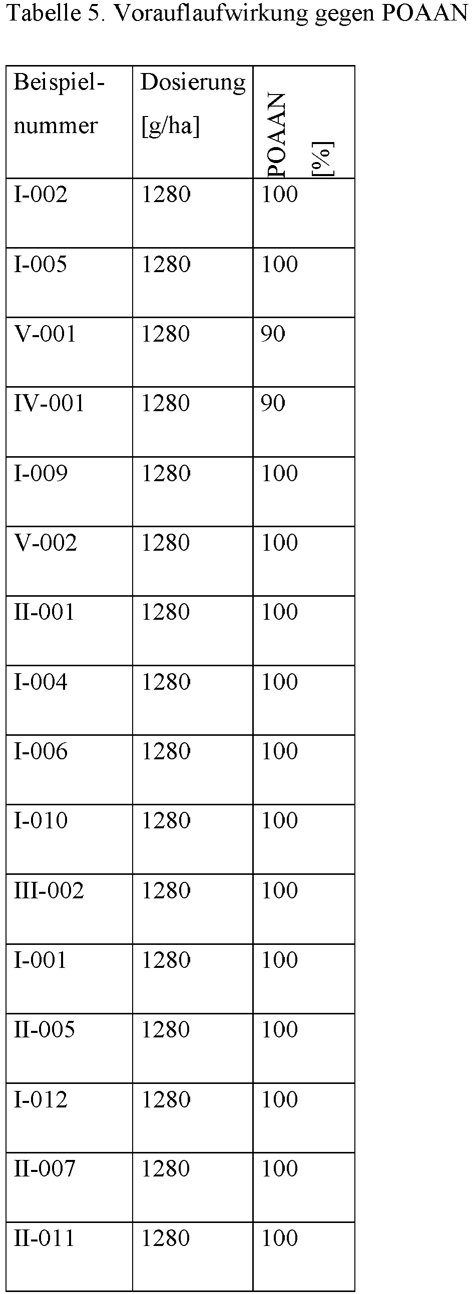

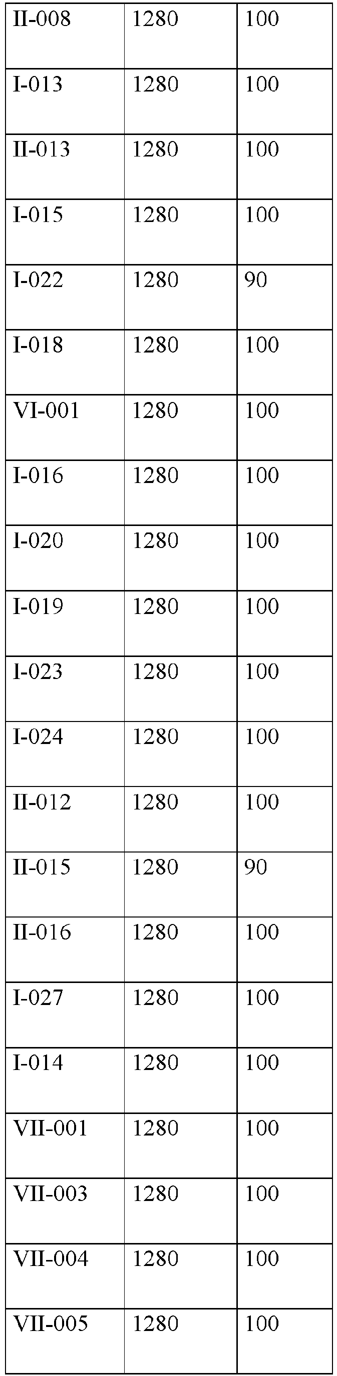

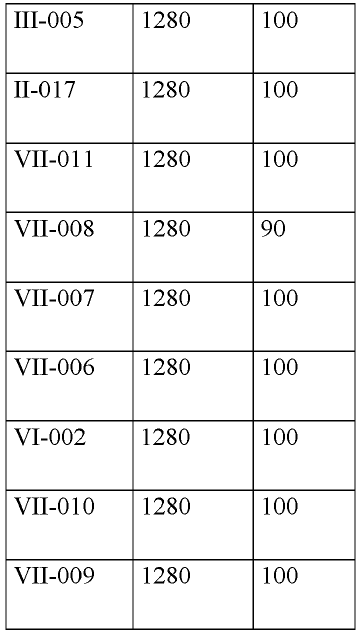

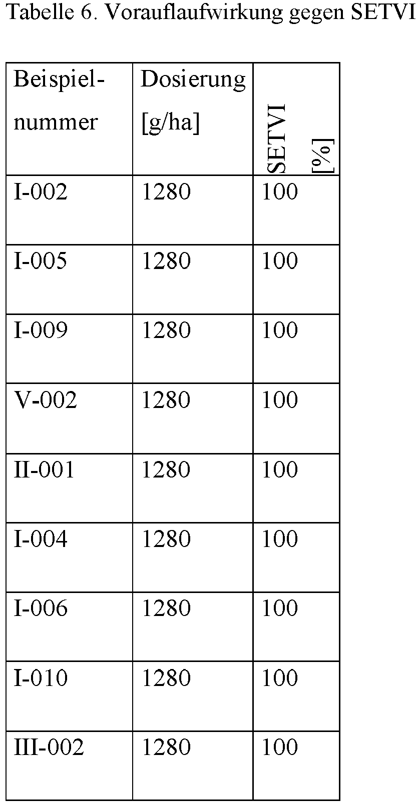

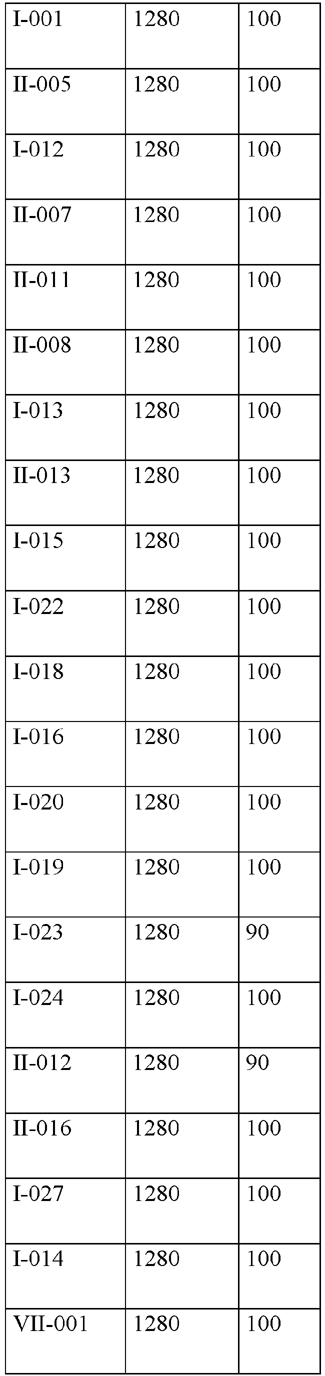

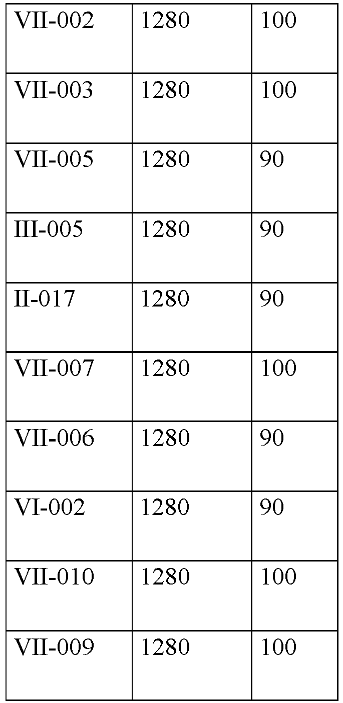

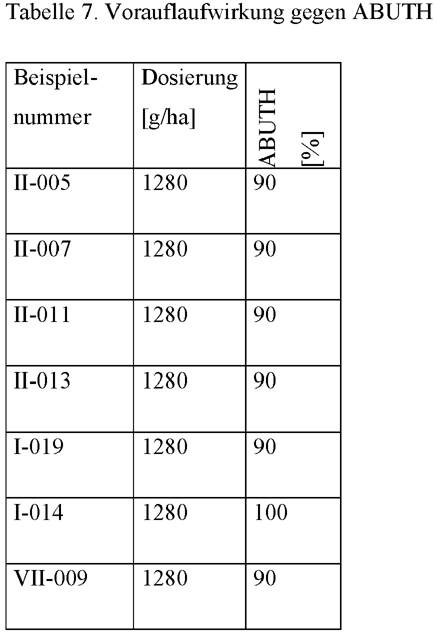

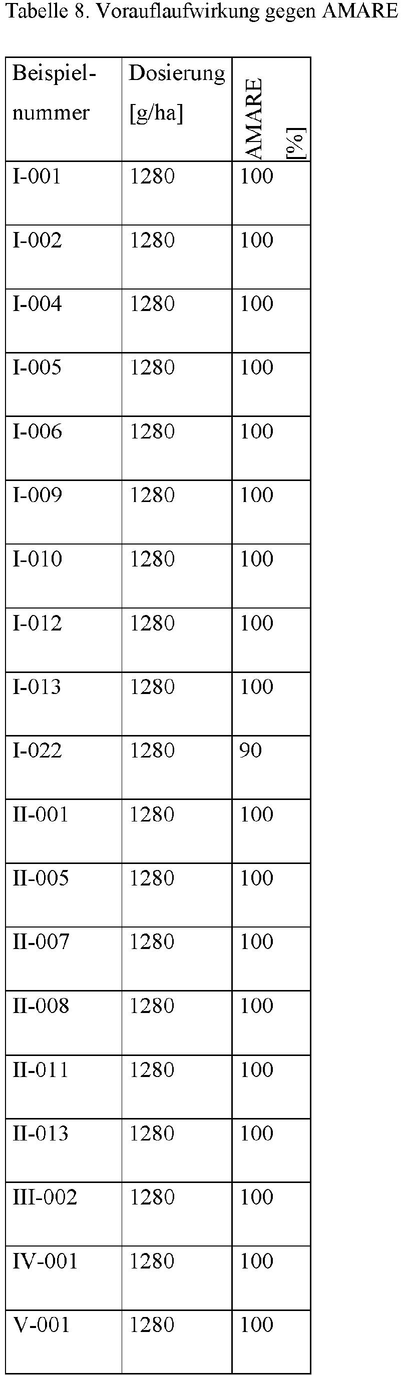

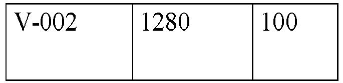

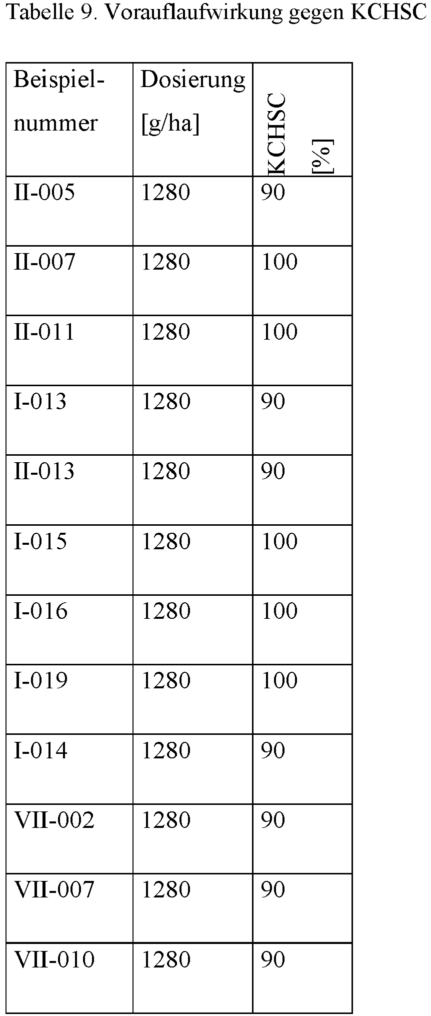

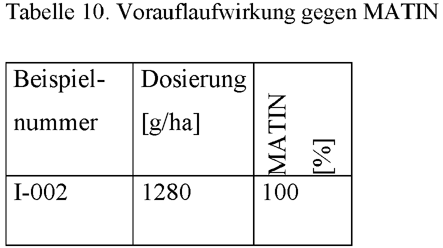

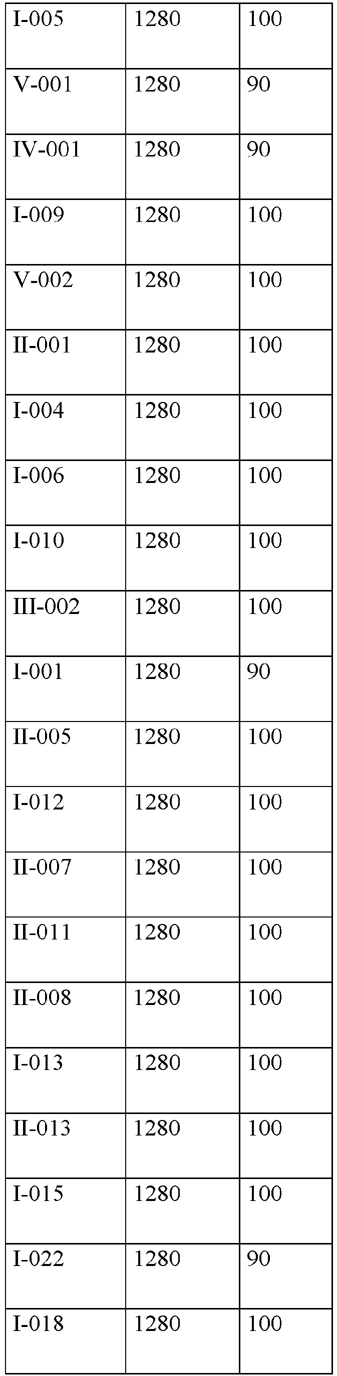

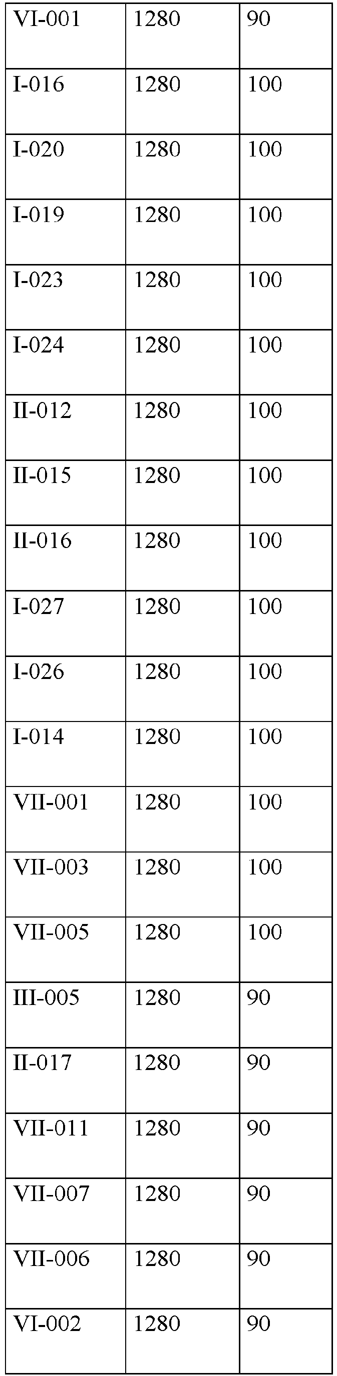

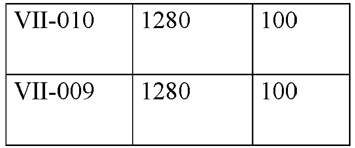

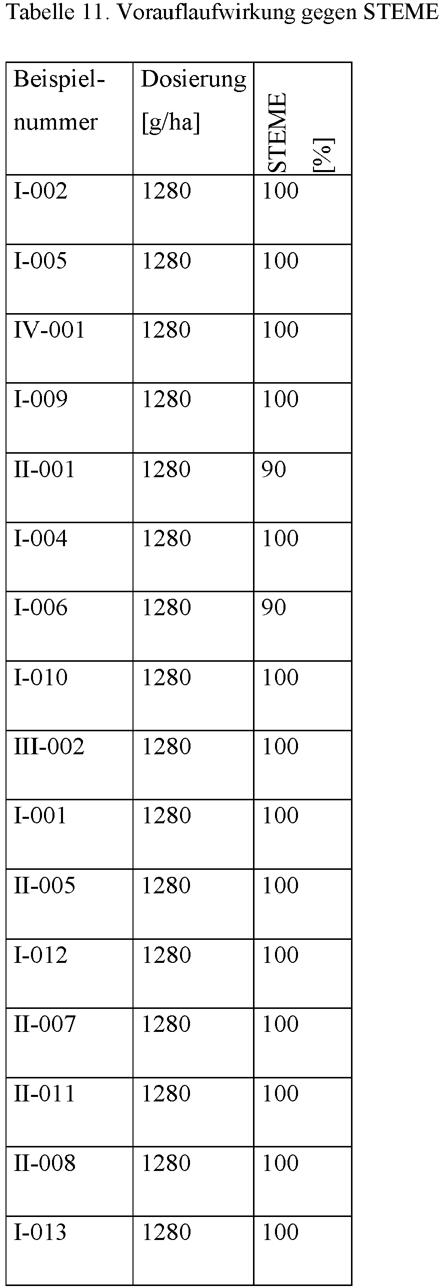

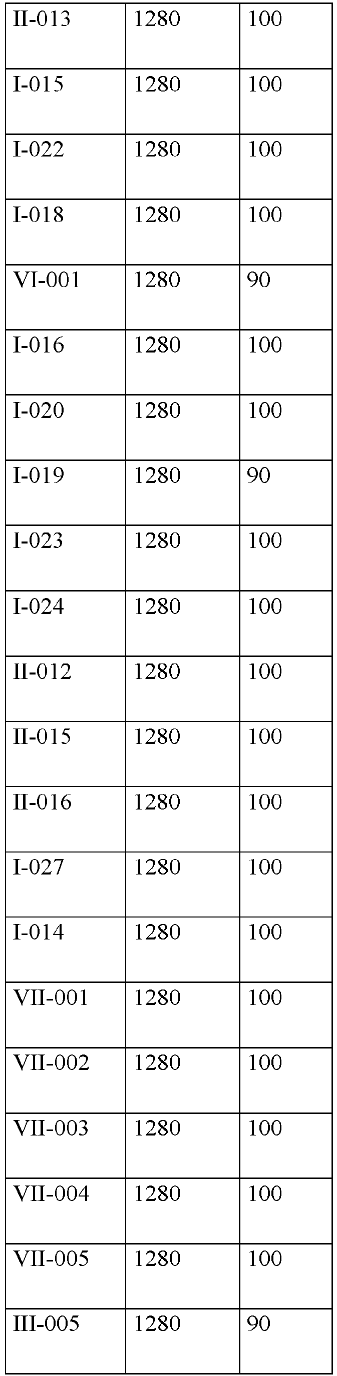

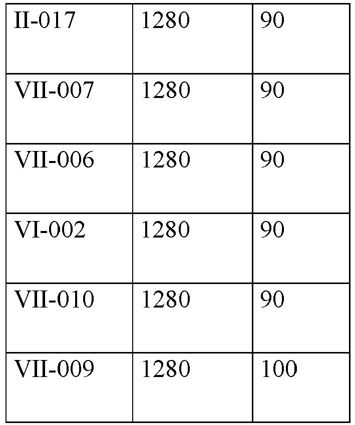

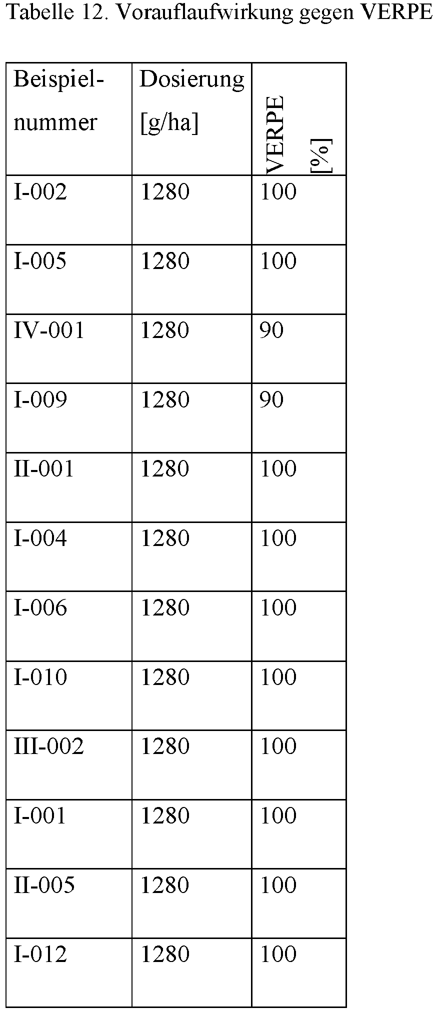

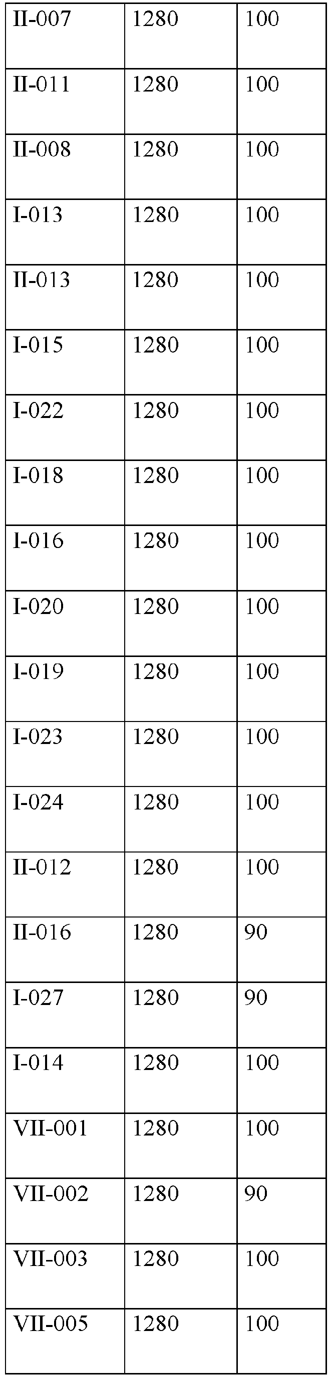

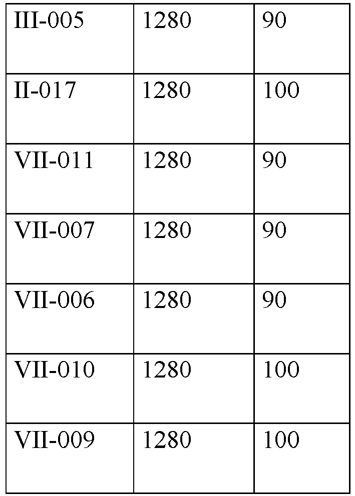

Beispielsweise zeigen die Verbindungen Nr. II-005, II-007, II-011 und II-013 in den Tabellen 1 bis 12 bei einer Aufwandmenge von 1280 g/ha jeweils eine 90-100%-ige Wirkung gegen *Alopecurus myrosoroides*, *Digitaria sanguinalis*, *Echinochloa crus-galli*, *Lolium rigidu* und *Setaria viridis.*

Die erfindungsgemäßen Verbindungen eignen sich deshalb im Vorauflaufverfahren zur Bekämpfung von unerwünschtem Pflanzenwuchs.

### 2. Herbizide Wirkung bzw. Kulturpflanzenverträglichkeit im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Wie die Ergebnisse aus der Tabelle 13 bis 24 zeigen, weisen erfindungsgemäße Verbindungen eine gute herbizide Nachauflaufwirksamkeit gegen ein breites Spektrum von Ungräsern und Unkräutern auf.

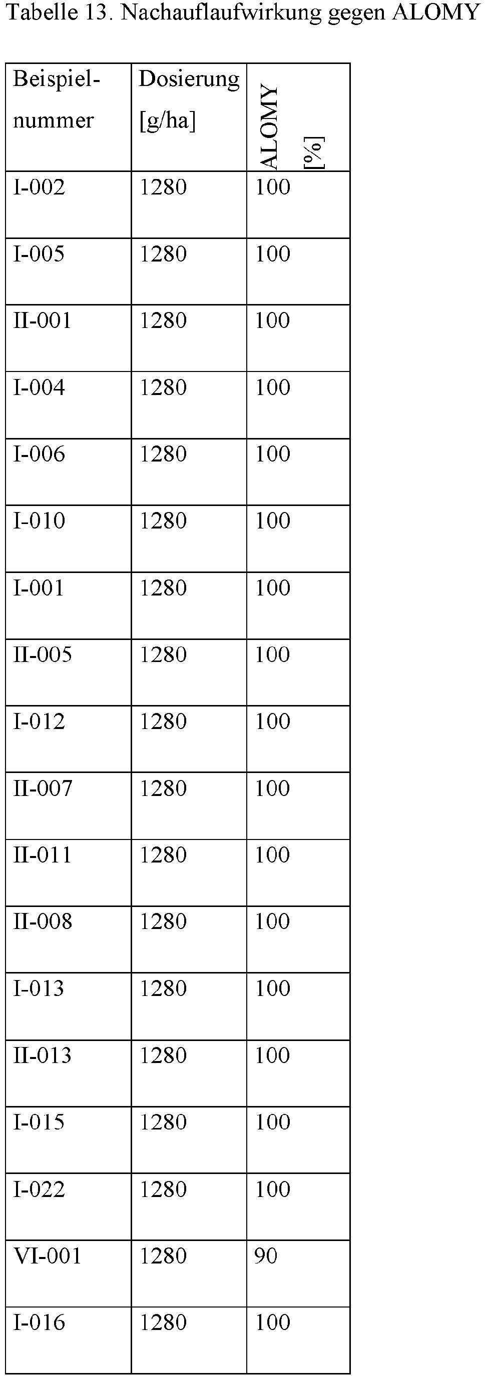

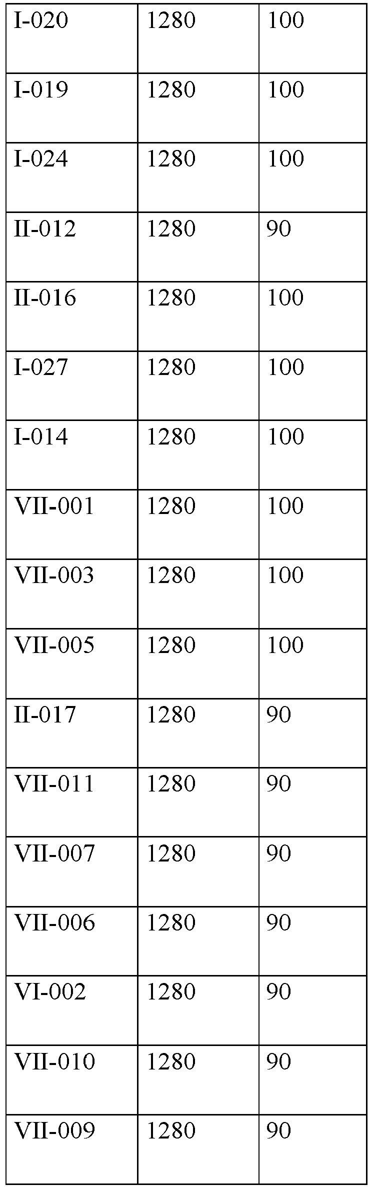

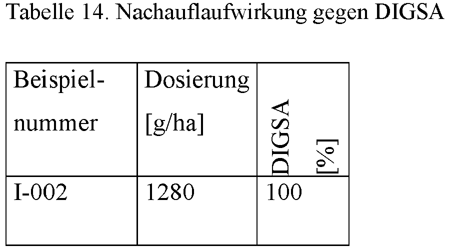

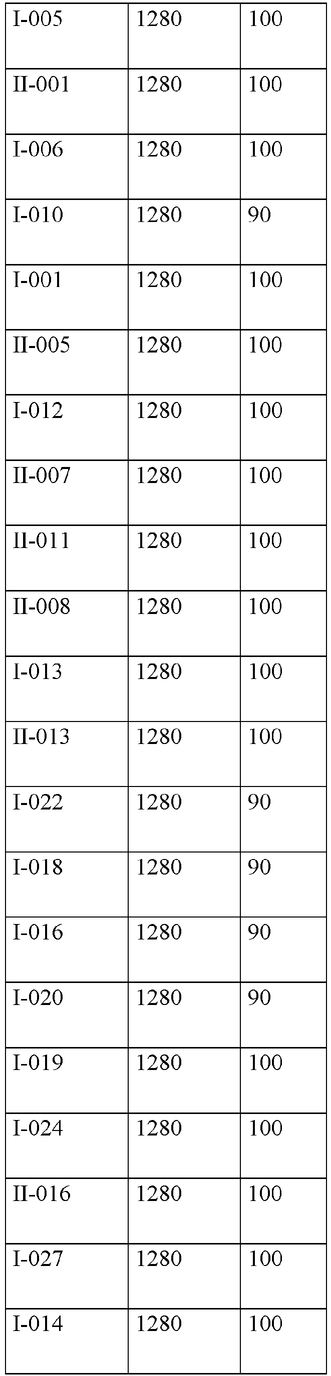

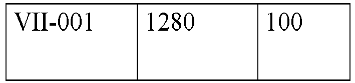

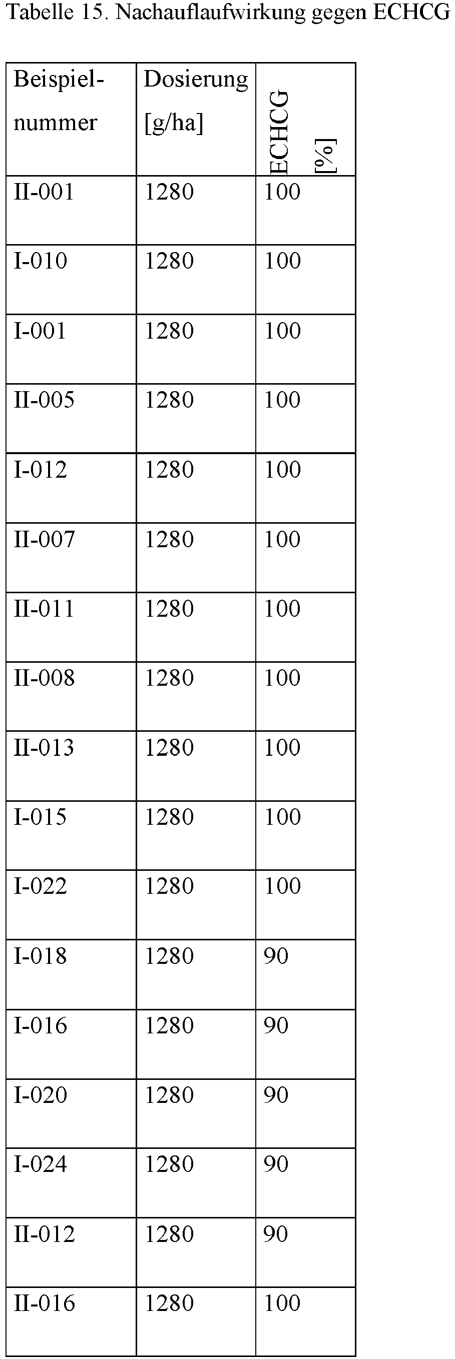

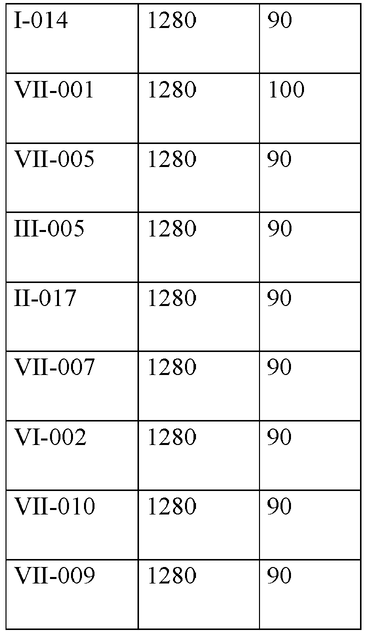

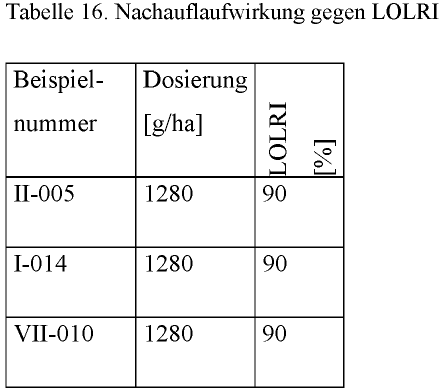

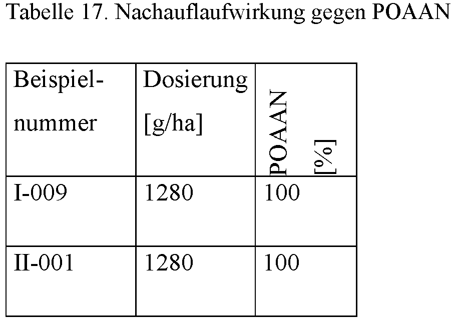

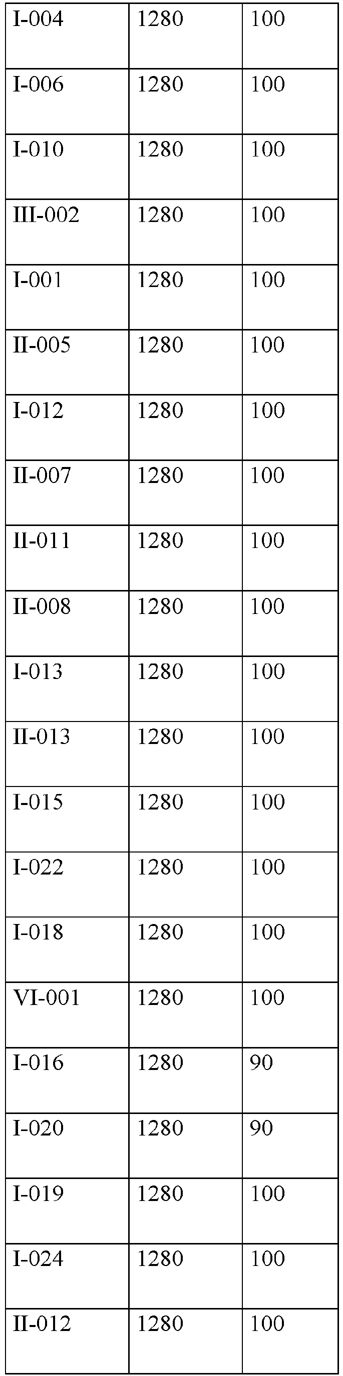

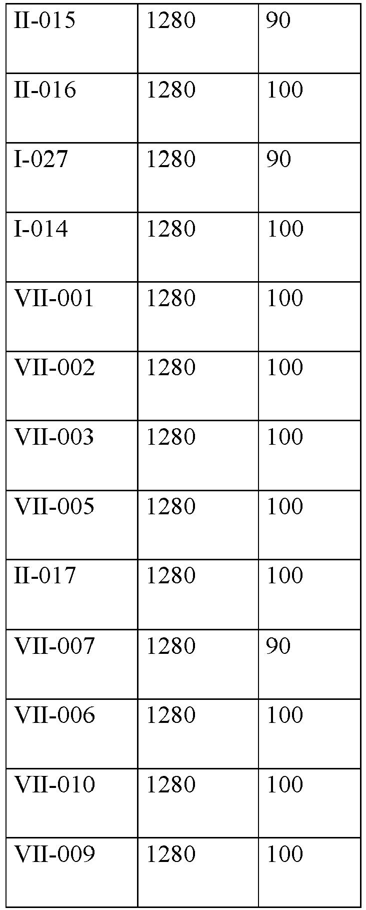

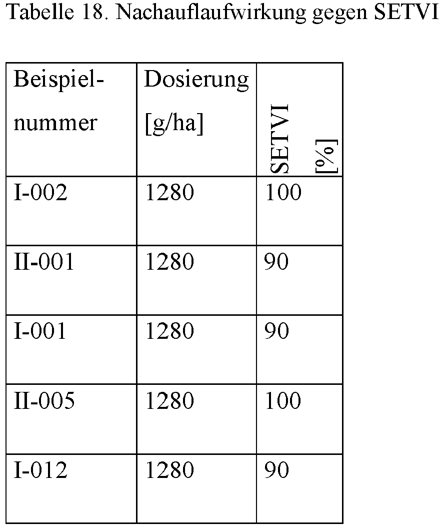

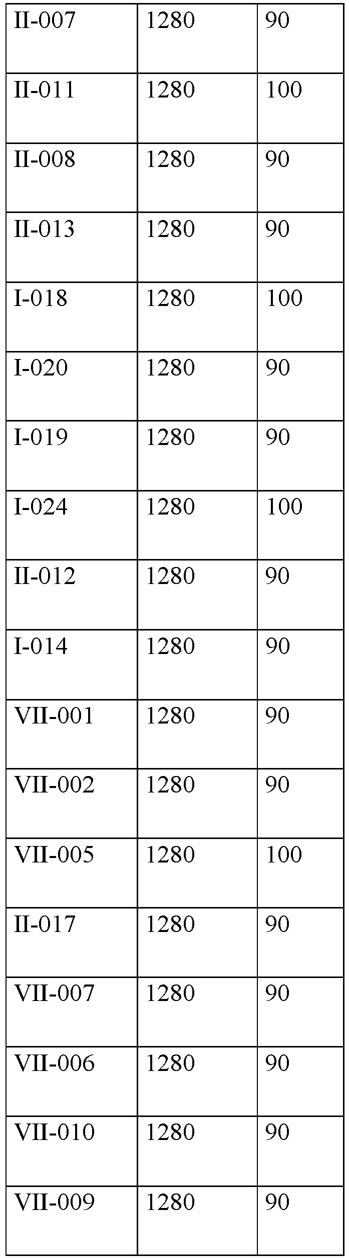

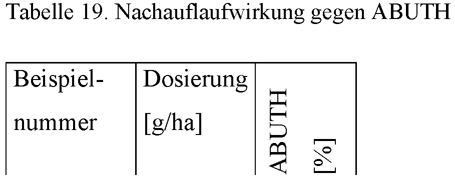

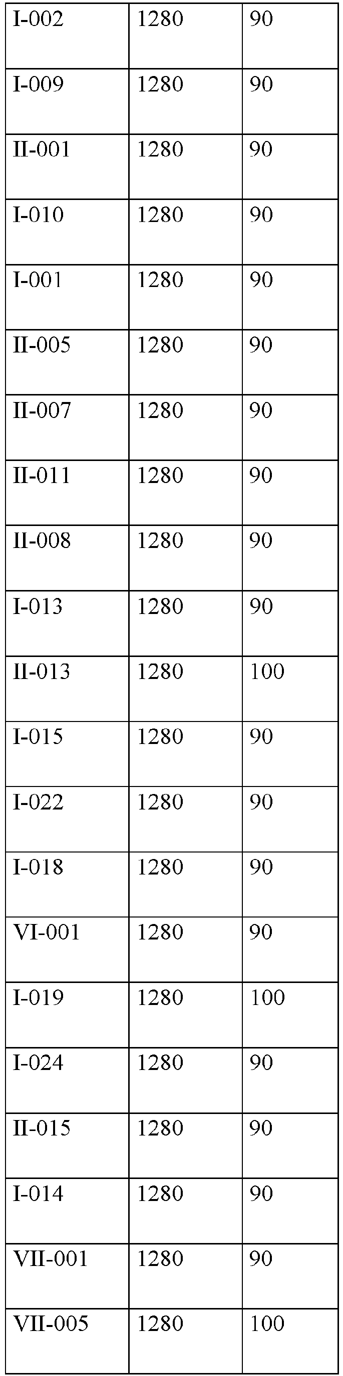

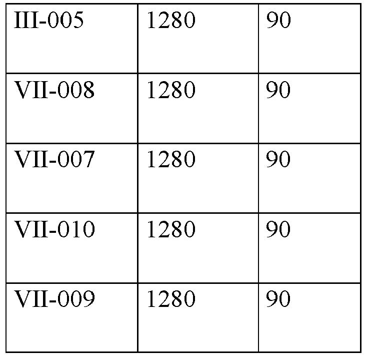

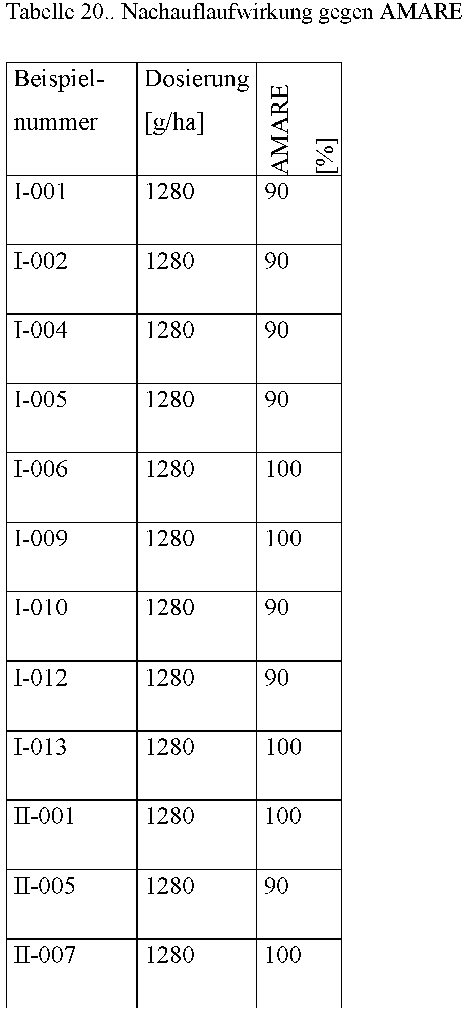

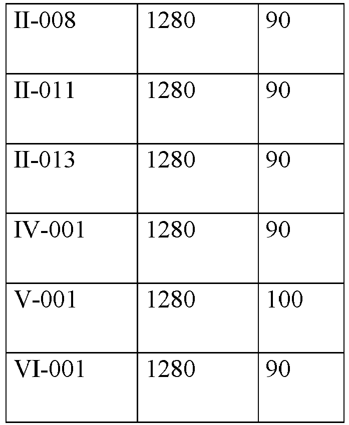

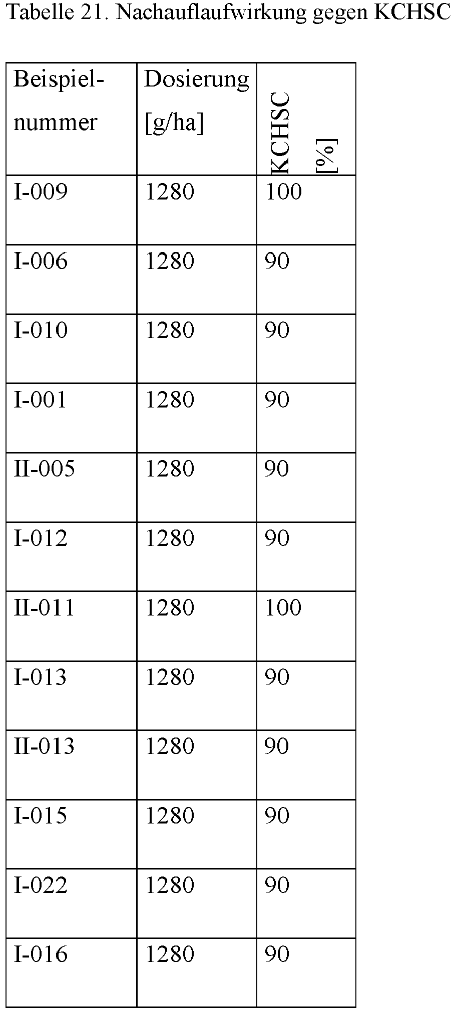

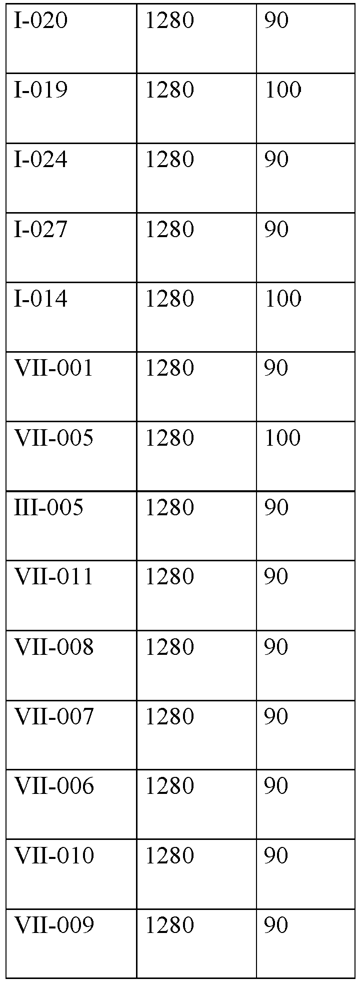

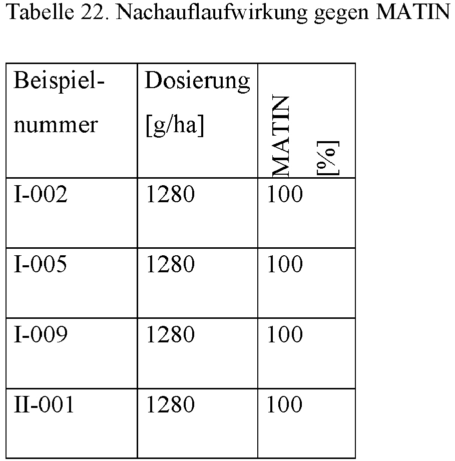

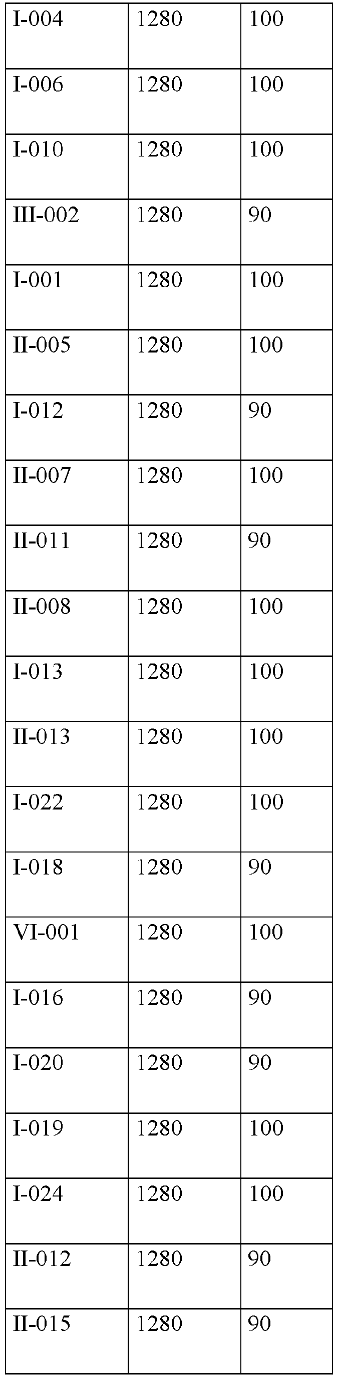

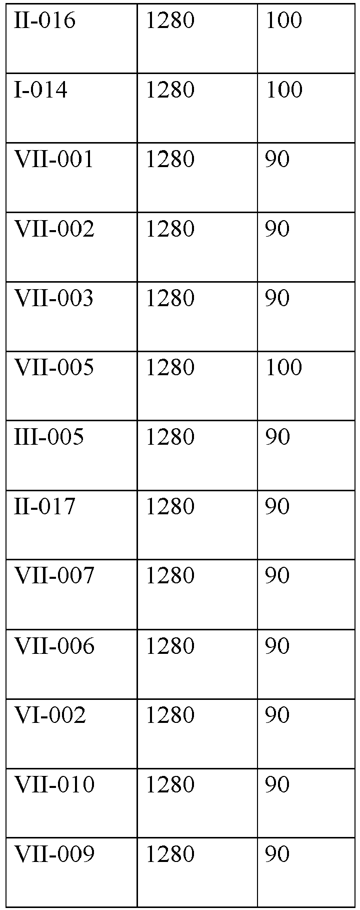

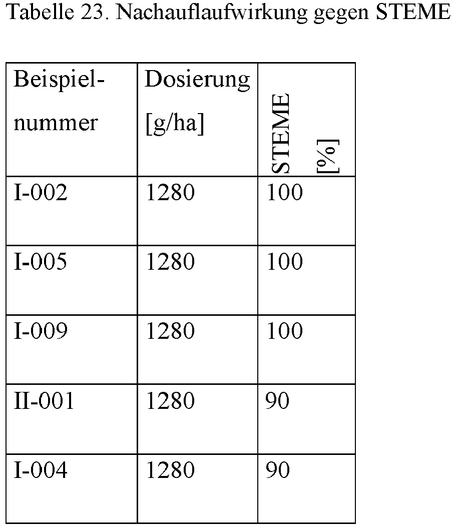

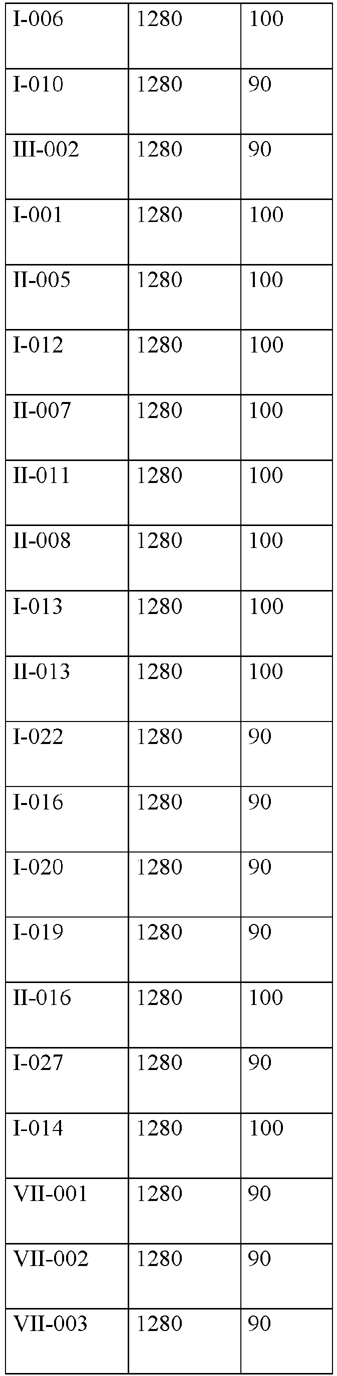

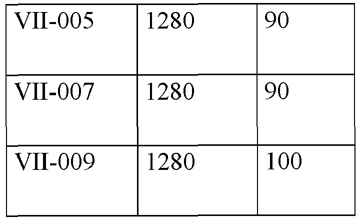

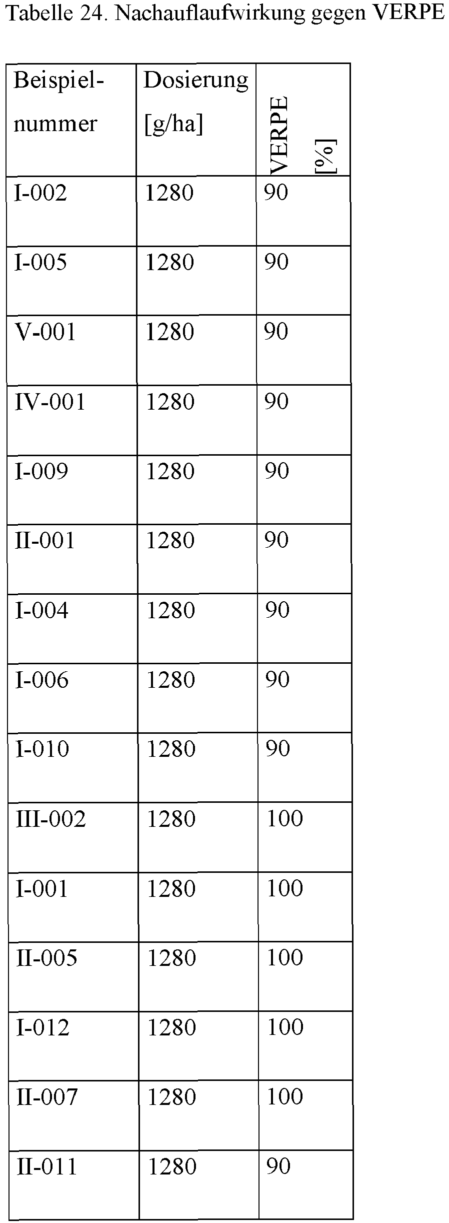

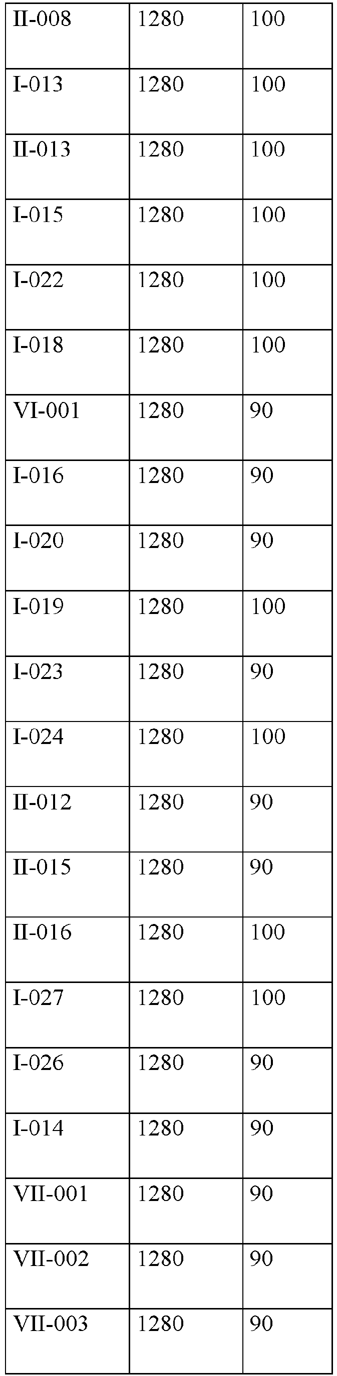

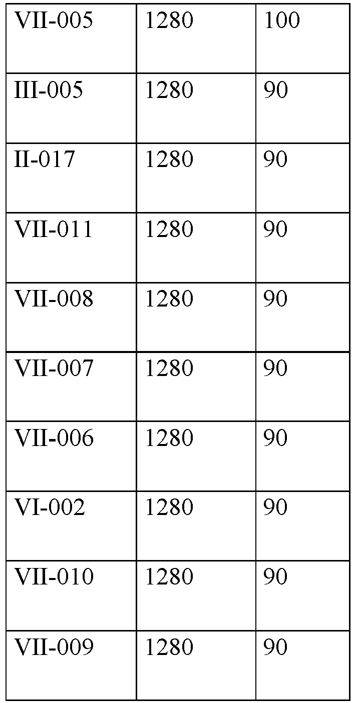

Beispielsweise zeigen alle erfindungsgemäßen Verbindungen II-001, II-005, II-007 und II-013 in den Tabellen 13 und 24 bei einer Aufwandmenge von 1280 g/ha jeweils eine 90 - 100%-ige Wirkung gegen *Alopecurus myosuroides*, *Digitaria sanguinalis*, *Setaria viridis* und *Echinochloa crus-galli* und
eignen sich deshalb im Nachauflaufverfahren zur Bekämpfung von unerwünschtem Pflanzenwuchs.

## Patentansprüche

1. [(1,4,5-Trisubstituierte-1H-pyrazol-3-yl)sulfanyl]essigsäure Derivate der allgemeinen Formel (I) oder ein agrochemisch akzeptables Salz davon, 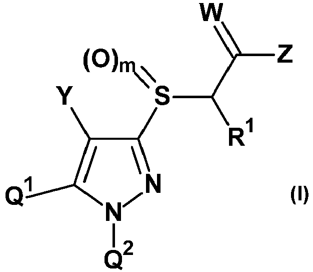 worin
Q¹ für Phenyl und Hetaryl steht,
wobei das Phenyl und das Hetaryl unsubstituiert oder jeweils unabhängig voneinander substituiert sind durch m Reste ausgewählt aus der Gruppe bestehend aus Wasserstoff, Halogen, Cyano, Isocyano, Nitro, Hydroxy, (C₁-C₆)-Alkyl, (C₁-C₆)-Haloalkyl, (C₃-C₆)-Cycloalkyl, (C₃-C₆)-Halocycloalkyl, (C₁-C₆)-Halogenalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, (C₁-C₄)-Halolkyl- S(O)ₙ, CHO, (C₁-C₄)-Alkyloxycarbonyl und NH₂ (Amino),
Q² für Phenyl steht,
welches unsubstituiert oder jeweils unabhängig voneinander substituiert ist durch m Reste ausgewählt aus der Gruppe bestehend aus Wasserstoff, Halogen, Cyano, Isocyano, NO₂, (C₁-C₆)-Alkyl, (C₁-C₆)-Haloalkyl, (C₁-C₆)-Haloalkoxy, (C₃-C₆)-Cycloalkyl, (C₃-C₆)-Halocycloalkyl, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, (C₁-C₄)-Haloalkyl- S(O)ₙ, CHO, (C₁-C₄)-Alkyloxycarbonyl und NH₂ (Amino),
Z für die Gruppen 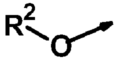 und 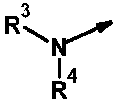 steht,
Y für - Halogen, Cyano, Isocyano, NO₂, NH₂ (Amino), (C₁-C₆)-Alkyl, (C₁-C₆)-Haloalkyl, (C₁-C₆)-Cyanoalkyl, (C₁-C₆)-Hydroxyalkyl, (C₁-C₆)-Alkoxy-(C₁-C₆)-alkyl, (C₃-C₆)-Cycloalkyl, (C₁-C₄)-Alkyloxycarbonyl , (C₁-C₆)-Alkylcarbonyl, CHO, (C₃-C₆)-Halocycloalkyl, (C₁-C₆)-Alkoxy, (C₁-C₆)-Haloalkoxy, (C₁-C₄)-Alkyl-S(O)ₙ, (C₁-C₄)-Haloalkyl- S(O)ₙ, (C₂-C₆)-Alkinyl, (C₂-C₆)-Haloalkinyl, (C₂-C₆)-Alkenyl und (C₂-C₆)-Haloalkenyl steht,
W für Sauerstoff oder Schwefel steht,
R¹ für Wasserstoff, Cyano, (C₁-C₆)-Alkyl, (C₁-C₆)-Haloalkyl, (C₁-C₆)-Alkoxy, (C₁-C₆)-Alkoxy-(C₁-C₆)-alkyl, (C₃-C₆)-Cycloalkyl, (C₃-C₆)-Halocycloalkyl, (C₂-C₆)-Alkinyl, (C₂-C₆)-Haloalkinyl, (C₂-C₆)-Alkenyl und (C₂-C₆)-Haloalkenyl steht,
R² für Wasserstoff, (C₁-C₁₀)-Alkyl und (C₃-C₁₀)-Cycloalkyl steht,
wobei das Alkyl und Cycloalkyl unsubstituiert oder jeweils unabhängig voneinander substituiert sind durch m Reste ausgewählt aus der Gruppe bestehend aus Halogen, Cyano, Isocyano, Nitro, NH₂ (Amino), (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl, Heterocyclyl-(C₁-C₄)-alkyl Heteroaryl-(C₁-C₄)-alkyl und Aryl-(C₁-C₄)-alkyl, wobei das Aryl, Heterocyclyl und Heteroaryl unsubstituiert oder jeweils unabhängig voneinander substituiert ist durch m Reste ausgewählt aus der Gruppe bestehend aus Halogen, (C₁-C₆)-Alkyl,(C₁-C₆)-Haloalkyl,
R³ für Wasserstoff und (C₁-C₁₂)-Alkyl steht,
R⁴ für Wasserstoff, Cyano, Nitro, (C₁-C₁₂)-Alkyl, (C₁-C₁₀)-Haloalkyl, (C₂-C₁₀)-Alkenyl, (C₃-C₁₀)-Alkinyl, (C₁-C₁₀)-Alkoxy-(C₁-C₁₀)-alkyl, (C₁-C₁₀)-Haloalkoxy-(C₁-C₁₀)-alkyl, (C₁-C₄)-Alkyl-S(O)ₙ-(C₁-C₄)-alkyl, (C₁-C₄)-Haloalkyl-S(O)ₙ-(C₁-C₄)-Alkyl, (C₃-C₁₀)-Cycloalkyl, (C₃-C₁₀)-Halocycloalkyl, Hydroxy-(C₁-C₁₀)-alkylcarbonyl, Amino-(C₁-C₁₀)-alkyl, (C₁-C₁₀)-Alkoxycarbonyl-(C₁-C₁₀)-alkyl, (C₁-C₁₀)-Cyanoalkyl, S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸, Aryl, Heteroaryl und Heterocyclyl steht,
welche unsubstituiert oder jeweils unabhängig voneinander substituiert sind durch m Reste ausgewählt aus der Gruppe bestehend aus Wasserstoff, Halogen, Cyano, Nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
oder
R³ und R⁴ bilden mit dem N-Atom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring,
R⁵ für Wasserstoff, (C₁-C₈)-Alkyl, (C₃-C₆)-Cycloalkyl, (C₁-C₈)-Haloalkyl und Aryl steht,
R⁶ für Wasserstoff und R⁵ steht,
R⁷ für Wasserstoff, (C₁-C₆)-Alkyl, (C₃-C₆)-Cycloalkyl, (C₃-C₄)-Alkenyl, (C₃-C₄)-Alkinyl und (C₁-C₁₀)-alkylcarbonyl-(C₁-C₆)-alkyl steht,
R⁸ für Wasserstoff, (C₁-C₆)-Alkyl, (C₃-C₆)-Cycloalkyl, (C₃-C₄)-Alkenyl, und (C₃-C₄)-Alkinyl, steht,
m für 0, 1 oder 2 steht,
und
n für 0, 1 oder 2 steht,
wobei die Verbindungen Ethyl-{[4-nitro-1-phenyl-5-(1H-tetrazol-5-yl)-1H-pyrazol-3-yl]sulfanyl}acetat CAS [1360702-86-8 ] und Methyl-{[5-(5-methyl-1,3,4-oxadiazol-2-yl)-4-nitro-1-phenyl-1H-pyrazol-3-yl]sulfanyl} acetat CAS [1360690-60-3] ausgenommen sind.

2. Verbindungen der Formel (I) gemäß Anspruch 1 oder ein agrochemisch akzeptables Salz davon, worin
Q¹ für die Gruppen Q¹-1.1 bis Q¹-6.5
| | | | | |
|---|---|---|---|---|
| 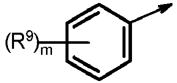 | 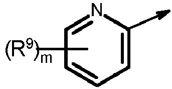 | 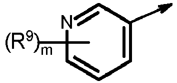 | 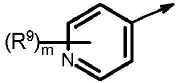 | 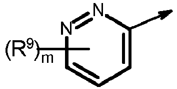 |
| Q¹-1.1 | Q¹-2.1 | Q¹-2.2 | Q¹-2.3 | Q¹-3.1 |
| 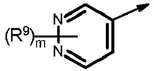 | 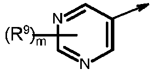 | 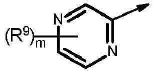 | 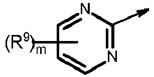 | 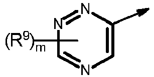 |
| Q¹-3.2 | Q¹-3.3 | Q¹-3.4 | Q¹-3.5 | Q¹-4.1 |
| 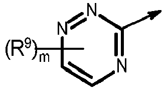 | 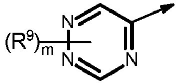 | 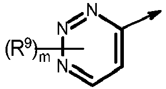 | 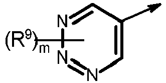 | 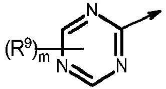 |
| Q¹-4.2 | Q¹-4.3 | Q¹-4.4 | Q¹-4.5 | Q¹-4.6 |
| 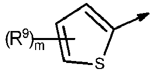 | 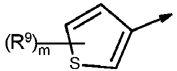 | 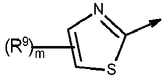 | 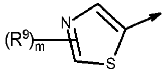 | 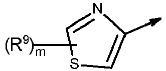 |
| Q¹-5.1 | Q¹-5.2 | Q¹-5.3 | Q¹-5.4 | Q¹-5.5 |
| 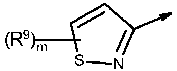 | 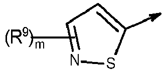 | 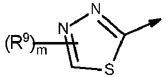 | 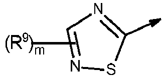 | 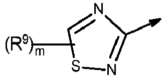 |
| Q¹-5.6 | Q¹-5.7 | Q¹-6.1 | Q¹-62 | Q¹-6.3 |
| 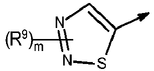 | 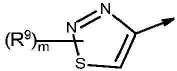 | | | |
| Q¹-6.4 | Q¹-6.5 | | | |
steht,
Q² für Phenyl steht,
welches unsubstituiert oder jeweils unabhängig voneinander substituiert ist durch m Reste ausgewählt aus der Gruppe bestehend aus Wasserstoff, Halogen, Cyano, Isocyano, NO₂, (C₁-C₆)-Alkyl, (C₁-C₆)-Haloalkyl, (C₁-C₆)-Haloalkoxy, (C₃-C₆)-Cycloalkyl, (C₃-C₆)-Halocycloalkyl, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, (C₁-C₄)-Haloalkyl- S(O)ₙ, CHO, (C₁-C₄)-Alkyloxycarbonyl und NH₂ (Amino),
Z für die Gruppen 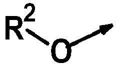 und 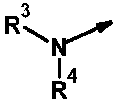 steht,
Y für - Halogen, Cyano, Isocyano, NO₂, NH₂ (Amino), (C₁-C₆)-Alkyl, (C₁-C₆)-Haloalkyl, (C₁-C₆)-Cyanoalkyl, (C₁-C₆)-Hydroxyalkyl, (C₁-C₆)-Alkoxy-(C₁-C₆)-alkyl, (C₃-C₆)-Cycloalkyl, (C₁-C₄)-Alkyloxycarbonyl , (C₁-C₆)-Alkylcarbonyl, CHO, (C₃-C₆)-Halocycloalkyl, (C₁-C₆)-Alkoxy, (C₁-C₆)-Haloalkoxy, (C₁-C₄)-Alkyl- S(O)ₙ, (C₁-C₄)-Haloalkyl- S(O)ₙ, (C₂-C₆)-Alkinyl, (C₂-C₆)-Haloalkinyl, (C₂-C₆)-Alkenyl und (C₂-C₆)-Haloalkenyl steht,
W für Sauerstoff oder Schwefel steht,
R¹ für Wasserstoff, Cyano, (C₁-C₆)-Alkyl, (C₁-C₆)-Haloalkyl, (C₁-C₆)-Alkoxy, (C₁-C₆)-Alkoxy-(C₁-C₆)-alkyl, (C₃-C₆)-Cycloalkyl, (C₃-C₆)-Halocycloalkyl, (C₂-C₆)-Alkinyl, (C₂-C₆)-Haloalkinyl, (C₂-C₆)-Alkenyl und (C₂-C₆)-Haloalkenyl steht,
R² für Wasserstoff ,(C₁-C₁₀)-Alkyl und (C₃-C₁₀)-Cycloalkyl steht,
wobei das Alkyl und Cycloalkyl unsubstituiert oder jeweils unabhängig voneinander substituiert ist durch m Reste ausgewählt aus der Gruppe bestehend aus Halogen, Cyano, Isocyano, Nitro, NH₂ (Amino), (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl, Heterocyclyl-(C₁-C₆)-alkyl Heteroaryl-(C₁-C₄)-alkyl und Aryl-(C₁-C₄)-alkyl, wobei das Aryl, Heterocyclyl und Heteroaryl unsubstituiert oder jeweils unabhängig voneinander substituiert ist durch m Reste ausgewählt aus der Gruppe bestehend aus Halogen, (C₁-C₆)-Alkyl und (C₁-C₆)-Haloalkyl,
R³ für Wasserstoff und (C₁-C₁₀)-Alkyl, steht,
R⁴ für Wasserstoff, Cyano, Nitro, (C₁-C₁₀)-Alkyl, (C₁-C₁₀)-Haloalkyl, (C₂-C₁₀)-Alkenyl, (C₃-C₁₀)-Alkinyl, (C₁-C₁₀)-Alkoxy-(C₁-C₁₀)-alkyl, (C₁-C₁₀)-Haloalkoxy-(C₁-C₁₀)-alkyl, (C₁-C₄)-Alkyl-S(O)ₙ(C₁-C₄)-alkyl, (C₁-C₄)-Haloalkyl-S(O)ₙ-(C₁-C₄)-Alkyl, (C₃-C₁₀)-Cycloalkyl, (C₃-C₁₀)-Halocycloalkyl, Hydroxy-(C₁-C₁₀)-alkylcarbonyl, Amino-(C₁-C₁₀)-alkyl, (C₁-C₁₀)-Alkoxycarbonyl-(C₁-C₁₀)-alkyl, (C₁-C₁₀)-Cyanoalkyl, S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸; Aryl, Heteroaryl und Heterocyclyl, steht,
welche unsubstituiert sind oder jeweils unabhängig voneinander substituiert sind durch m Reste ausgewählt aus der Gruppe bestehend aus Wasserstoff, Halogen, Cyano, Nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
oder R³ und R⁴ bilden mit dem N-Atom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring,
R⁵ für Wasserstoff, (C₁-C₈)-Alkyl, (C₃-C₆)-Cycloalkyl, (C₁-C₈)-Haloalkyl und Aryl steht,
R⁶ für Wasserstoff und R⁵ steht,
R⁷ für Wasserstoff, (C₁-C₆)-Alkyl, (C₃-C₆)-Cycloalkyl, (C₃-C₄)-Alkenyl, (C₃-C₄)-Alkinyl, und (C₁-C₆)-alkylcarbonyl-(C₁-C₄)-alkyl steht,
R⁸ für Wasserstoff, (C₁-C₆)-Alkyl, (C₃-C₆)-Cycloalkyl, (C₃-C₄)-Alkenyl und (C₃-C₄)-Alkinyl steht,
R⁹ für Wasserstoff, Halogen, Cyano, Isocyano, Nitro, Hydroxy, (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl-S(O)ₙ, CHO, (C₁-C₄)-Alkyloxycarbonyl und NH₂ (Amino) steht,
m für 0, 1 und 2, steht,
n für 0, 1 und 2, steht.

3. Verbindungen der Formel (I) gemäß Anspruch 1 oder 2 oder ein agrochemisch akzeptables Salz davon, worin
Q¹ für die Gruppen Q¹-1.1 bis Q¹-6.3 steht,
| | | | | |
|---|---|---|---|---|
| 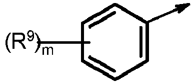 | 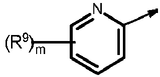 | 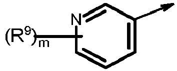 | 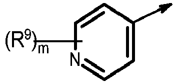 | 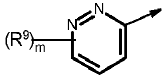 |
| Q¹-1.1 | Q¹-2.1 | Q¹-2.2 | Q¹-2.3 | Q¹-3.1 |
| 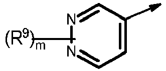 | 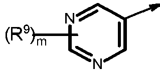 | 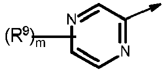 | 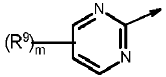 | 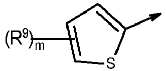 |
| Q¹-3.2 | Q¹-3.3 | Q¹-3.4 | Q¹-3.5 | Q¹-5.1 |
| 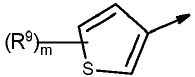 | 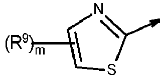 | 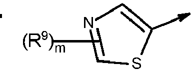 | 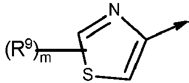 | 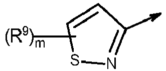 |
| Q¹-5.2 | Q¹-5.3 | Q¹-5.4 | Q¹-5.5 | Q¹-5.6 |
| 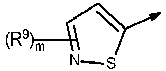 | 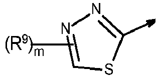 | 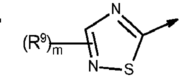 | 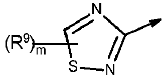 | |
| Q¹-5.7 | Q¹-6.1 | Q¹-6.2 | Q¹-6.3 | |
Q² für Phenyl steht,
welches unsubstituiert oder jeweils unabhängig voneinander substituiert ist durch m Reste ausgewählt aus der Gruppe bestehend aus Wasserstoff, Halogen, Cyano, (C₁-C₆)-Alkyl, (C₁-C₆)-Haloalkyl, (C₁-C₆)-Haloalkoxy, (C₃-C₆)-Cycloalkyl, (C₁-C₆)-Alkoxy, (C₁-C₄)-Alkyl-S(O)ₙ und (C₁-C₄)-Haloalkyl-S(O)ₙ,
Z für die Gruppen 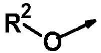 und 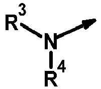 steht,
Y für Halogen, Cyano, Isocyano, NO₂, (C₁-C₆)-Alkyl, (C₁-C₆)-Haloalkyl, (C₁-C₆)-Alkoxy-(C₁-C₆)-alkyl, (C₁-C₄)-Alkyloxycarbonyl , (C₁-C₆)-Alkylcarbonyl, (C₁-C₆)-Alkoxy, (C₁-C₆)-Haloalkoxy, (C₁-C₄)-Alkyl- S(O)ₙ, (C₁-C₄)-Haloalkyl-S(O)ₙ, (C₂-C₆)-Alkinyl, (C₂-C₆)-Haloalkinyl, (C₂-C₆)-Alkenyl und (C₂-C₆)-Haloalkenyl steht,
W für Sauerstoff steht,
R¹ für Wasserstoff, Cyano, (C₁-C₆)-Alkyl, (C₁-C₆)-Haloalkyl, (C₁-C₆)-Alkoxy, (C₁-C₄)-Alkoxy-(C₁-C₄)-alkyl, (C₃-C₆)-Cycloalkyl, (C₂-C₆)-Alkinyl, (C₂-C₆)-Haloalkinyl, (C₂-C₆)-Alkenyl und (C₂-C₆)-Haloalkenyl steht,
R² für Wasserstoff, (C₁-C₆)-Alkyl und (C₃-C₆)-Cycloalkyl steht,
wobei das Alkyl und Cycloalkyl unsubstituiert oder jeweils unabhängig voneinander substituiert ist durch m Reste ausgewählt aus der Gruppe bestehend aus Halogen, Cyano, Nitro, NH₂ (Amino), (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl, Heterocyclyl-(C₁-C₄)-alkyl Heteroaryl-(C₁-C₄)-alkyl und Aryl-(C₁-C₄)-alkyl, wobei das Aryl, Heterocyclyl und Heteroaryl unsubstituiert oder jeweils unabhängig voneinander substituiert ist durch m Reste ausgewählt aus der Gruppe bestehend aus Halogen, (C₁-C₆)-Alkyl, (C₁-C₆)-Haloalkyl,
R³ für Wasserstoff, und (C₁-C₆)-Alkyl, steht,
R⁴ für Wasserstoff, Cyano, Nitro, (C₁-C₆)-Alkyl, (C₁-C₆)-Haloalkyl, (C₂-C₆)-Alkenyl, (C₃-C₆)-Alkinyl, (C₁-C₆)-Alkoxy-(C₁-C₆)-alkyl, (C₁-C₆)-Haloalkoxy-(C₁-C₆)-alkyl, (C₁-C₄)-Alkyl-S(O)ₙ-(C₁-C₄)-alkyl und (C₁-C₄)-Haloalkyl-S(O)ₙ-(C₁-C₄)-Alkyl, (C₃-C₆)-Cycloalkyl, (C₃-C₆)-Halocycloalkyl, Hydroxy-(C₁-C₆)-alkylcarbonyl, Amino-(C₁-C₆)-alkyl, (C₁-C₆)-Alkoxycarbonyl-(C₁-C₆)-alkyl, S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸; Aryl, Heteroaryl und Heterocyclyl steht,
welche unsubstituiert sind oder jeweils unabhängig voneinander substituiert sind durch m Reste ausgewählt aus der Gruppe bestehend aus Wasserstoff, Halogen, Cyano, Nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
oder R³ und R⁴ bilden mit dem N-Atom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring,
R⁵ für Wasserstoff, (C₁-C₈)-Alkyl, (C₃-C₆)-Cycloalkyl, (C₁-C₈)-Haloalkyl und Aryl steht,
R⁶ für Wasserstoff und R⁵ steht,
R⁷ für Wasserstoff, (C₁-C₆)-Alkyl, (C₃-C₆)-Cycloalkyl, (C₃-C₄)-Alkenyl, und (C₃-C₄)-Alkinyl, steht,
R⁸ für Wasserstoff, (C₁-C₆)-Alkyl, (C₃-C₆)-Cycloalkyl, (C₃-C₄)-Alkenyl, (C₃-C₄)-Alkinyl, und (C₁-C₁₀)-alkylcarbonyl-(C₁-C₆)-alkyl, steht,
R⁹ für Wasserstoff, Halogen, Cyano, Isocyano, Nitro, Hydroxy, (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl und NH₂ (Amino) steht,
m für 0, 1 und 2 steht,
n für 0, 1 und 2 steht.

4. Verbindungen der Formel (I) gemäß einem der Ansprüche 1 bis 3 oder ein agrochemisch akzeptables Salz davon, worin
Q¹ für die Gruppen Q¹-1.1 bis Q¹-6.3 steht,
| | | | | |
|---|---|---|---|---|
| 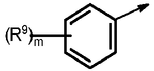 | 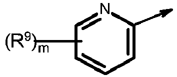 | 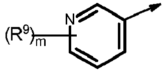 | 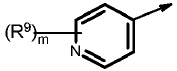 | 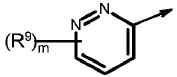 |
| Q¹-1.1 | Q¹-2.1 | Q¹-2.2 | Q¹-2.3 | Q¹-3.1 |
| 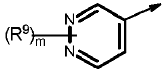 | 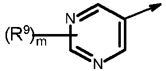 | 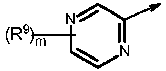 | 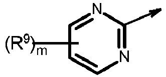 | 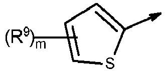 |
| Q¹-3.2 | Q¹-3.3 | Q¹-3.4 | Q¹-3.5 | Q¹-5.1 |
| 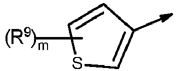 | 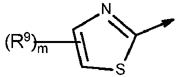 | 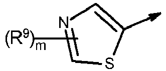 | 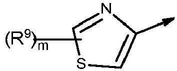 | 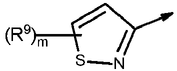 |
| Q¹-5.2 | Q¹-5.3 | Q¹-5.4 | Q¹-5.5 | Q¹-5.6 |
| 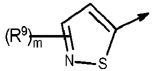 | 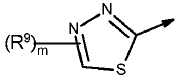 | 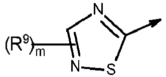 | 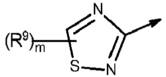 | |
| Q¹-5.7 | Q¹-6.1 | Q¹-6.2 | Q¹-6.3 | |
Q² für Phenyl steht,
welcher unsubstituiert oder jeweils unabhängig voneinander substituiert ist durch m Reste ausgewählt aus der Gruppe bestehend aus Wasserstoff, Fluor, Chlor und Brom,
Z für die Gruppen 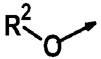 und 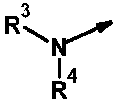 steht,
Y für Fluor, Chlor, Brom, Cyano, NO₂, (C₁-C₂)-Alkyl, (C₁-C₂)-Haloalkyl, (C₁-C₂)-Alkylcarbonyl, (C₁-C₂)-Alkoxy, (C₁-C₂)-Haloalkoxy, (C₁-C₂)-Alkyl- S(O)ₙ, und (C₁-C₂)-Haloalkyl- S(O)ₙ, steht,
W für Sauerstoff steht,
R¹ für Wasserstoff, (C₁-C₄)-Alkyl, (C₁-C₄)-Haloalkyl, und (C₁-C₆)-Alkoxy, steht,
R² für Wasserstoff, (C₁-C₆)-Alkyl und (C₃-C₆)-Cycloalkyl steht,
wobei das Alkyl und Cycloalkyl unsubstituiert oder jeweils unabhängig voneinander substituiert ist durch m Reste ausgewählt aus der Gruppe bestehend aus Halogen, Cyano, Nitro, NH₂ (Amino), (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl, Heterocyclyl-(C₁-C₄)-alkyl Heteroaryl-(C₁-C₄)-alkyl und Aryl-(C₁-C₄)-alkyl, wobei das Aryl, Heterocyclyl und Heteroaryl unsubstituiert oder jeweils unabhängig voneinander substituiert ist durch m Reste ausgewählt aus der Gruppe bestehend aus Halogen, (C₁-C₆)-Alkyl, (C₁-C₆)-Haloalkyl,
R³ für Wasserstoff, und (C₁-C₆)-Alkyl steht,
R⁴ für Wasserstoff, Cyano, Nitro, (C₁-C₆)-Alkyl, (C₁-C₆)-Haloalkyl, (C₂-C₆)-Alkenyl, (C₃-C₆)-Alkinyl, (C₁-C₆)-Alkoxy-(C₁-C₆)-alkyl, (C₁-C₆)-Haloalkoxy-(C₁-C₆)-alkyl, (C₁-C₄)-Alkyl-S(O)ₙ-(C₁-C₄)-alkyl und (C₁-C₄)-Haloalkyl-S(O)ₙ-(C₁-C₄)-Alkyl, (C₃-C₆)-Cycloalkyl, (C₃-C₆)-Halocycloalkyl, Hydroxy-(C₁-C₆)-alkylcarbonyl, Amino-(C₁-C₆)-alkyl, (C₁-C₆)-Alkoxycarbonyl-(C₁-C₆)-alkyl, S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸; Aryl, Heteroaryl und Heterocyclyl steht,
welche unsubstituiert oder jeweils unabhängig voneinander substituiert sind durch m Reste ausgewählt aus der Gruppe bestehend aus Wasserstoff, Halogen, Cyano, Nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
oder R³ und R⁴ bilden mit dem N-Atom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring,
R⁵ für Wasserstoff, (C₁-C₈)-Alkyl, (C₃-C₆)-Cycloalkyl, (C₁-C₈)-Haloalkyl und Aryl steht,
R⁶ für Wasserstoff und R⁵ steht,
R⁷ für Wasserstoff, (C₁-C₆)-Alkyl, (C₃-C₆)-Cycloalkyl, (C₃-C₄)-Alkenyl, (C₃-C₄)-Alkinyl und (C₁-C₁₀)-alkylcarbonyl-(C₁-C₆)-alkyl steht,
R⁸ für Wasserstoff, (C₁-C₆)-Alkyl, (C₃-C₆)-Cycloalkyl, (C₃-C₄)-Alkenyl und (C₃-C₄)-Alkinyl steht,
R⁹ für Wasserstoff, Halogen, Cyano, Isocyano, Nitro, Hydroxy, (C₁-C₆)-Alkyl, (C₁-C₆)-Haloalkyl, (C₁-C₆)-Haloalkoxy, (C₂-C₃)-Alkenyl, (C₂-C₃)-Haloalkenyl, (C₁-C₆)-Alkoxy, (C₂-C₃)-Alkinyl, (C₂-C₃)-Haloalkinyl, (C₁-C₄)-Alkyl- S(O)ₙ, CHO, (C₁-C₄)-Alkyloxycarbonyl und NH₂ (Amino) steht,
m für 0, 1 und 2 steht,
n für 0, 1 und 2 steht.

5. Verbindungen der Formel **(I)** gemäß einem der Ansprüche 1 bis 3 oder ein agrochemisch akzeptables Salz davon, worin
Q¹ für die Gruppen Q¹-1.1 bis Q¹-5.7
| | | | | |
|---|---|---|---|---|
| 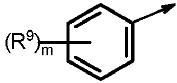 | 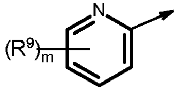 | 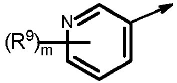 | 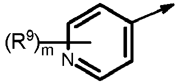 | 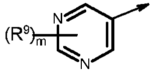 |
| Q¹-1.1 | Q¹-2.1 | Q¹-2.2 | Q¹-2.3 | Q¹-3.3 |
| 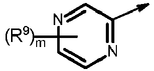 | 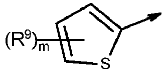 | 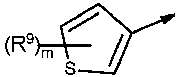 | 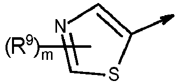 | 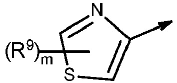 |
| Q¹-3.4 | Q¹-5.1 | Q¹-5.2 | Q¹-5.4 | Q¹-5.5 |
| 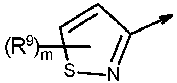 | 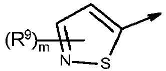 | | | |
| Q¹-5.6 | Q¹-5.7 | | | |
steht,
Q² für Phenyl steht,
welches unsubstituiert oder jeweils unabhängig voneinander substituiert ist durch m Reste ausgewählt aus der Gruppe bestehend aus Wasserstoff, Fluor, Chlor und Brom,
Z für die Gruppen 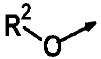
und 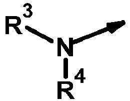 steht,
Y für Fluor, Chlor, Brom, Cyano, NO₂, Methyl, CF₃ und OCF₃ steht,
W für Sauerstoff steht,
R¹ für Wasserstoff, Methyl und Ethyl steht,
R² für Wasserstoff, Methyl, Ethyl, Allyl, Propargyl und PhCH₂ steht,
R³ für Wasserstoff steht,
R⁴ für (C₁-C₆)-Alkyl, S(O)ₙR⁵, OR⁵, SO₂NR⁶R⁷, CO₂R⁸, COR⁸ steht,
welche unsubstituiert sind oder jeweils unabhängig voneinander substituiert sind durch m Reste ausgewählt aus der Gruppe bestehend aus Wasserstoff, OR⁵, S(O)ₙR⁵, SO₂NR⁶R⁷, CO₂R⁸, COR⁶, NR⁶CO₂R⁸,
R⁵ für Methyl, Ethyl, CF₃, CH₂CF₃ steht,
R⁶ für Wasserstoff, und R⁵ steht,
R⁷ für Wasserstoff, Methyl, Ethyl, und Ethyl-2-ethanoyl steht,
R⁸ für Methyl, und Ethyl steht,
R⁹ für Wasserstoff, Fluor, Chlor, Brom, Cyano, Hydroxy, Methyl, Ethyl, OCH₃, CF₃, und OCF₃ steht,
m für 0, 1 und 2 steht,
n für 0, 1 und 2 steht.

6. Verfahren zur Herstellung von Verbindungen der allgemeinen Formel **(Ia)** oder eines agrochemisch akzeptablen Salzes davon gemäß einem der Ansprüche 1 bis 5, indem Verbindungen der allgemeinen Formeln (II) und (III) zu Verbindungen der allgemeinen Formel **(Ia)** umgesetzt werden 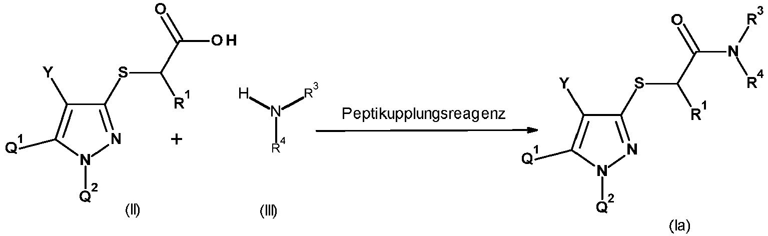 in welchen Q¹, Q², Y, R¹, R², R³ und R⁴ die oben angegebenen Bedeutungen.

7. Agrochemisches Mittel, enthaltend a) mindestens eine Verbindung der Formel (I) oder ein agrochemisch akzeptables Salz davon, wie in einem oder mehreren der Ansprüche 1 bis 5 definiert, und b) im Pflanzenschutz übliche Hilfs- und Zusatzstoffe.

8. Agrochemisches Mittel, enthaltend
a) mindestens eine Verbindung der Formel **(I)** oder ein agrochemisch akzeptables Salz davon, wie in einem oder mehreren der Ansprüche 1 bis 5 definiert,
b) einen oder mehrere von Komponente a) verschiedene agrochemische Wirkstoffe, und optional
c) im Pflanzenschutz übliche Hilfs- und Zusatzstoffe.

9. Verfahren zur Bekämpfung von unerwünschten Pflanzen oder zur Wachstumsregulierung von Pflanzen, wobei eine wirksame Menge mindestens einer Verbindung der Formel **(I)** oder ein agrochemisch akzeptables Salz davon, wie in einem oder mehreren der Ansprüche 1 bis 5 definiert, auf die Pflanzen, das Saatgut oder die Fläche, auf der die Pflanzen wachsen, appliziert wird.

10. Verwendung von Verbindungen der Formel **(I)** oder ein agrochemisch akzeptables Salz davon, wie in einem oder mehreren der Ansprüche 1 bis 5 definiert, als Herbizide oder Pflanzenwachstumsregulatoren.

11. Verwendung nach Anspruch 10, wobei die Verbindungen der Formel **(I)** oder ein agrochemisch akzeptables Salz davon zur Bekämpfung von Schadpflanzen oder zur Wachstumsregulierung in Pflanzenkulturen eingesetzt werden.

12. Verwendung nach Anspruch 11, wobei die Kulturpflanzen transgene oder nicht transgene Kulturpflanzen sind.

## Claims

1. [(1,4,5-Trisubstituted 1H-pyrazol-3-yl)sulfanyl]acetic acid derivatives of the general formula (I) or an agrochemically acceptable salt thereof: 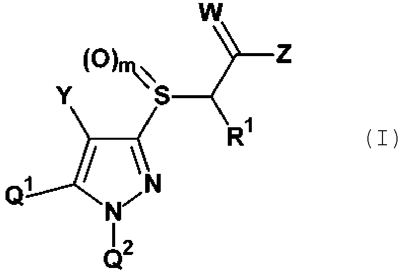 in which
Q¹ is phenyl and hetaryl,
where the phenyl and the hetaryl are unsubstituted or each independently substituted by m radicals selected from the group consisting of hydrogen, halogen, cyano, isocyano, nitro, hydroxy, (C₁-C₆) -alkyl, (C₁-C₆)-haloalkyl, (C₃-C₆) -cycloalkyl, (C₃-C₆) -halocycloalkyl, (C₁-C₆) -haloalkoxy, (C₂-C₃) -alkenyl, (C₂-C₃) -haloalkenyl, (C₁-C₆) -alkoxy, (C₂-C₃) -alkynyl, (C₂-C₃) -haloalkynyl, (C₁-C₄)-alkyl-S(O)ₙ, (C₁-C₄)-haloalkyl-S(O)ₙ, CHO, (C₁-C₄)-alkyloxycarbonyl and NH₂ (amino),
Q² is phenyl,
which is unsubstituted or in each case independently substituted by m radicals selected from the group consisting of hydrogen, halogen, cyano, isocyano, NO₂, (C₁-C₆)-alkyl, (C₁-C₆)-haloalkyl, (C₁-C₆)-haloalkoxy, (C₃-C₆)-cycloalkyl, (C₃-C₆)-halocycloalkyl, (C₂-C₃)-alkenyl, (C₂-C₃)-haloalkenyl, (C₁-C₆)-alkoxy, (C₂-C₃)-alkynyl, (C₂-C₃)-haloalkynyl, (C₁-C₄)-alkyl-S(O)ₙ, (C₁-C₄)-haloalkylS(O)ₙ, CHO, (C₁-C₄)-alkyloxycarbonyl and NH₂ (amino),
Z is the groups 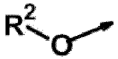 and 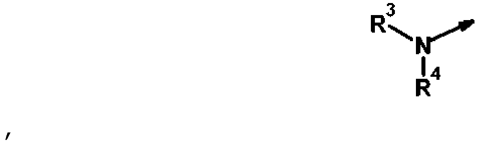
Y is halogen, cyano, isocyano, NO₂, NH₂ (amino), (C₁-C₆)-alkyl, (C₁-C₆)-haloalkyl, (C₁-C₆)-cyanoalkyl, (C₁-C₆)-hydroxyalkyl, (C₁-C₆)-alkoxy- (C₁-C₆)-alkyl, (C₃-C₆)-cycloalkyl, (C₁-C₄)-alkyloxycarbonyl, (C₁-C₆)-alkylcarbonyl, CHO, (C₃-C₆)-halocycloalkyl, (C₁-C₆)-alkoxy, (C₁-C₆)-haloalkoxy, (C₁-C₄)-alkyl-S(O)ₙ, (C₁-C₄)-haloalkyl-S(O)ₙ, (C₂-C₆)-alkynyl, (C₂-C₆)-haloalkynyl, (C₂-C₆)-alkenyl and (C₂-C₆)-haloalkenyl,
W is oxygen or sulfur,
R¹ is hydrogen, cyano, (C₁-C₆)-alkyl, (C₁-C₆)-haloalkyl, (C₁-C₆)-alkoxy, (C₁-C₆)-alkoxy- (C₁-C₆)-alkyl, (C₃-C₆)-cycloalkyl, (C₃-C₆)-halocycloalkyl, (C₂-C₆)-alkynyl, (C₂-C₆)-haloalkynyl, (C₂-C₆)-alkenyl and (C₂-C₆)-haloalkenyl,
R² is hydrogen, (C₁-C₁₀)-alkyl and (C₃-C₁₀)-cycloalkyl,
where the alkyl and cycloalkyl are unsubstituted or each independently substituted by m radicals selected from the group consisting of halogen, cyano, isocyano, nitro, NH₂ (amino), (C₁-C₆)-alkyl, (C₁-C₆)-haloalkyl, (C₁-C₆)-haloalkoxy, (C₂-C₃)-alkenyl, (C₂-C₃)-haloalkenyl, (C₁-C₆)-alkoxy, (C₂-C₃)-alkynyl, (C₂-C₃)-haloalkynyl, (C₁-C₄)-alkyl-S(O)ₙ, CHO, (C₁-C₄)-alkyloxycarbonyl, heterocyclyl-(C₁-C₄)-alkyl, heteroaryl-(C₁-C₄)-alkyl and aryl-(C₁-C₄)-alkyl, where the aryl, heterocyclyl and heteroaryl are unsubstituted or each independently substituted by m radicals selected from the group consisting of halogen, (C₁-C₆)-alkyl and (C₁-C₆)-haloalkyl,
R³ is hydrogen and (C₁-C₁₂)alkyl,
R⁴ is hydrogen, cyano, nitro, (C₁-C₁₂)-alkyl, (C₁-C₁₀)-haloalkyl, (C₂-C₁₀)-alkenyl, (C₃-C₁₀)-alkynyl, (C₁-C₁₀)-alkoxy- (C₁-C₁₀)-alkyl, (C₁-C₁₀)-haloalkoxy- (C₁-C₁₀)-alkyl, (C₁-C₄)-alkyl-S(O)ₙ- (C₁-C₄)-alkyl, (C₁-C₄)-haloalkylS(O)ₙ-(C₁-C₄)-alkyl, (C₃-C₁₀)-cycloalkyl, (C₃-C₁₀)-halocycloalkyl, hydroxy-(C₁ -C₁₀)-alkylcarbonyl, amino-(C₁-C₁₀)-alkyl, (C₁-C₁₀)-alkoxycarbonyl- (C₁-C₁₀)-alkyl, (C₁-C₁₀)-cyanoalkyl, S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸, aryl, heteroaryl and heterocyclyl,
which are unsubstituted or each independently substituted by m radicals selected from the group consisting of hydrogen, halogen, cyano, nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
or
R³ and R⁴ together with the nitrogen atom to which they are bonded form a fully saturated or partly saturated 3-to 10-membered monocyclic or bicyclic ring optionally interrupted by heteroatoms and optionally having further substitution,
R⁵ is hydrogen, (C₁-C₈)-alkyl, (C₃-C₆)-cycloalkyl, (C₁-C₈)-haloalkyl and aryl,
R⁶ is hydrogen and R⁵,
R⁷ is hydrogen, (C₁-C₆)-alkyl, (C₃-C₆)-cycloalkyl, (C₃-C₄)-alkenyl, (C₃-C₄)-alkynyl and (C₁-C₁₀)-alkylcarbonyl-(C₁-C₆)-alkyl,
R⁸ is hydrogen, (C₁-C₆)-alkyl, (C₃-C₆)-cycloalkyl, (C₃-C₄)-alkenyl and (C₃-C₄)-alkynyl,
m is 0, 1 or 2,
and
n is 0, 1 or 2,
excluding the compounds ethyl {[4-nitro-1-phenyl-5-(1H-tetrazol-5-yl)-1H-pyrazol-3-yl]sulfanyl}acetate CAS [1360702-86-8] and methyl {[5-(5-methyl-1,3,4-oxadiazol-2-yl)-4-nitro-1-phenyl-1H-pyrazol-3-yl]sulfanyl}acetate CAS [1360690-60-3].

2. Compounds of the formula (I) according to Claim 1 or an agrochemically acceptable salt thereof, in which Q¹ is the groups Q¹-1.1 to Q¹-6.5
| | | | | |
|---|---|---|---|---|
| 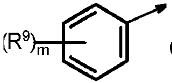 | 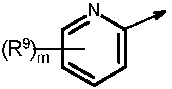 | 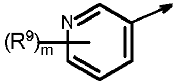 | 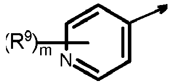 | 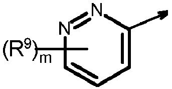 |
| Q¹-1.1 | Q¹-2.1 | Q¹-2.2 | Q¹-2.3 | Q¹-3.1 |
| 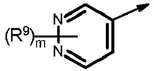 | 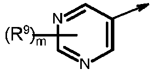 | 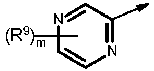 | 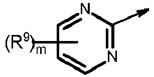 | 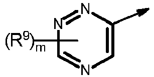 |
| Q¹-3.2 | Q¹-3.4 | Q¹-3.4 | Q¹-3.5 | Q¹-4.1 |
| 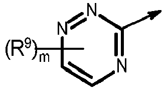 | 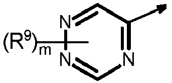 | 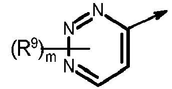 | 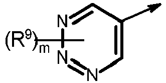 | 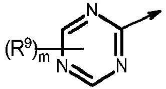 |
| Q¹-4.2 | Q¹-4.3 | Q¹-4.4 | Q¹-4.5 | Q¹-4.6 |
| 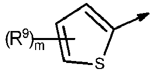 | 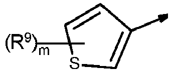 | 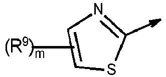 | 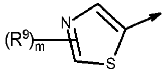 | 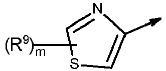 |
| Q¹-5.1 | Q¹-5.2 | Q¹-5.3 | Q¹-5.4 | Q¹-5.5 |
| 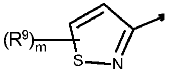 | 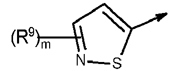 | 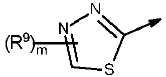 | 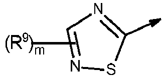 | 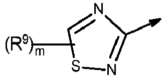 |
| Q¹-5.6 | Q¹-5.7 | Q¹-6.1 | Q¹-6.2 | Q¹-6.3 |
| 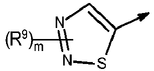 | 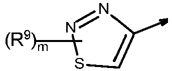 | | | |
| Q¹-6.4 | Q¹-6.5 | | | |
,
Q² is phenyl,
which is unsubstituted or in each case independently substituted by m radicals selected from the group consisting of hydrogen, halogen, cyano, isocyano, NO₂, (C₁-C₆)-alkyl, (C₁-C₆)-haloalkyl, (C₁-C₆)-haloalkoxy, (C₃-C₆)-cycloalkyl, (C₃-C₆)-halocycloalkyl, (C₂-C₃)-alkenyl, (C₂-C₃)-haloalkenyl, (C₁-C₆)-alkoxy, (C₂-C₃)-alkynyl, (C₂-C₃)-haloalkynyl, (C₁-C₄)-alkyl-S(O)ₙ, (C₁-C₄)-haloalkylS(O)ₙ, CHO, (C₁-C₄)-alkyloxycarbonyl and NH₂ (amino),
Z is the groups 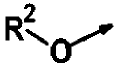 and 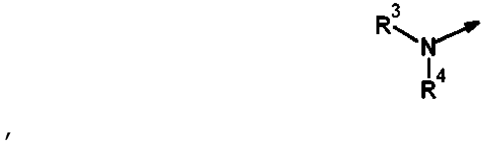
Y is halogen, cyano, isocyano, NO₂, NH₂ (amino), (C₁-C₆)-alkyl, (C₁-C₆)-haloalkyl, (C₁-C₆)-cyanoalkyl, (C₁-C₆)-hydroxyalkyl, (C₁-C₆)-alkoxy- (C₁-C₆)-alkyl, (C₃-C₆)-cycloalkyl, (C₁-C₄)-alkyloxycarbonyl, (C₁-C₆)-alkylcarbonyl, CHO, (C₃-C₆)-halocycloalkyl, (C₁-C₆)-alkoxy, (C₁-C₆)-haloalkoxy, (C₁-C₄)-alkyl-S(O)ₙ, (C₁-C₄)-haloalkyl-S(O)ₙ, (C₂-C₆)-alkynyl, (C₂-C₆)-haloalkynyl, (C₂-C₆)-alkenyl and (C₂-C₆)-haloalkenyl,
W is oxygen or sulfur,
R¹ is hydrogen, cyano, (C₁-C₆)-alkyl, (C₁-C₆)-haloalkyl, (C₁-C₆)-alkoxy, (C₁-C₆)-alkoxy- (C₁-C₆)-alkyl, (C₃-C₆)-cycloalkyl, (C₃-C₆)-halocycloalkyl, (C₂-C₆)-alkynyl, (C₂-C₆)-haloalkynyl, (C₂-C₆)-alkenyl and (C₂-C₆)-haloalkenyl,
R² is hydrogen, (C₁-C₁₀)-alkyl and (C₃-C₁₀)-cycloalkyl,
where the alkyl and cycloalkyl are unsubstituted or each independently substituted by m radicals selected from the group consisting of halogen, cyano, isocyano, nitro, NH₂ (amino), (C₁-C₆)-alkyl, (C₁-C₆)-haloalkyl, (C₁-C₆)-haloalkoxy, (C₂-C₃)-alkenyl, (C₂-C₃)-haloalkenyl, (C₁-C₆)-alkoxy, (C₂-C₃)-alkynyl, (C₂-C₃)-haloalkynyl, (C₁-C₄)-alkyl-S(O)ₙ, CHO, (C₁-C₄)-alkyloxycarbonyl, heterocyclyl-(C₁-C₆)-alkyl, heteroaryl- (C₁-C₄)-alkyl and aryl-(C₁-C₄)-alkyl, where the aryl, heterocyclyl and heteroaryl are unsubstituted or each independently substituted by m radicals selected from the group consisting of halogen, (C₁-C₆)-alkyl and (C₁-C₆)-haloalkyl,
R³ is hydrogen and (C₁-C₁₀)alkyl,
R⁴ is hydrogen, cyano, nitro, (C₁-C₁₀)-alkyl, (C₁-C₁₀)-haloalkyl, (C₂-C₁₀)-alkenyl, (C₃-C₁₀)-alkynyl, (C₁-C₁₀)-alkoxy- (C₁-C₁₀)-alkyl, (C₁-C₁₀)-haloalkoxy- (C₁-C₁₀)-alkyl, (C₁-C₄)-alkyl-S(O)ₙ- (C₁-C₄)-alkyl, (C₁-C₄)-haloalkylS(O)ₙ- (C₁-C₄)-alkyl, (C₃-C₁₀)-cycloalkyl, (C₃-C₁₀) - halocycloalkyl, hydroxy-(C₁-C₁₀)-alkylcarbonyl, amino-(C₁-C₁₀)-alkyl, (C₁-C₁₀)-alkoxycarbonyl- (C₁-C₁₀)-alkyl, (C₁-C₁₀)-cyanoalkyl, S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸; aryl, heteroaryl and heterocyclyl,
which are unsubstituted or each independently substituted by m radicals selected from the group consisting of hydrogen, halogen, cyano, nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
or R³ and R⁴ together with the nitrogen atom to which they are bonded form a fully saturated or partly saturated 3-to 10-membered monocyclic or bicyclic ring optionally interrupted by heteroatoms and optionally having further substitution,
R⁵ is hydrogen, (C₁-C₈) -alkyl, (C₃-C₆)-cycloalkyl, (C₁-C₈)-haloalkyl and aryl,
R⁶ is hydrogen and R⁵,
R⁷ is hydrogen, (C₁-C₆) -alkyl, (C₃-C₆)-cycloalkyl, (C₃-C₄)-alkenyl, (C₃-C₄)-alkynyl and (C₂-C₆)-alkylcarbonyl-(C₁-C₄)-alkyl,
R⁸ is hydrogen, (C₁-C₆)-alkyl, (C₃-C₆)-cycloalkyl, (C₃-C₄)-alkenyl and (C₃-C₄)-alkynyl;
R⁹ is hydrogen, halogen, cyano, isocyano, nitro, hydroxy, (C₁-C₆)-alkyl, (C₁-C₆)-haloalkyl, (C₂-C₆)-haloalkoxy, (C₂-C₃)-alkenyl, (C₂-C₃)-haloalkenyl, (C₂-C₆)-alkoxy, (C₂-C₃)-alkynyl, (C₂-C₃)-haloalkynyl, (C₂-C₄)-alkyl-S(O)ₙ, CHO, (C₁-C₄)-alkyloxycarbonyl and NH₂ (amino),
m is 0, 1 and 2,
n is 0, 1 and 2.

3. Compounds of the formula (I) according to Claim 1 or 2 or an agrochemically acceptable salt thereof, in which
Q¹ is the groups Q⁷-1.1 to Q¹-6.3,
| | | | | |
|---|---|---|---|---|
| 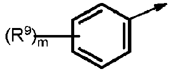 | 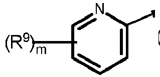 | 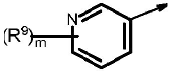 | 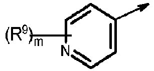 | 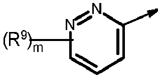 |
| Q¹-1.1 | Q¹-2.1 | Q¹-2.2 | Q¹-2.3 | Q¹-3.1 |
| 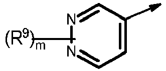 | 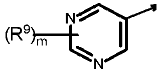 | 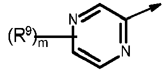 | 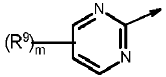 | 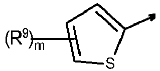 |
| Q¹-3.2 | Q¹-3.4 | Q¹-3.4 | Q¹-3.5 | Q¹-5.1 |
| 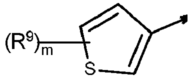 | 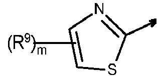 | 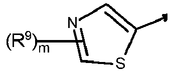 | 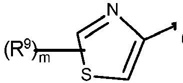 | 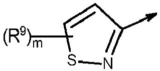 |
| Q¹-5.2 | Q¹-5.3 | Q¹-5.4 | Q¹-5.5 | Q¹-5.6 |
| 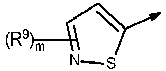 | 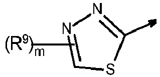 | 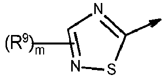 | 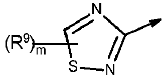 | |
| Q¹-5.7 | Q¹-6.1 | Q¹-6.2 | Q¹-6.3 | |
Q² is phenyl,
which is unsubstituted or in each case independently substituted by m radicals selected from the group consisting of hydrogen, halogen, cyano, (C₁-C₆)-alkyl, (C₁-C₆)-haloalkyl, (C₁-C₆)-haloalkoxy, (C₃-C₆)-cycloalkyl, (C₁-C₆)-alkoxy, (C₁-C₄)-alkyl-S(O)ₙ and (C₁-C₄)-haloalkylS(O)ₙ,
Z is the groups 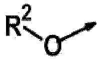 and 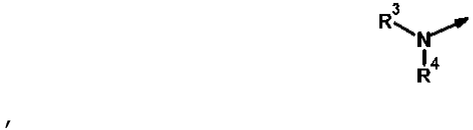
Y is halogen, cyano, isocyano, NO₂, (C₁-C₆)-alkyl, (C₁-C₆)-haloalkyl, (C₁-C₆)-alkoxy- (C₁-C₆)-alkyl, (C₁-C₄)-alkyloxycarbonyl , (C₁-C₆)-alkylcarbonyl, (C₁-C₆)-alkoxy, (C₁-C₆)-haloalkoxy, (C₁-C₄)-alkyl-S(O)ₙ, (C₁-C₄)-haloalkyl-S(O)ₙ, (C₂-C₆)-alkynyl, (C₂-C₆)-haloalkynyl, (C₂-C₆)-alkenyl and (C₂-C₆)-haloalkenyl,
W is oxygen,
R¹ is hydrogen, cyano, (C₁-C₆)-alkyl, (C₁-C₆)-haloalkyl, (C₁-C₆)-alkoxy, (C₁-C₄)-alkoxy- (C₁-C₄)-alkyl, (C₃-C₆)-cycloalkyl, (C₂-C₆)-alkynyl, (C₂-C₆)-haloalkynyl, (C₂-C₆)-alkenyl and (C₂-C₆)-haloalkenyl,
R² is hydrogen, (C₁-C₆)-alkyl and (C₃-C₆)-cycloalkyl, where the alkyl and cycloalkyl are unsubstituted or each independently substituted by m radicals selected from the group consisting of halogen, cyano, nitro, NH₂ (amino), (C₁-C₆)-alkyl, (C₁-C₆)-haloalkyl, (C₁-C₆)-haloalkoxy, (C₂-C₃)-alkenyl, (C₂-C₃)-haloalkenyl, (C₁-C₆)-alkoxy, (C₂-C₃)-alkynyl, (C₂-C₃)-haloalkynyl, (C₁-C₄)-alkyl-S(O)ₙ, CHO, (C₁-C₄)-alkyloxycarbonyl, heterocyclyl- (C₁-C₄)-alkyl, heteroaryl-(C₁-C₄)-alkyl and aryl-(C₁-C₄)-alkyl, where the aryl, heterocyclyl and heteroaryl are unsubstituted or each independently substituted by m radicals selected from the group consisting of halogen, (C₁-C₆)-alkyl and (C₁-C₆)-haloalkyl,
R³ is hydrogen and (C₁-C₆)alkyl,
R⁴ is hydrogen, cyano, nitro, (C₁-C₆)-alkyl, (C₁-C₆)-haloalkyl, (C₂-C₆)-alkenyl, (C₃-C₆)-alkynyl, (C₁-C₆)-alkoxy- (C₁-C₆)-alkyl, (C₁-C₆)-haloalkoxy- (C₁-C₆)-alkyl, (C₁-C₄)-alkyl-S(O)ₙ- (C₁-C₄)-alkyl and (C₁-C₄)-haloalkylS(O)ₙ- (C₁-C₄)-alkyl, (C₃-C₆)-cycloalkyl, (C₃-C₆)-halocycloalkyl, hydroxy-(C₁-C₆)-alkylcarbonyl, amino-(C₁-C₆)-alkyl, (C₁-C₆)-alkoxycarbonyl-(C₁-C₆)-alkyl, S(O)ₙR5, OR⁵, SO₂NR⁶R⁷, CO₂R⁸, COR⁸, NR⁶R⁸, NR⁶COR⁸, NR⁶CO₂R⁸, NR⁶SO₂R⁸; aryl, heteroaryl and heterocyclyl,
which are unsubstituted or each independently substituted by m radicals selected from the group consisting of hydrogen, halogen, cyano, nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
or R³ and R⁴ together with the nitrogen atom to which they are bonded form a fully saturated or partly saturated 3-to 10-membered monocyclic or bicyclic ring optionally interrupted by heteroatoms and optionally having further substitution,
R⁵ is hydrogen, (C₁-C₈)-alkyl, (C₃-C₆)-cycloalkyl, (C₁-C₈)-haloalkyl and aryl,
R⁶ is hydrogen and R⁵,
R⁷ is hydrogen, (C₁-C₆)-alkyl, (C₃-C₆)-cycloalkyl, (C₃-C₄)-alkenyl and (C₃-C₄)-alkynyl,
R⁸ is hydrogen, (C₁-C₆)-alkyl, (C₃-C₆)-cycloalkyl, (C₃-C₄)-alkenyl, (C₃-C₄)-alkynyl and (C₁-C₁₀)-alkylcarbonyl-(C₁-C₆)-alkyl,
R⁹ is hydrogen, halogen, cyano, isocyano, nitro, hydroxy, (C₁-C₆)-alkyl, (C₁-C₆)-haloalkyl, (C₂-C₆)-haloalkoxy, (C₂-C₃)-alkenyl, (C₂-C₃)-haloalkenyl, (C₂-C₆)-alkoxy, (C₂-C₃)-alkynyl, (C₂-C₃)-haloalkynyl, (C₂-C₄)-alkyl-S(O)ₙ, CHO, (C₁-C₄)-alkyloxycarbonyl and NH₂ (amino),
m is 0, 1 and 2,
n is 0, 1 and 2.

4. Compounds of the formula (I) according to any of Claims 1 to 3 or an agrochemically acceptable salt thereof, in which
Q¹ is the groups Q¹-1.1 to Q¹-6.3,
| | | | | |
|---|---|---|---|---|
| 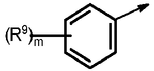 | 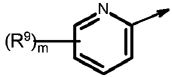 | 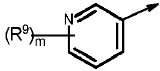 | 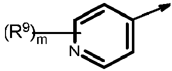 | 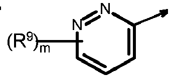 |
| Q¹-1.1 | Q¹-2.1 | Q¹-2.2 | Q¹-2.3 | Q¹-3.1 |
| 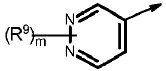 | 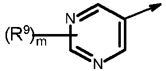 | 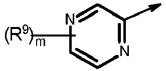 | 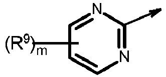 | 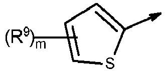 |
| Q¹-3.2 | Q¹-3.4 | Q¹-3.4 | Q¹-3.5 | Q¹-5.1 |
| 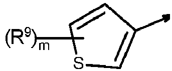 | 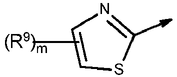 | 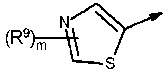 | 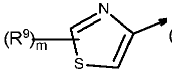 | 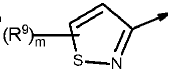 |
| Q¹-5.2 | Q¹-5.3 | Q¹-5.4 | Q¹-5.5 | Q¹-5.6 |
| 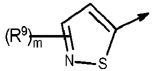 | 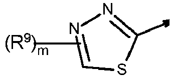 | 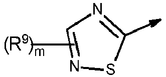 | 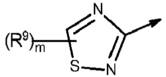 | |
| Q¹-5.7 | Q¹-6.1 | Q¹-6.2 | Q¹-6. 3 | |
Q² is phenyl,
which is unsubstituted or in each case independently substituted by m radicals selected from the group consisting of hydrogen, fluorine, chlorine and bromine,
Z is the groups 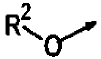 and 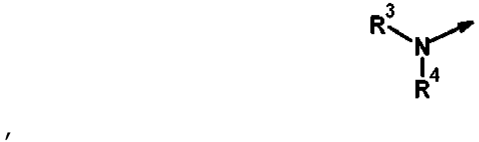
Y is fluorine, chlorine, bromine, cyano, NO₂, (C₁-C₂)-alkyl, (C₁-C₂)-haloalkyl, (C₁-C₂)-alkylcarbonyl, (C₁-C₂)-alkoxy, (C₁-C₂)-haloalkoxy, (C₁-C₂)-alkyl-S(O)ₙ and (C₁-C₂)-haloalkyl-S(O)ₙ,
W is oxygen,
R¹ is hydrogen, (C₁-C₄)-alkyl, (C₁-C₄)-haloalkyl and (C₁-C₆)-alkoxy,
R² is hydrogen, (C₁-C₆)-alkyl and (C₃-C₆)-cycloalkyl, where the alkyl and cycloalkyl are unsubstituted or each independently substituted by m radicals selected from the group consisting of halogen, cyano, nitro, NH₂ (amino), (C₁-C₆)-alkyl, (C₁-C₆)-haloalkyl, (C₁-C₆)-haloalkoxy, (C₂-C₃)-alkenyl, (C₂-C₃)-haloalkenyl, (C₂-C₆)-alkoxy, (C₂-C₃)-alkynyl, (C₂-C₃)-haloalkynyl, (C₁-C₄)-alkyl-S(O)ₙ, CHO, (C₁-C₄) -alkyloxycarbonyl, heterocyclyl- (C₁-C₄) -alkyl, heteroaryl-(C₁-C₄)-alkyl and aryl-(C₁-C₄)-alkyl, where the aryl, heterocyclyl and heteroaryl are unsubstituted or each independently substituted by m radicals selected from the group consisting of halogen, (C₁-C₆)-alkyl and (C₁-C₆)-haloalkyl,
R³ is hydrogen and (C₁-C₆)alkyl,
R⁴ is hydrogen, cyano, nitro, (C₁-C₆)-alkyl, (C₁-C₆)-haloalkyl, (C₂-C₆)-alkenyl, (C₃-C₆)-alkynyl, (C₂-C₆) - alkoxy- (C₁-C₆)-alkyl, (C₂-C₆)-haloalkoxy- (C₁-C₆)-alkyl, (C₁-C₄)-alkyl-S(O)ₙ- (C₁-C₄)-alkyl and (C₁-C₄)-haloalkylS(O)ₙ-(C₁-C₄)-alkyl, (C₃-C₆)-cycloalkyl, (C₃-C₆)-halocycloalkyl, hydroxy-(C₁-C₆)-alkylcarbonyl, amino-(C₁-C₆) -alkyl, (C₁-C₆)-alkoxycarbonyl-(C₁-C₆)-alkyl, S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸; aryl, heteroaryl and heterocyclyl,
which are unsubstituted or each independently substituted by m radicals selected from the group consisting of hydrogen, halogen, cyano, nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
or R³ and R⁴ together with the nitrogen atom to which they are bonded form a fully saturated or partly saturated 3-to 10-membered monocyclic or bicyclic ring optionally interrupted by heteroatoms and optionally having further substitution,
R⁵ is hydrogen, (C₂-C₈)-alkyl, (C₃-C₆)-cycloalkyl, (C₁-C₈)-haloalkyl and aryl,
R⁶ is hydrogen and R⁵,
R⁷ is hydrogen, (C₁-C₆)-alkyl, (C₃-C₆)-cycloalkyl, (C₃-C₄)-alkenyl, (C₃-C₄)-alkynyl and (C₁-C₁₀)-alkylcarbonyl-(C₁-C₆)-alkyl,
R⁸ is hydrogen, (C₁-C₆)-alkyl, (C₃-C₆)-cycloalkyl, (C₃-C₄)-alkenyl and (C₃-C₄)-alkynyl;
R⁹ is hydrogen, halogen, cyano, isocyano, nitro, hydroxy, (C₁-C₆)-alkyl, (C₁-C₆)-haloalkyl, (C₂-C₆)-haloalkoxy, (C₂-C₃)-alkenyl, (C₂-C₃)-haloalkenyl, (C₂-C₆)-alkoxy, (C₂-C₃)-alkynyl, (C₂-C₃)-haloalkynyl, (C₂-C₄)-alkyl-S(O)ₙ, CHO, (C₁-C₄)-alkyloxycarbonyl and NH₂ (amino),
m is 0, 1 and 2,
n is 0, 1 and 2.

5. Compounds of the formula (I) according to any of Claims 1 to 3 or an agrochemically acceptable salt thereof, in which Q¹ is the groups Q¹-1.1 to Q¹-5.7
| | | | | |
|---|---|---|---|---|
| 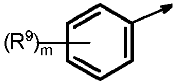 | 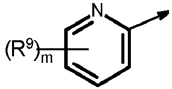 | 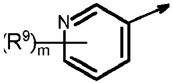 | 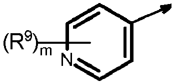 | 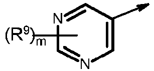 |
| Q¹-1.1 | Q¹-2.1 | Q¹-2.2 | Q¹-2.3 | Q¹-3.3 |
| 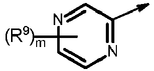 | 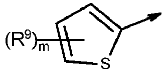 | 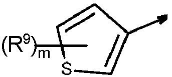 | 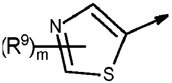 | 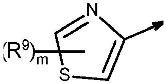 |
| Q¹-3.4 | Q¹-5.1 | Q¹-5.2 | Q¹-5.4 | Q¹-5.5 |
| 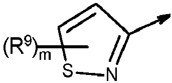 | 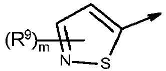 | | | |
| Q¹-5.6 | Q¹-5.7 | | | |
,
Q² is phenyl,
which is unsubstituted or in each case independently substituted by m radicals selected from the group consisting of hydrogen, fluorine, chlorine and bromine,
Z is the groups 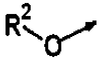 and 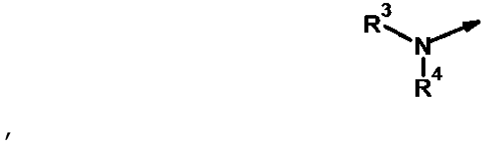
Y is fluorine, chlorine, bromine, cyano, NO₂, methyl, CF₃ and OCF₃,
W is oxygen,
R¹ is hydrogen, methyl and ethyl,
R² is hydrogen, methyl, ethyl, allyl, propargyl and PhCH₂,
R³ is hydrogen,
R⁴ is (C₁-C₆)-alkyl, S(O)ₙR⁵, OR⁵, SO₂NR⁶R⁷, CO₂R⁸, COR⁸, which are unsubstituted or in each case independently of one another substituted by m radicals selected from the group consisting of hydrogen, OR⁵, S(O)ₙR⁵, SO₂NR⁶R⁷, CO₂R⁸, COR⁶, NR⁶CO₂R⁸,
R⁵ is methyl, ethyl, CF₃, CH₂CF₃,
R⁶ is hydrogen and R⁵,
R⁷ is hydrogen, methyl, ethyl and ethyl-2-ethanoyl,
R⁸ is methyl and ethyl,
R⁹ is hydrogen, fluorine, chlorine, bromine, cyano, hydroxy, methyl, ethyl, OCH₃, CF₃ and OCF₃,
m is 0, 1 and 2,
n is 0, 1 and 2.

6. Process for preparing compounds of the general formula (Ia) or an agrochemically acceptable salt thereof according to any of Claims 1 to 5 by converting compounds of the general formulae (II) and (III) to compounds of the general formula (Ia) 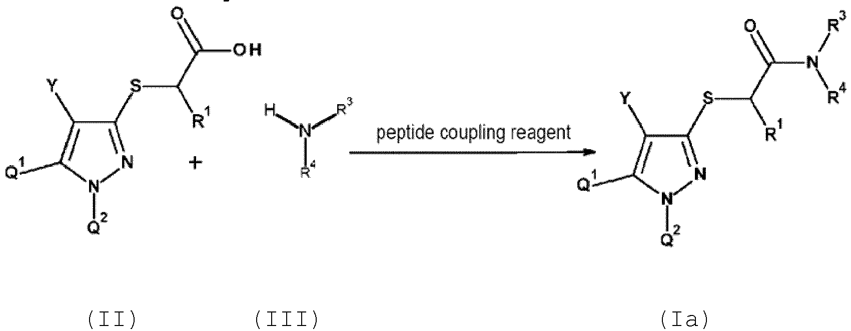 in which Q¹, Q², Y, R¹, R², R³ and R⁴ have the definitions given above.

7. Agrochemical composition comprising a) at least one compound of the formula (I) or an agrochemically acceptable salt thereof as defined in one or more of Claims 1 to 5, and b) auxiliaries and additives customary in crop protection.

8. Agrochemical composition comprising
a) at least one compound of the formula (I) or an agrochemically acceptable salt thereof as defined in one or more of Claims 1 to 5,
b) one or more active agrochemical ingredients other than component a), and optionally
c) auxiliaries and additives customary in crop protection.

9. Method of controlling unwanted plants or of regulating the growth of plants, wherein an effective amount of at least one compound of the formula (I) or an agrochemically acceptable salt thereof, as defined in one or more of Claims 1 to 5, is applied to the plants, the seed or the area in which the plants grow.

10. Use of compounds of the formula (I) or an agrochemically acceptable salt thereof, as defined in one or more of Claims 1 to 5, as herbicides or plant growth regulators.

11. Use according to Claim 10, wherein the compounds of the formula (I) or an agrochemically acceptable salt thereof are used for controlling harmful plants or for regulating growth in plant crops.

12. Use according to Claim 11, wherein the crop plants are transgenic or nontransgenic crop plants.

## Revendications

1. Dérivés d'acide [(1,4,5-trisubstitué-1H-pyrazol-3-yl)sulfanyl]acétique de formule générale (I) ou leurs sels agrochimiquement acceptables 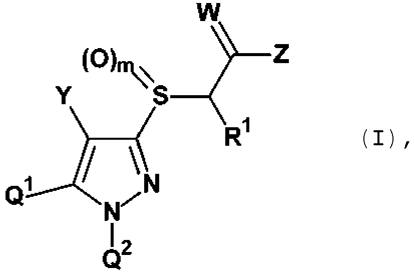 formule dans laquelle
Q¹ représente un groupe phényle ou hétéroaryle,
le groupe phényle et le groupe hétéroaryle étant non substitués ou substitués chacun indépendamment l'un de l'autre par m radicaux choisis dans le groupe constitué par un atome d'hydrogène, un atome d'halogène, un groupe cyano, isocyano, nitro, hydroxy, alkyle en C₁-C₆, halogénoalkyle (C₁-C₆), cycloalkyle en C₃-C₆, halogénocycloalkyle (C₃-C₆), halogénoalcoxy (C₁-C₆), alcényle en C₂-C₃, halogénoalcényle(C₂-C₃), alcoxy en C₁-C₆, alcynyle en C₂-C₃, halogénoalcynyle (C₂-C₃), alkyl (C₁-C₄)-S(O)ₙ, halogénoalkyl (C₁-C₄)- S(O)ₙ, CHO, alkyloxy(C₁-C₄)-carbonyle et NH₂ (amino),
Q² représente un groupe phényle,
qui est non substitué ou substitué chaque fois indépendamment par m radicaux choisis dans le groupe constitué par un atome d'hydrogène, un atome d'halogène, un groupe cyano, isocyano, NO₂, alkyle en C₁-C₆, halogénoalkyle (C₁-C₆), halogénoalcoxy (C₁-C₆), cycloalkyle en C₃-C₆, halogénocycloalkyle (C₃-C₆), alcényle en C₂-C₃, halogénoalcényle(C₂-C₃), alcoxy en C₁-C₆, alcynyle en C₂-C₃, halogénoalcynyle (C₂-C₃), alkyl(C₁-C₄)-S(O)ₙ, halogénoalkyl (C₁-C₄)-S(O)ₙ, CHO, alkyloxy(C₁-C₄) - carbonyle et NH₂ (amino),
Z représente les groupes 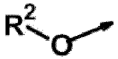 et 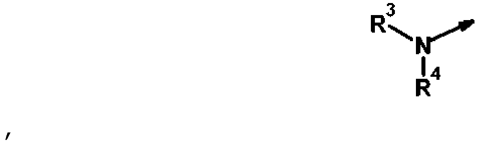
Y représente un atome d'halogène, un groupe cyano, isocyano, NO₂, NH₂ (amino), alkyle en C₁-C₆, halogénoalkyl (C₁-C₆), cyanoalkyle (C₁-C₆), hydroxyalkyle (C₁-C₆), alcoxy (C₁-C₆)-alkyle (C₁-C₆), cycloalkyle en C₃-C₆, alkyloxy(C₁-C₄)-carbonyle, alkyl (C₁-C₆)-carbonyle, CHO, halogénocycloalkyle(C₃-C₆), alcoxy en C₁-C₆, halogénoalcoxy (C₁-C₆), alkyl (C₁-C₄)-S(O)ₙ, halogénoalkyl (C₁-C₄)- S(O)ₙ, alcynyle en C₂-C₆, halogénoalcynyle (C₂-C₆), alcényle en C₂-C₆ ou halogénoalcényle (C₂-C₆),
W représente un atome d'oxygène ou de soufre,
R¹ représente un atome d'hydrogène, un groupe cyano, alkyle en C₁-C₆, halogénoalkyle (C₁-C₆), alcoxy en C₁-C₆, alcoxy(C₁-C₆)-alkyle(C₁-C₆), cycloalkyle en (C₃-C₆), halogénocycloalkyle(C₃-C₆), alcynyle en C₂-C₆, halogénoalcynyle(C₂-C₆), alcényle en C₂-C₆) ou halogénoalcényle(C₂-C₆),
R² représente un atome d'hydrogène, un groupe alkyle en C₁-C₁₀ ou cycloalkyle en C₃-C₁₀),
les groupes alkyle et cycloalkyle étant non substitués ou substitués chacun indépendamment l'un de l'autre par m radicaux choisis dans le groupe constitué par halogène, cyano, isocyano, nitro, NH₂ (amino), alkyle en C₁-C₆, halogénoalkyle(C₁-C₆), halogénoalcoxy (C₁-C₆), alcényle en C₂-C₃, halogénoalcényle(C₂-C₃), alcoxy en C₁-C₆, alcynyle en C₂-C₃, halogénoalcycnyle(C₂-C₃), alkyl(C₁-C₄)-S(O)ₙ, CHO, alkyloxy(C₁-C₄)-carbonyle, hétérocyclyl-alkyle(C₁-C₄), hétéroaryl-alkyle(C₁-C₄) et aryl-alkyle(C₁-C₄), les groupes aryle, hétérocyclyle et hétéroaryle étant non substitués ou substitués chacun indépendamment les uns des autres par m radicaux choisis dans le groupe constitué par halogène, alkyle en C₁-C₆, halogénoalkyle (C₁-C₆),
R³ représente un atome d'hydrogène ou un groupe alkyle en C₂-C₁₂ ;
R⁴ représente un atome d'hydrogène, un groupe cyano, nitro, alkyle en C₁-C₁₂, halogénoalkyle (C₁-C₁₀), alcényle en C₂-C₁₀, alcynyle en C₃-C₁₀, alcoxy (C₁-C₁₀) -alkyle(C₁-C₁₀), halogénoalcoxy(C₁-C₁₀)-alkyle(C₁-C₁₀), alkyl (C₁-C₄) - S(O)ₙ-alkyle(C₁-C₄), halogénoalkyl (C₁-C₄)-S(O)ₙ-alkyle (C₁-C₄), cycloalkyle en (C₃-C₁₀), halogénocycloalkyle(C₃-C₁₀), hydroxy-alkyl(C₁-C₁₀)-carbonyle, amino-alkyle(C₁-C₁₀), alcoxy(C₁-C₁₀)-carbonyl-alkyle(C₁-C₁₀), cyanoalkyle(C₁-C₁₀), S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸, aryle, hétéroaryle et hétérocyclyle , qui sont non substitués ou substitués chacun indépendamment les uns des autres par m radicaux choisis dans le groupe constitué par hydrogène, halogène, cyano, nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
ou
R³ et R⁴ forment avec l'atome d'azote, auquel ils sont liés, un cycle monocyclique ou bicyclique à 3 à 10 chaînons, totalement saturé ou partiellement saturé, éventuellement interrompu par des hétéroatomes et éventuellement substitué davantage,
R⁵ représente un atome d'hydrogène, un groupe alkyle en C₁-C₈, cycloalkyle en C₃-C₆, halogénoalkyle (C₁-C₈) ou aryle,
R⁶ représente un atome d'hydrogène ou R⁵,
R⁷ représente un atome d'hydrogène , un groupe alkyle en C₁-C₆, cycloalkyle en C₃-C₆, alcényle en C₃-C₄, alcynyle en C₃-C₄ ou alkyl (C₁-C₁₀) carbonyl-alkyle (C₁-C₆),
R⁸ représente un atome d'hydrogène, un groupe alkyle en C₁-C₆, cycloalkyle en C₃-C₆, alcényle en C₃-C₄ ou alcynyle en C₃-C₄,
m représente 0, 1 ou 2,
et
n représente 0, 1 ou 2,
les composés {[4-nitro-1-phényl-5-(1H-tétrazol-5-yl)-1H-pyrazol-3-yl]sulfanyl}acétate d'éthyle CAS [1360702-86-8] et {[5-(5-méthyl-1,3,4-oxadiazol-2-yl)-4-nitro-1-phényl-1H-pyrazol-3-yl]sulfanyl}acétate de méthyle CAS [1360690-60-3] étant exclus.

2. Composés de formule (I) selon la revendication 1 ou leurs sels agrochimiquement acceptables, dans lesquels
Q¹ représente les groupes Q¹-1.1 à Q¹-6.5
| | | | | |
|---|---|---|---|---|
| 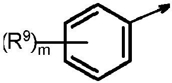 | 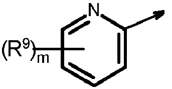 | 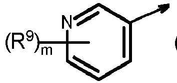 | 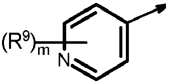 | 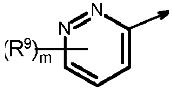 |
| Q¹-1.1 | Q¹-2.1 | Q¹-2.2 | Q¹-2.3 | Q¹-3.1 |
| 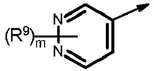 | 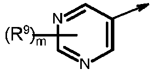 | 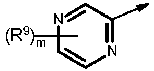 | 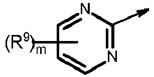 | 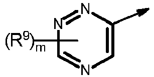 |
| Q¹-3.2 | Q¹-3.4 | Q¹-3.4 | Q¹-3.5 | Q¹-4.1 |
| 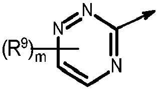 | 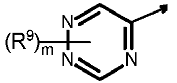 | 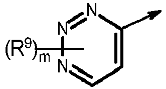 | 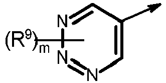 | 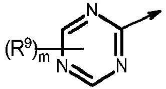 |
| Q¹-4.2 | Q¹-4.3 | Q¹-4.4 | Q¹-4.5 | Q¹-4.6 |
| 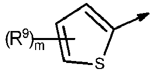 | 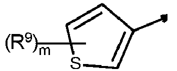 | 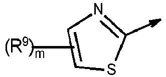 | 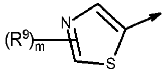 | 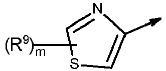 |
| Q¹-5.1 | Q¹-5.2 | Q¹-5.3 | Q¹-5.4 | Q¹-5.5 |
| 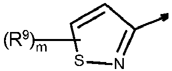 | 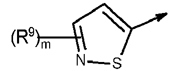 | 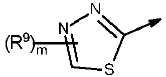 | 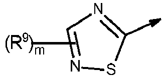 | 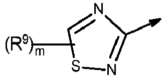 |
| Q¹-5.6 | Q¹-5.7 | Q¹-6.1 | Q¹-6.2 | Q¹-6.3 |
| 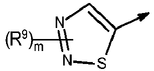 | 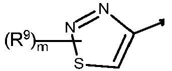 | | | |
| Q¹-6.4 | Q¹-6.5 | | | |
,
Q² représente un groupe phényle,
qui est non substitué ou substitué chaque fois indépendamment par m radicaux choisis dans le groupe constitué par hydrogène, halogène, cyano, isocyano, NO₂, alkyle en C₁-C₆, halogénoalkyle (C₁-C₆), halogénoalcoxy (C₁-C₆), cycloalkyle en C₃-C₆, halogénocycloalkyle (C₃-C₆), alcényle en C₂-C₃, halogénoalcényle (C₂-C₃), alcoxy en C₁-C₆, alcynyle en C₂-C₃, halogénoalcynyle (C₂-C₃), alkyl (C₁-C₄)-S(O)ₙ, halogénoalkyl(C₁-C₄)-S(O)ₙ, CHO, alkyloxy(C₂-C₄)-carbonyle et NH₂ (amino),
Z représente les groupes 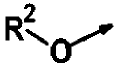 et 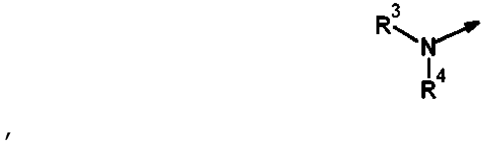
Y représente un atome d'halogène, un groupe cyano, isocyano, NO₂, NH₂ (amino), alkyle en C₁-C₆, halogénoalkyle (C₁-C₆), cyanoalkyle (C₁-C₆), hydroxyalkyle (C₁-C₆), alcoxy (C₁-C₆)-alkyle (C₁-C₆), cycloalkyle en C₃-C₆, alkyloxy (C₁-C₄) -carbonyle , alkyl (C₁-C₆)carbonyle, CHO, halogénocycloalkyle (C₃-C₆), alcoxy en C₁-C₆, halogénoalcoxy (C₁-C₆), alkyl (C₁-C₄) -S(O)ₙ, halogénoalkyl (C₁-C₄) -S(O)ₙ, alcynyle en C₂-C₆, halogénoalcynyle (C₂-C₆), alcényle en C₂-C₆ et halogénoalcényle (C₂-C₆),
W représente un atome d'oxygène ou de soufre,
R¹ représente un atome d'hydrogène, un groupe cyano, alkyle en C₁-C₆, halogénoalkyle (C₁-C₆), alcoxy en C₁-C₆, alcoxy (C₁-C₆)-alkyle (C₁-C₆), cycloalkyle en (C₃-C₆), halogénocycloalkyle (C₃-C₆), alcynyle en C₂-C₆, halogénoalcynyle (C₂-C₆), alcényle en C₂-C₆) ou halogénoalcényle (C₂-C₆),
R² représente un atome d'hydrogène, un groupe alkyle en C₁-C₁₀ ou cycloalkyle (C₃-C₁₀),
les groupes alkyle et cycloalkyle étant non substitués ou substitués chacun indépendamment l'un de l'autre par m radicaux choisis dans le groupe constitué par halogène, cyano, isocyano, nitro, NH₂ (amino), alkyle en C₁-C₆, halogénoalkyle (C₁-C₆), halogénoalcoxy (C₁-C₆), alcényle (C₂-C₃), halogénoalcényle (C₂-C₃), alcoxy (C₁-C₆), alcynyle (C₂-C₃), halogénoalcynyle (C₂-C₃), alkyl (C₁-C₄) - S(O)ₙ, CHO, alkyloxy (C₁-C₄) -carbonyle, hétérocyclyl-alkyl (C₁-C₆), hétéroaryl-alkyle(C₁-C₄) et aryl-alkyle(C₁-C₄), les groupes aryle, hétérocyclyle et hétéroaryle étant non substitués ou substitués chacun indépendamment les uns des autres par m radicaux choisis dans le groupe constitué par halogène, alkyle(C₁-C₆) et halogénoalkyle (C₁-C₆),
R³ représente un atome d'hydrogène ou un groupe alkyle en C₁-C₁₀.
R⁴ représente un atome d'hydrogène, un groupe cyano, nitro, alkyle (C₁-C₁₀), halogénoalkyle (C₁-C₁₀), alcényle (C₂-C₁₀), alcynyle (C₃-C₁₀), alcoxy (C₁-C₁₀)-alkyle (C₁-C₁₀), halogénoalcoxy (C₁-C₁₀)-alkyle (C₁-C₁₀), alkyl (C₁-C₄) -S(O)ₙ-(C₁-C₄) -alkyle, halogénoalkyl (C₁-C₄) - S(O)ₙ-alkyle(C₁-C₄) , cycloalkyle (C₃-C₁₀), halogénocycloalkyle (C₃-C₁₀), hydroxy-alkyl(C₁-C₁₀)-carbonyle, amino-alkyle (C₁-C₁₀), alcoxy (C₁-C₁₀)carbonyl-alkyle (C₁-C₁₀), cyanoalkyle (C₁-C₁₀), S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸ ; aryle, hétéroaryle et hétérocyclyle,
qui sont non substitués ou substitués chacun indépendamment les uns des autres par m radicaux choisis dans le groupe constitué par hydrogène, halogène, cyano, nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸ ;
ou R³ et R⁴ forment avec l'atome d'azote, auquel ils sont liés, un cycle monocyclique ou bicyclique à 3 à 10 chaînons, totalement saturé ou partiellement saturé, éventuellement interrompu par des hétéroatomes et éventuellement substitué davantage,
R⁵ représente un atome d'hydrogène, un groupe alkyle en C₁-C₈, cycloalkyle en C₃-C₆, halogénoalkyle (C₁-C₈) ou aryle,
R⁶ représente un atome d'hydrogène ou R⁵,
R⁷ représente un atome d'hydrogène, un groupe alkyle (C₁-C₆), cycloalkyle (C₃-C₆), alcényle (C₃-C₄), alcynyle (C₃-C₄)ou alkyl (C₁-C₆)carbonyl-alkyle(C₁-C₄) ,
R⁸ représente un atome d'hydrogène, un groupe alkyle (C₁-C₆), cycloalkyle (C₃-C₆), alcényle (C₃-C₄) ou alcynyle (C₃-C₄),
R⁹ für un atome d'hydrogène, un atome d'halogène, un groupe cyano, isocyano, nitro, hydroxy, alkyle(C₁-C₆), halogénoalkyle (C₁-C₆), halogénoalcoxy (C₁-C₆), alcényle (C₂-C₃), halogénoalcényle (C₂-C₃), alcoxy (C₁-C₆), alcynyle (C₂-C₃), halogénoalcynyle (C₂-C₃), alkyl (C₁-C₄) - S(O)ₙ, CHO, alkyloxy (C₁-C₄) carbonyle ou NH₂ (amino),
m représente 0, 1 ou 2,
n représente 0, 1 ou 2,

3. Composés de formule (I) selon la revendication 1 ou 2 ou leurs sels agrochimiquement, dans lesquels Q¹ représente les groupes Q¹-1.1 à Q¹-6.3
| | | | | |
|---|---|---|---|---|
| 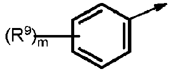 | 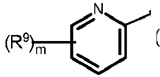 | 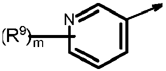 | 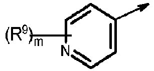 | 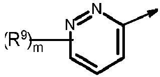 |
| Q¹-1.1 | Q¹-2.1 | Q¹-2.2 | Q¹-2.3 | Q¹-3.1 |
| 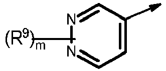 | 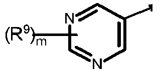 | 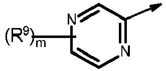 | 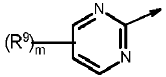 | 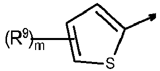 |
| Q¹-3.2 | Q¹-3.4 | Q¹-3.4 | Q¹-3.5 | Q¹-5.1 |
| 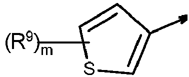 | 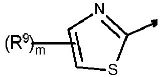 | 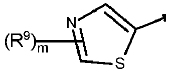 | 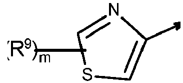 | 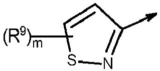 |
| Q¹-5.2 | Q¹-5.3 | Q¹-5.4 | Q¹-5.5 | Q²-5.6 |
| 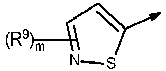 | 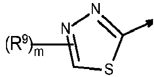 | 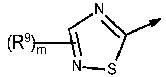 | 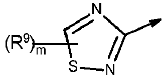 | |
| Q¹-5.7 | Q¹-6.1 | Q¹-6.2 | Q¹-6.3 | |
Q² représente un groupe phényle,
qui est non substitué ou substitué chaque fois indépendamment par m radicaux choisis dans le groupe constitué par hydrogène, halogène, cyano, alkyle(C₁-C₆), halogénoalkyle (C₁-C₆), halogénoalcoxy (C₁-C₆), cycloalkyle (C₃-C₆), alcoxy (C₁-C₆), alkyl (C₁-C₄) - S(O)ₙ et halogénoalkyl (C₁-C₄) - S(O)ₙ,
Z représente les groupes 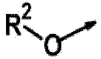 et 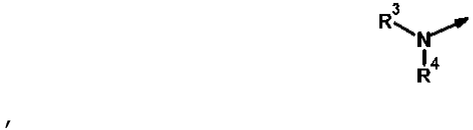
Y représente un atome d'halogène, un groupe cyano, isocyano, NO₂, alkyl (C₁-C₆), halogénoalkyl (C₁-C₆), alcoxy (C₁-C₆)-alkyle (C₁-C₆), alkyloxy (C₁-C₄) -carbonyle, alkyl (C₁-C₆)-carbonyle, alcoxy (C₁-C₆), halogénoalcoxy(C₁-C₆), alkyl (C₁-C₄) - S(O)ₙ, halogénoalkyl (C₁-C₄) -S(O)ₙ, alcynyle (C₂-C₆), halogénoalcynyle (C₂-C₆), alcényle (C₂-C₆) ou halogénoalcényle (C₂-C₆),
W représente un atome d'oxygène,
R¹ représente un atome d'hydrogène, un groupe cyano, alkyle (C₁-C₆), halogénoalkyle (C₁-C₆), alcoxy (C₁-C₆), alcoxy (C₁-C₄) -alkyle (C₁-C₄) , cycloalkyle (C₃-C₆), alcynyle (C₂-C₆), halogénoalcynyle (C₂-C₆), alcényle (C₂-C₆) ou halogénoalcényle(C₂-C₆),
R² représente un atome d'hydrogène, un groupe alkyle (C₁-C₆)ou cycloalkyle (C₃-C₆),
les groupes alkyle et cycloalkyle étant non substitués ou substitués chacun indépendamment l'une de l'autre par m radicaux choisis dans le groupe constitué par halogène, cyano, nitro, NH₂ (amino), alkyle (C₁-C₆), halogénoalkyle (C₁-C₆), halogénoalcoxy (C₁-C₆), alcényle (C₂-C₃), halogénoalcényle (C₂-C₃), alcoxy (C₁-C₆), alcynyle (C₂-C₃), halogénoalcynyle (C₂-C₃), alkyl (C₁-C₄)-S(O)ₙ, CHO, alkyloxy (C₁-C₄) -carbonyle, hétérocyclyl-alkyle (C₁-C₄) , hétéroaryl-alkyle(C₁-C₄) et aryl-alkyle(C₁-C₄), les groupes aryle, hétérocyclyle et hétéroaryle étant non substitués ou substitués chacun indépendamment les uns des autres par m radicaux choisis dans le groupe constitué par halogène, alkyle(C₁-C₆), halogénoalkyle(C₁-C₆),
R³ représente un atome d'hydrogène ou un groupe alkyle en C₁-C₆ ;
R⁴ représente un atome d'hydrogène, un groupe cyano, nitro, alkyle (C₁-C₆), halogénoalkyle (C₁-C₆), alcényle (C₂-C₆), alcynyle (C₃-C₆), alcoxy (C₁-C₆)-alkyle (C₁-C₆), halogénoalcoxy (C₁-C₆)-alkyle (C₁-C₆), alkyl (C₁-C₄) -S(O)ₙ-alkyle (C₁-C₄) et halogénoalkyl (C₁-C₄) -S(O)ₙ-alkyle(C₁-C₄), cycloalkyle (C₃-C₆), halogénocycloalkyle(C₃-C₆), hydroxy-alkyl (C₁-C₆)-carbonyle, amino-alkyle (C₁-C₆), alcoxy (C₁-C₆)-carbonyl-alkyle (C₁-C₆), S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸; aryle, hétéroaryle ou hétérocyclyle,
qui sont non substitués ou substitués chacun indépendamment les uns des autres par m radicaux choisis dans le groupe constitué par hydrogène, halogène, cyano, nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
ou R³ et R⁴ forment avec l'atome d'azote, auquel ils sont liés, un cycle monocyclique ou bicyclique à 3 à 10 chaînons, totalement saturé ou partiellement saturé, éventuellement interrompu par des hétéroatomes et éventuellement substitué davantage,
R⁵ représente un atome d'hydrogène, un groupe alkyle en C₁-C₈, cycloalkyle en C₃-C₆, halogénoalkyle (C₁-C₈) ou aryle,
R⁶ représente un atome d'hydrogène ou R⁵,
R⁷ représente un atome d'hydrogène, un groupe alkyle (C₁-C₆), cycloalkyle (C₃-C₆), alcényle (C₃-C₄), ou alcynyle (C₃-C₄),
R⁸ représente un atome d'hydrogène, un groupe alkyle (C₁-C₆), cycloalkyle (C₃-C₆), alcényle (C₃-C₄), alcynyle (C₃-C₄), ou alkyl (C₁-C₁₀) carbonyl-alkyle (C₁-C₆),
R⁹ représente un atome d'hydrogène, un atome d'halogène, un groupe cyano, isocyano, nitro, hydroxy, alkyle (C₁-C₆), halogénoalkyle (C₁-C₆), halogénoalcoxy(C₁-C₆), alcényle (C₂-C₃), halogénoalcényle (C₂-C₃), alcoxy (C₁-C₆), alcynyle (C₂-C₃), halogénoalcynyle (C₂-C₃), alkyl (C₁-C₄)-S(O)ₙ, CHO, alkyloxy(C₂-C₄)-carbonyle ou NH₂ (amino),
m représente 0, 1 ou 2,
n représente 0, 1 ou 2.

4. Composés de formule (I) selon l'une quelconque des revendications 1 à 3 ou sels agrochimiquement acceptables de ceux-ci, dans lesquels
Q¹ représente les groupes Q¹-1.1 à Q¹-6.3
| | | | | |
|---|---|---|---|---|
| 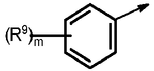 | 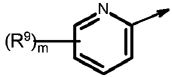 | 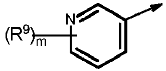 | 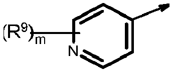 | 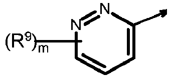 |
| Q¹-1.1 | Q¹-2.1 | Q¹-2.2 | Q¹-2.3 | Q¹-3.1 |
| 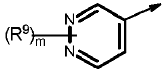 | 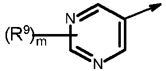 | 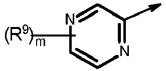 | 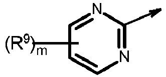 | 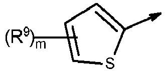 |
| Q¹-3.2 | Q¹-3.4 | Q¹-3.4 | Q¹-3.5 | Q²-5.1 |
| 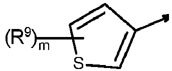 | 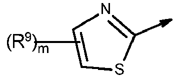 | 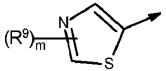 | 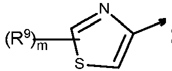 | 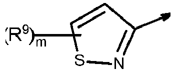 |
| Q¹-5.2 | Q¹-5.3 | Q¹-5.4 | Q¹-5.5 | Q²-5.6 |
| 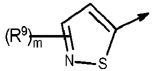 | 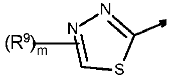 | 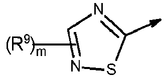 | 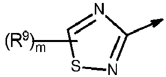 | |
| Q¹-5.7 | Q¹-6.1 | Q¹-6.2 | Q¹-6.3 | |
Q² représente un groupe phényle,
qui est non substitué ou substitué chaque fois indépendamment par m radicaux choisis dans le groupe constitué par les atomes d'hydrogène, de fluor, chlore et brome.
Z représente les groupes 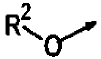 et 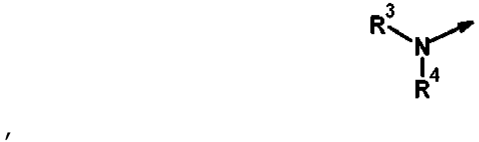
Y représente un atome de fluor, chlore, brome, un groupe cyano, NO₂, alkyle (C₁-C₂), halogénoalkyle (C₁-C₂), alkyl (C₁-C₂)-carbonyle, alcoxy(C₁-C₂), halogénoalcoxy(C₁-C₂), alkyl (C₁-C₂)-S(O)ₙ, ou halogénoalkyl (C₁-C₂)-S(O)ₙ,
W représente un atome d'oxygène,
R¹ représente un atome d'hydrogène, un groupe alkyle(C₁-C₄), halogénoalkyle (C₁-C₄) ou alcoxy (C₁-C₆),
R² représente un atome d'hydrogène, un groupe alkyle(C₁-C₆) ou cycloalkyle (C₃-C₆),
les groupes alkyle et cycloalkyle étant non substitués ou substitués chacun indépendamment l'une de l'autre par m radicaux choisis dans le groupe constitué par halogène, cyano, nitro, NH₂ (amino), alkyle (C₁-C₆), halogénoalkyle (C₁-C₆), halogénoalcoxy (C₁-C₆), alcényle (C₂-C₃), halogénoalcényle (C₂-C₃), alcoxy (C₁-C₆), alcynyle (C₂-C₃), halogénoalcynyle (C₂-C₃), alkyl (C₁-C₄)-S(O)ₙ, CHO, alkyloxy (C₁-C₄) -carbonyle, hétérocyclyl-alkyle(C₁-C₄), hétéroaryl-alkyle(C₁-C₄) et aryl-alkyle(C₁-C₄), les groupes aryle, hétérocyclyle et hétéroaryle étant non substitués ou substitués chacun indépendamment les uns des autres par m radicaux choisis dans le groupe constitué par halogène, alkyle(C₁-C₆), halogénoalkyle(C₁-C₆),
R³ représente un atome d'hydrogène ou un groupe alkyle en C₂-C₆;
R⁴ représente un atome d'hydrogène, un groupe cyano, nitro, alkyle (C₁-C₆), halogénoalkyle (C₁-C₆), alcényle (C₂-C₆), alcynyle (C₃-C₆), alcoxy (C₁-C₆)-alkyle (C₁-C₆), halogénoalcoxy (C₁-C₆)-alkyle (C₁-C₆), alkyl (C₁-C₄) -S(O)ₙ-alkyle (C₁-C₄) et halogénoalkyl (C₁-C₄) -S(O)ₙ-alkyle(C₁-C₄), cycloalkyle (C₃-C₆), halogénocycloalkyle(C₃-C₆), hydroxy-alkyl (C₁-C₆)-carbonyle, amino-alkyle (C₁-C₆), alcoxy (C₁-C₆)-carbonyl-alkyle (C₁-C₆), S(O)ₙR⁵, OR⁵, SO₂NR⁶R⁷, CO₂R⁸, COR⁸, NR⁶R⁸, NR⁶COR⁸, NR⁶CO₂R⁸, NR⁶SO₂R⁸; aryle, hétéroaryle ou hétérocyclyle,
qui sont non substitués ou substitués chacun indépendamment les uns des autres par m radicaux choisis dans le groupe constitué par hydrogène, halogène, cyano, nitro, OR⁵, S(O)ₙR⁵, SO₂NR⁶R⁷, CO₂R⁸, CONR⁶R⁸, COR⁶, NR⁶R⁸, NR⁶COR⁸, NR⁶CONR⁸R⁸, NR⁶CO₂R⁸, NR⁶SO₂R⁸, NR⁶SO₂NR⁶R⁸, C(R⁶)=NOR⁸;
ou R³ et R⁴ forment avec l'atome d'azote, auquel ils sont liés, un cycle monocyclique ou bicyclique à 3 à 10 chaînons, totalement saturé ou partiellement saturé, éventuellement interrompu par des hétéroatomes et éventuellement substitué davantage,
R⁵ représente un atome d'hydrogène, un groupe alkyle en C₁-C₈, cycloalkyle en C₃-C₆, halogénoalkyle (C₁-C₈) ou aryle,
R⁶ représente un atome d'hydrogène ou R⁵,
R⁷ représente un atome d'hydrogène, un groupe alkyle (C₁-C₆), cycloalkyle (C₃-C₆), alcényle (C₃-C₄), alcynyle(C₃-C₄) ou alkyl (C₁-C₁₀) carbonyl-alkyle (C₁-C₆),
R⁸ représente un atome d'hydrogène, un groupe alkyle (C₁-C₆), cycloalkyle (C₃-C₆), alcényle (C₃-C₄) ou alcynyle (C₃-C₄),
R⁹ représente un atome d'hydrogène, un atome d'halogène, un groupe cyano, isocyano, nitro, hydroxy, alkyle (C₁-C₆), halogénoalkyle (C₁-C₆), halogénoalcoxy(C₁-C₆), alcényle (C₂-C₃), halogénoalcényle (C₂-C₃), alcoxy (C₁-C₆), alcynyle (C₂-C₃), halogénoalcynyle (C₂-C₃), alkyl (C₁-C₄)-S(O)ₙ, CHO, alkyloxy(C₂-C₄)-carbonyle ou NH₂ (amino),
m représente 0, 1 ou 2,
n représente 0, 1 ou 2.

5. Composés de formule (I) selon l'une quelconque des revendications 1 à 3 ou sels agrochimiquement acceptables de ceux-ci, dans lesquels
Q¹ représente les groupes Q¹-1.1 à Q¹-5.7
| | | | | |
|---|---|---|---|---|
| 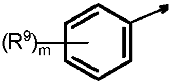 | 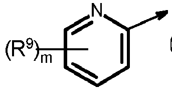 | 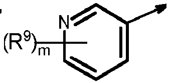 | 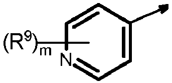 | 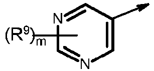 |
| Q¹-1.1 | Q¹-2.1 | Q¹-2.2 | Q¹-2.3 | Q¹-3.3 |
| 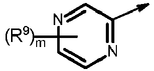 | 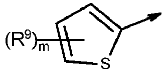 | 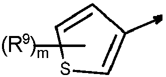 | 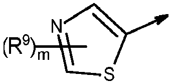 | 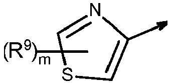 |
| Q¹-3.4 | Q¹-5.1 | Q¹-5.2 | Q¹-5.4 | Q¹-5.5 |
| 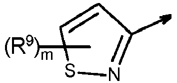 | 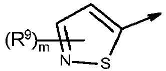 | | | |
| Q¹-5.6 | Q¹-5.7 | | | |
,
Q² représente un groupe phényle,
qui est non substitué ou substitué chaque fois indépendamment par m radicaux choisis dans le groupe constitué par les atomes d'hydrogène, de fluor, chlore et brome,
Z représente les groupes 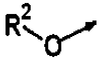 et 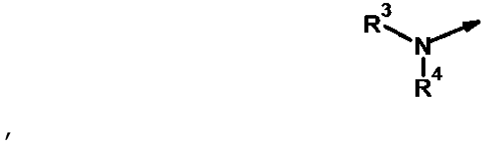
Y représente un atome de fluor, chlore, brome, un groupe cyano, NO₂, méthyle, CF₃ ou OCF₃,
W représente un atome d'oxygène,
R¹ représente un atome d'hydrogène, un groupe méthyle ou éthyle,
R² représente un atome d'hydrogène, un groupe méthyle, éthyle, allyle, propargyle ou PhCH₂,
R³ représente un atome d'hydrogène,
R⁴ représente des groupes alkyle (C₁-C₆)S(O)ₙR⁵, OR⁵, SO₂NR⁶R⁷, CO₂R⁸, COR⁸,
qui sont non substitués ou substitués chacun indépendamment les uns des autres par m radicaux choisis dans le groupe constitué par hydrogène, OR⁵, S(O)ₙR⁵, SO₂NR⁶R⁷, CO₂R⁸, COR⁶, NR⁶CO₂R⁸,
R⁵ représente un groupe méthyle, éthyle, CF₃, CH₂CF₃,
R⁶ représente un atome d'hydrogène ou R⁵,
R⁷ représente un atome d'hydrogène, un groupe méthyle, éthyle ou éthyl-2-éthanoyle,
R⁸ représente un groupe méthyle ou éthyle,
R⁹ représente un atome d'hydrogène, de fluor, chlore, brome, un groupe cyano, hydroxy, méthyle, éthyle, OCH₃, CF₃, ou OCF₃,
m représente 0, 1 ou 2,
n représente 0, 1 ou 2.

6. Procédé pour la préparation de composés de formule générale (Ia) ou de leurs sels agrochimiquement acceptables selon l'une quelconque des revendications 1 à 5, par mise en réaction de composés de formules générales (II) et (III), conduisant à des composés de formule générale (Ia) 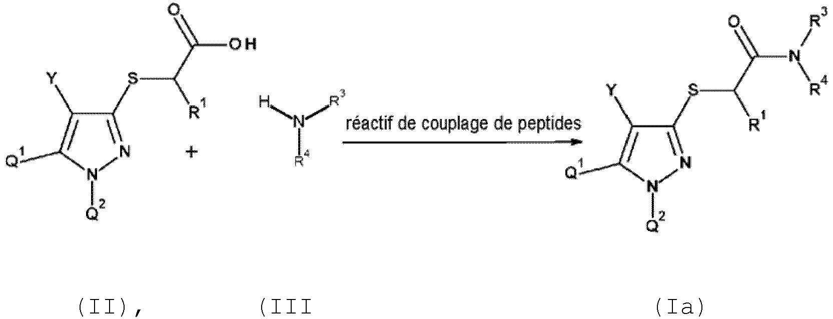 dans lesquels Q¹, Q², Y, R¹, R², R³ et R⁴ ont les significations indiquées plus haut.

7. Agent agrochimique, contenant a) au moins un composé de formule (I) ou un sel agrochimiquement acceptable de celui-ci, tel que défini dans une ou plusieurs des revendications 1 à 5, et b) des adjuvants et additifs usuels dans la protection des plantes.

8. Agent agrochimique, contenant
a) au moins un composé de formule (I) ou un sel agrochimiquement acceptable de celui-ci, tel que défini dans une ou plusieurs des revendications 1 à 5,
b) une ou plusieurs substances actives agrochimiques différentes du composant (a), et en option
c) des adjuvants et additifs usuels dans la protection des plantes.

9. Procédé pour la lutte contre des plantes indésirables ou pour la régulation de la croissance de plantes, dans lequel une quantité efficace d'au moins un composé de formule (I) ou un sel agrochimiquement acceptable de celui-ci, tel que défini dans une ou plusieurs des revendications 1 à 5, est appliquée sur les plantes, les semences ou la surface sur laquelle poussent les plantes.

10. Utilisation de composés de formule (I) ou de leurs sels pharmaceutiquement acceptables, tels que définis dans une ou plusieurs des revendications 1 à 5, en tant qu'herbicides ou régulateurs de la croissance végétale.

11. Utilisation selon la revendication 10, dans laquelle les composés de formule (I) ou leurs sels pharmaceutiquement acceptables sont utilisés pour la lutte contre des plantes nuisibles ou pour la régulation de la croissance dans des cultures de plantes.

12. Utilisation selon la revendication 11, dans laquelle les plantes cultivées sont des plantes cultivées transgéniques ou non transgéniques.
